# EUROPEAN PATENT APPLICATION

(11) **EP 4 775 680 A1**
(43) Date of publication of application: **15.07.2026**
(21) Application number: 24862899.2
(22) Date of filing: 06.09.2024
(51) Int. Cl.: C12P 21/02, C12N 9/12, C12N 15/09, C12P 19/34

(54) **METHOD FOR PRODUCING PROTEIN OR PEPTIDE, METHOD FOR PRODUCING 5'-CAPPED POLYNUCLEOTIDE, AND REAGENT FOR USE IN SAID PRODUCTION METHODS**

(30) Priority: 06.09.2023 JP 2023144835
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: ABE, Hiroshi, Nagoya-shi, Aichi 464-8601 (JP); INAGAKI, Masahito, Nagoya-shi, Aichi 464-8601 (JP); NAKASHIMA, Yuko, Nagoya-shi, Aichi 464-8601 (JP); ABE, Naoko, Nagoya-shi, Aichi 464-8601 (JP); HASHIYA, Fumitaka, Nagoya-shi, Aichi 464-8601 (JP); KIMURA, Yasuaki, Nagoya-shi, Aichi 464-8601 (JP)
(74) Representative: Müller-Boré & Partner Patentanwälte PartG mbB
(86) International application number: PCT/JP2024/032005
(87) International publication number: WO 2025/053250

(57) **Abstract**

An object of the present invention is to further enhance the translation efficiency in a technique in which a protein is translated by utilizing RNA encoding the protein and a capped polynucleotide capable of binding to the RNA via complementary base pairing, and to improve the capping efficiency in the production of a 5'-capped polynucleotide. This object is achieved by configuring the 5'-capped polynucleotide and/or the single-stranded RNA to have a translation efficiency enhancement structure, and by inserting base(s) into a promoter so that a base sequence of a polynucleotide of a cap analog and a base sequence upstream from a transcription initiation site of the promoter are complementary to each other in a specific positional relationship.

## Description

### Technical Field

The present invention relates to a method for producing a protein or a peptide, a method for producing a 5'-capped polynucleotide, a reagent for use in these methods, and the like.

### Background Art

In recent years, the use of mRNA vaccines has become widespread. The structure of an mRNA vaccine is divided into a cap structure, a 5'-untranslated region, a protein-coding region, a 3'-untranslated region, and a poly (A) tail region from the 5' end. The cap structure is required for protein translation to occur from the mRNA vaccine. However, in existing mRNA synthesis techniques, the ratio of mRNA having the cap structure to the total amount of mRNA (capping efficiency) is low, and immune response induced by uncapped mRNA has been a problem.

Under such circumstances, Non-patent Literature 1 has reported that capped mRNA could be produced with high purity by using a hydrophobic purification tag that can be removed by light as a cap analog, separating and purifying only capped mRNA by reversed-phase chromatography after the transcription reaction, and then removing the hydrophobic tag by photoreaction.

### Citation List

### Non-patent Literature

NPL 1: Nat Commun. 2023 May 11;14(1):2657. doi: 10.1038/s41467-023-38244-8

### Summary of Invention

### Technical Problem

Translation efficiency is also important to improve the performance of mRNA vaccines. In the course of research, the present inventors have developed a technique in which a protein is translated by utilizing RNA encoding the protein and capped RNA capable of binding to the RNA via complementary base pairing, and have focused on further enhancement of the translation efficiency in this technique.

An object of the present invention is to further enhance the translation efficiency in a technique in which a protein or a peptide is translated by utilizing RNA encoding the protein or peptide and a capped polynucleotide capable of binding to the RNA via complementary base pairing, and to improve the capping efficiency in the production of a 5'-capped polynucleotide.

### Solution to Problem

As a result of extensive research in view of the above problem, the present inventors found that the structure of a 5'-capped polynucleotide and/or a single-stranded RNA (RNA encoding a protein or a peptide) contributes to enhancing the translation efficiency, and further found that the capping efficiency is improved by inserting base(s) into a promoter so that a base sequence of a polynucleotide of a cap analog and a base sequence upstream from a transcription initiation site of the promoter are complementary to each other in a specific positional relationship. As a result of further research based on these findings, the present inventors have completed the present invention. Specifically, the present invention includes the following aspects.

Item 1. A method for producing or expressing a protein or a peptide, the method comprising:
carrying out a translation reaction in a reaction system comprising:
a 5'-capped polynucleotide containing an arbitrary base sequence A; and
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure.

Item 2. The method according to Item 1, wherein the 5'-capped polynucleotide is a 5'-capped RNA.

Item 3. The method according to Item 1 or 2, wherein the 5'-capped polynucleotide is a polynucleotide endogenous to a living organism or a cell.

Item 4. The method according to Item 3, wherein the polynucleotide endogenous to a living organism or a cell is a lncRNA or an mRNA.

Item 5. The method according to any one of Items 1 to 4, wherein the translation efficiency enhancement structure is a structure for stabilizing the binding between the 5'-capped polynucleotide and the single-stranded RNA.

Item 6. The method according to any one of Items 1 to 5, wherein the method satisfies the requirement (b) that the 5'-capped polynucleotide and/or the single-stranded RNA has a nuclease-binding structure, and the reaction system contains the nuclease,
wherein the nuclease is RISC or Cas, and
the 5'-capped polynucleotide is a siRNA or a miRNA, or
the 5'-capped polynucleotide is a lncRNA or an mRNA, and the single-stranded RNA contains a tracrRNA sequence.

Item 7. The method according to any one of Items 1 to 6, wherein the method satisfies at least one requirement selected from the group consisting of:
(a) the single-stranded RNA is a circular RNA; and
(c) the 5'-capped polynucleotide and/or the single-stranded RNA has a structure allowing the 5'-capped polynucleotide and the single-stranded RNA to be covalently linked.

Item 8. The method according to Item 7, wherein the method satisfies the requirement (a), and the circular RNA contains no stop codon, and/or
the method satisfies the requirement (c), and the structure is a photocrosslinkable structure.

Item 9. A composition comprising:
a 5'-capped polynucleotide containing an arbitrary base sequence A; and/or
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure.

Item 10. The composition according to Item 9 for use in the method according to any one of Items 1 to 8.

Item 11. A method for producing or expressing a protein or a peptide, the method comprising:
carrying out a translation reaction in a reaction system comprising:
a single-stranded circular RNA having a length of 500 or less bases, the single-stranded circular RNA comprising a 5'-capped polynucleotide linked thereto via a linker.

Item 12. A composition comprising a single-stranded circular RNA having a length of 500 or less bases, the single-stranded circular RNA comprising a 5'-capped polynucleotide linked thereto via a linker.

Item 13. The composition according to Item 12 for use in the method according to Item 11.

Item 14. A method for producing a 5'-capped polynucleotide comprising:
carrying out a transcription reaction in a reaction system comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side)-Y1-Y2-Y3, wherein Y1 and Y2 each represent a base in the base-inserted mutant sequence, and Y3 represents a base at the transcription initiation site; and
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2.

Item 15. The method according to Item 14, wherein an antisense strand of the phage promoter contains the base sequence Y: (upstream side)-Y1-Y2-Y3, and wherein X1 is a base complementary to Y1, and X2 is a base complementary to Y2.

Item 16. The method according to Item 14 or 15, wherein the 5'-capped polynucleotide is a 5'-capped RNA.

Item 17. The method according to any one of Items 14 to 16, wherein the phage promoter is a T7 promoter.

Item 18. The method according to any one of Items 14 to 17, wherein the base sequence X is (cap side)-CUG, CAG, GUG, or GAG, and Y3 is C.

Item 19. A composition comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side)-Y1-Y2-Y3, wherein Y1 and Y2 each represent a base in the base-inserted mutant sequence, and Y3 represents a base at the transcription initiation site; and/or
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2.

Item 20. The composition according to Item 19 for use in the method according to any one of Items 14 to 18.

Item 21. A T7 RNA polymerase comprising a D130W mutation.

### Advantageous Effects of Invention

According to the present invention, it is possible to provide a technique for further enhancing the translation efficiency in a technique in which a protein or a peptide is translated by utilizing RNA encoding the protein or peptide and a capped polynucleotide capable of binding to the RNA via complementary base pairing, and provide a technique for improving the capping efficiency in the production of a 5'-capped polynucleotide.

### Brief Description of Drawings

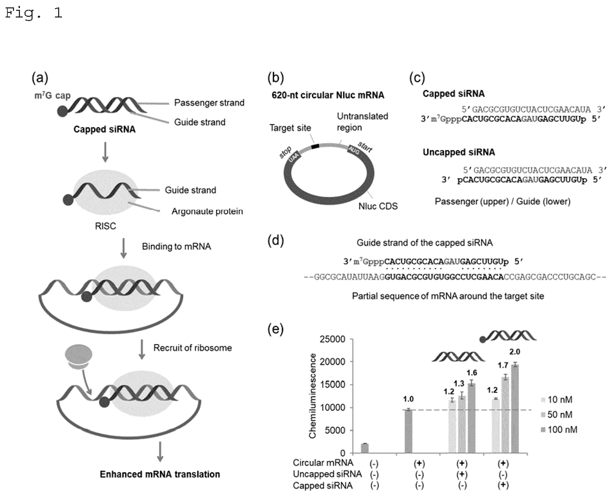

Fig. 1 shows a phenomenon of enhanced translation reaction of circular mRNA utilizing the RNA interference mechanism. (a) A conceptual diagram of this test. Short double-stranded RNA (capped siRNA) carrying an m7G cap structure at the 3' end of the guide strand is incorporated into a RISC complex and binds to circular mRNA containing a mostly complementary sequence. Since a non-complementary region is present in the central portion of the guide strand in the resulting RNA double-stranded structure, a cleavage reaction of the mRNA is not induced. The m7G cap structure present at the end of the guide strand recruits a ribosome to the vicinity of the start codon of the circular mRNA to enhance protein expression from the circular mRNA. (b) A schematic diagram of the circular mRNA sequence used. (c) A schematic diagram of the sequences and structures of synthesized capped and uncapped short double-stranded RNAs (capped siRNA, uncapped siRNA). (d) A double-stranded structure of the guide strand and mRNA expected to form in the RISC complex. (e) Experimental results showing that the capped siRNA can enhance the translation reaction of circular mRNA in a dose-dependent manner using human-derived cultured cells (HeLa). The graph shows the results of the experiment in which the circular RNA and siRNA molecule were transfected into HeLa cells using a commercially available lipofection reagent, the cells were lysed after culturing for 24 hours, and the amount of translation product (NanoLuc luciferase, Nluc) contained in the lysate was evaluated.

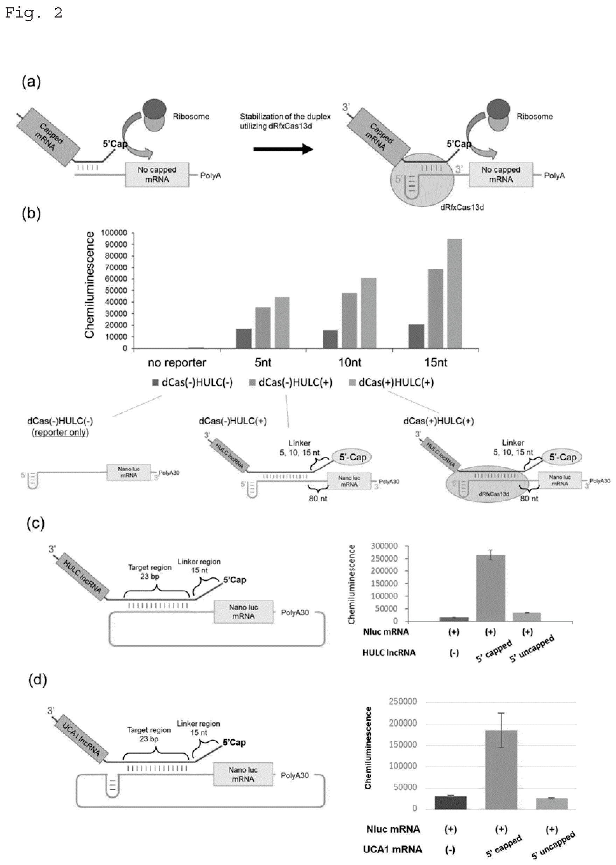

Fig. 2 shows a phenomenon of enhanced translation of circular mRNA utilizing hybridization with long non-coding RNA (lncRNA) and stabilization thereof by the dCas13 protein. (a) (b) An experiment evaluating the translation activity of uncapped linear mRNA encoding NanoLuc luciferase (Nluc) in HeLa cells (model experiment using linear mRNA). The mRNA was transfected into HeLa cells using a commercially available lipofection reagent, and HULC RNA alone separately mixed with the lipofection reagent or HULC RNA and dRfxCas13d mRNA separately mixed with the lipofection reagent were transfected into the cells. HULC RNA when added alone or in combination with dRfxCas13d mRNA improved the protein expression level from the uncapped linear Nluc mRNA. The length of the sequence of the portion corresponding to a linker between the 5'-cap structure and the hybridization region was changed to 5, 10, or 15 bases; however, no significant difference was observed, and a similar increasing tendency was observed in all cases. (c) The interaction with HULC lncRNA enhanced the translation reaction of circular Nluc mRNA in the HeLa cells. This phenomenon was dependent on the 5'-cap structure of the HULC lncRNA. (d) The interaction with UCA1 lncRNA enhanced the translation reaction of circular Nluc mRNA in the HeLa cells. This phenomenon was dependent on the 5'-cap structure of the UCA1 lncRNA.

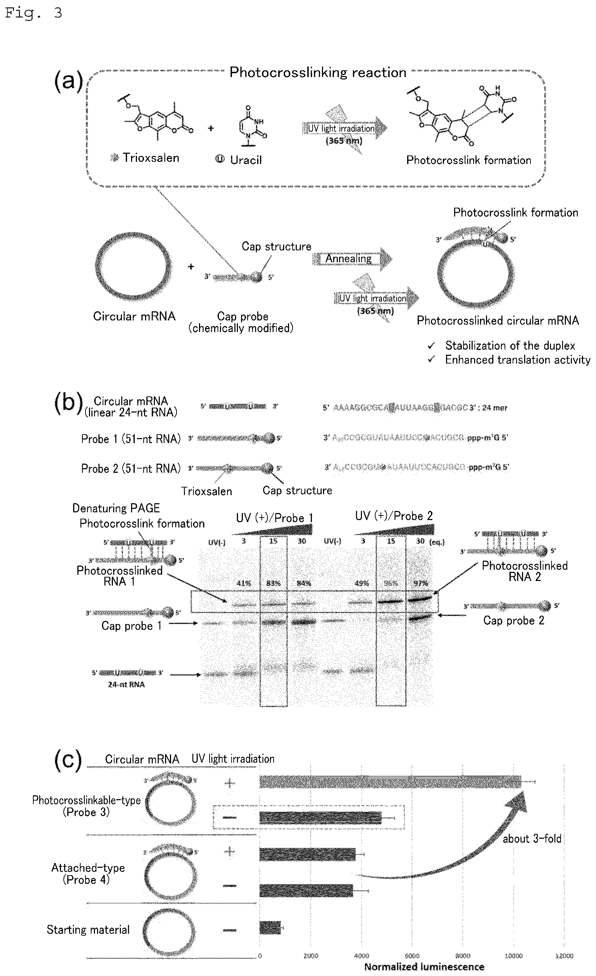

Fig. 3 shows a phenomenon in which a photocrosslinkable capped RNA probe enhances the translation reaction of circular mRNA in a manner dependent on light irradiation. (a) A conceptual diagram of the system. A capped RNA probe containing trioxsalen can form a covalent linkage with a complementary strand by a photocrosslinking reaction. The binding of the capped probe to circular mRNA can be stabilized to enhance the translation enhancing effect. (b) A model experiment using a short RNA oligonucleotide consisting of 24 bases as a probe target, as a model of circular mRNA. The crosslinking reaction product can form a stable complex in denaturing polyacrylamide gel electrophoresis. It is observed that when 15 molar equivalents of the probe were added to the mRNA model, the mRNA model formed a covalent linkage with the probe almost quantitatively. (c) The capped probe was annealed to 650-base circular mRNA encoding Nluc, followed by 365-nm light irradiation. It is observed that when transfected into HeLa cells, the translation enhancing effect of the photocrosslinkable capped probe increased as compared to the control group.

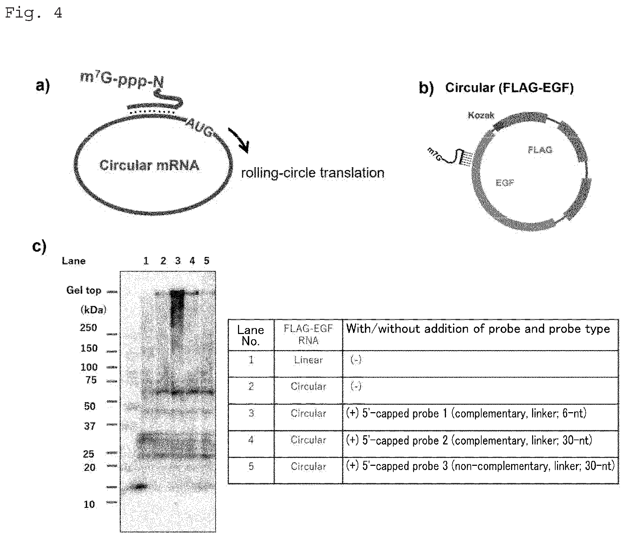

Fig. 4 shows an enhanced rolling circle translation reaction by capped RNA. (a) A conceptual diagram of this test. The rolling circle translation phenomenon is enhanced by binding capped RNA to circular RNA having no stop codon. (b) A schematic diagram of the circular mRNA used. (c) Experimental results showing that capped oligo RNA probes enhance the rolling circle translation reaction, using rabbit reticulocyte lysate (RRL). The circular RNA annealed with the probes was translated in RRL, and the translation products were detected by Western blot analysis using FLAG antibody. In the presence of the capped oligo RNA probes containing a region complementary to the circular RNA sequence, a significant increase in the translation products attributed to multiple rounds of translation by a ribosome on the circular RNA was observed.

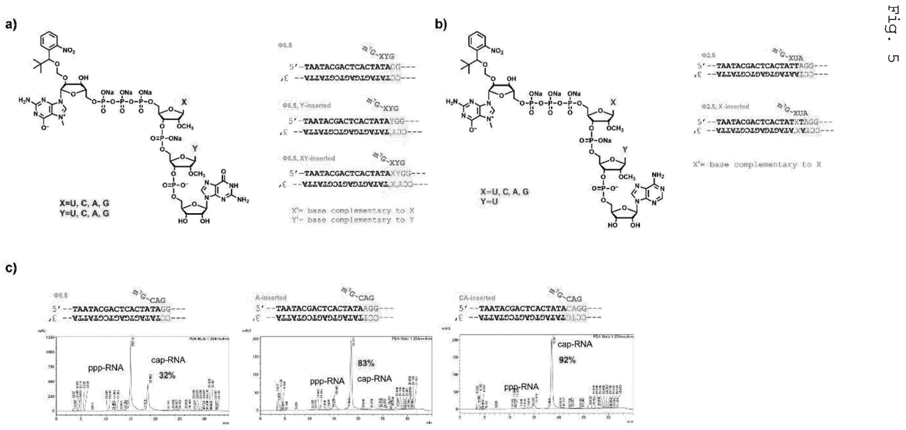

Fig. 5 shows the development of a highly efficient method of synthesizing Cap-2 mRNAs using TetraPureCap analogs and modified promoters. (a) The structure and sequence of analogs of the TetraPureCap-XYG series, and the sequences of modified promoters of T7 RNA polymerase. (b) The structure and sequence of analogs of the TetraPureCap-XYA series, and the sequence of a modified promoter of T7 RNA polymerase. (c) Experimental results showing that the transcription reactions using template DNAs having modified promoters improved the incorporation efficiency of the TetraPureCap analogs. For the native promoter sequence, the incorporation efficiency of the cap analog was about 30% due to the competition with GTP; however, the insertion of a sequence complementary to the cap analog increased the thermodynamic stability and improved the incorporation of the cap analog to 80 to 90%.

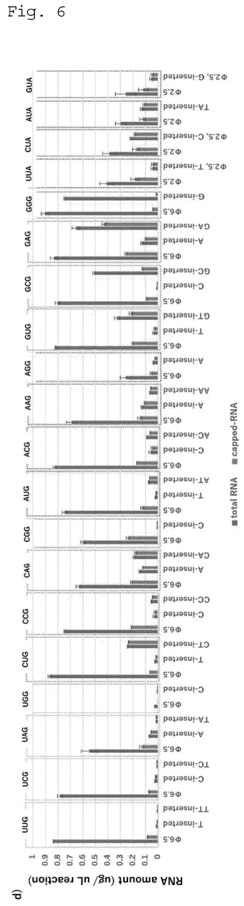

Fig. 6 shows the development of a highly efficient method of synthesizing Cap-2 mRNAs utilizing TetraPureCap analogs and modified promoters. (d) Experimental results of quantification by HPLC analysis of total transcription amounts and amounts of synthesized capped RNAs by combinations of TetraPureCap analogs and modified promoters.

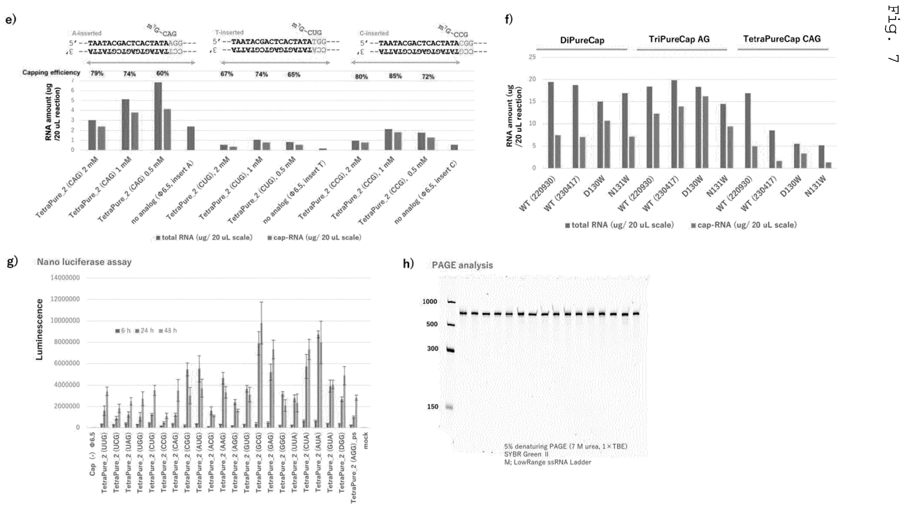

Fig. 7 shows the development of a highly efficient method of synthesizing Cap-2 mRNAs utilizing TetraPureCap analogs and modified promoters. (e) Experimental results showing that, by utilizing complementarity between the TetraPureCap analogs and promoters, the incorporation efficiency of the analogs is maintained even when the concentrations of the TetraPureCap analogs are lower than the concentration of NTPs (2 mM), in the transcription reactions. (f) Experimental results showing the effect of a T7 RNA polymerase mutant enzyme (D130W) designed and synthesized for the purpose of improving the incorporation efficiency of the TetraPureCap analogs. D130W successfully improved the incorporation efficiency of the cap analogs even when using template DNA having a native promoter sequence. (g) Experimental data showing the translation activities of cap-2 mRNAs synthesized using the TetraPureCap analogs. Depending on the base sequence at the 5' end, differences in translation activity of about 3-fold were observed. (h) PAGE analysis data confirming the purity of the mRNAs used for the translation activity experiment.

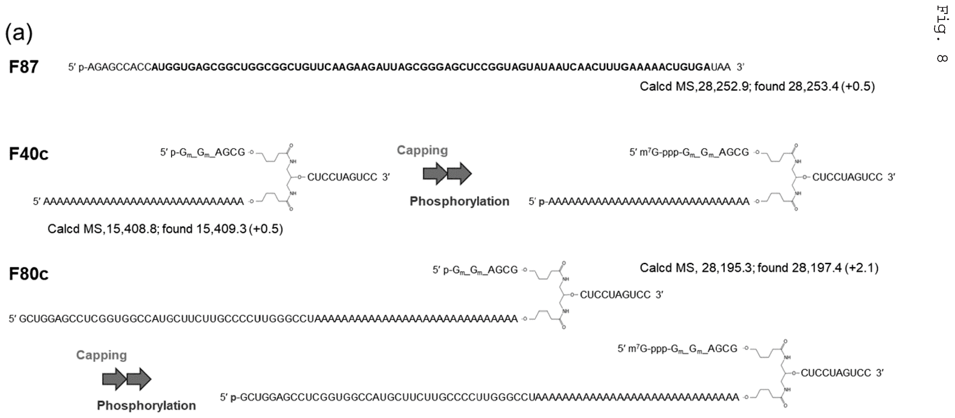

Fig. 8 shows experimental results demonstrating that small circular mRNAs (circRNAs; with a circular size of 127 or 167 bases) containing an m7G cap structure constructed from two fragments of chemically synthesized oligonucleotides have translation activity in a human cell line. (a) A diagram showing chemically synthesized RNA oligonucleotide sequences as starting materials, for preparing small circular mRNAs. The calculated and measured values of the molecular weights of the chemically synthesized fragments are shown in the figure. Both values were in good agreement. The bold text in the F87 sequence represents a coding region of the peptide (translation product peptide sequence, MVSGWRLFKKISGSSGSIINFEKL (SEQ ID NO: 51)). In the figure, the base sequence of F87 is shown in SEQ ID NO: 46; the base sequences of F40c are shown in SEQ ID NOS: 47 and 48; and the base sequences of F80c are shown in SEQ ID NOS: 49 and 50.

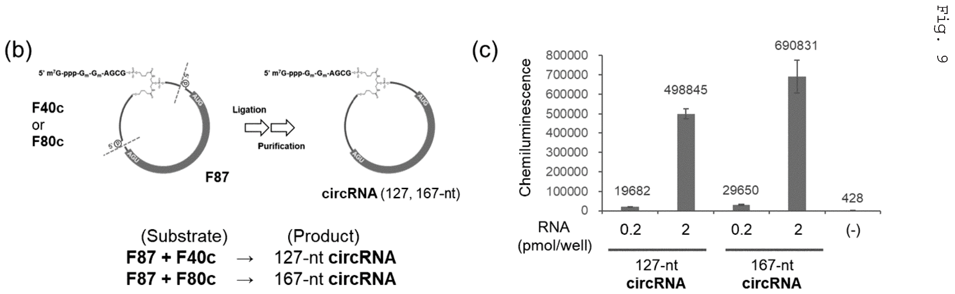

Fig. 9 shows experimental results demonstrating that small circular mRNAs (circRNAs; with a circular size of 127 or 167 bases) containing an m7G cap structure constructed from two fragments of chemically synthesized oligonucleotides have translation activity in a human cell line. (b) A conceptual diagram of a method of constructing circRNA by enzymatically ligating the substrate RNA oligonucleotides shown in (a). The relationship between the combinations of starting material sequences and products is shown in the figure. (c) Results of translation activity evaluation of the prepared circRNAs in human-derived cultured cells (HeLa). The RNAs were transfected into HeLa cells using a commercially available transfection reagent, and the amounts of produced HiBiT peptide encoded by the RNAs were measured. It was found that the peptide was produced as a translation product, from the 127-nt and 167-nt circular RNAs. The mean values (n = 4) and standard errors are shown as error bars.

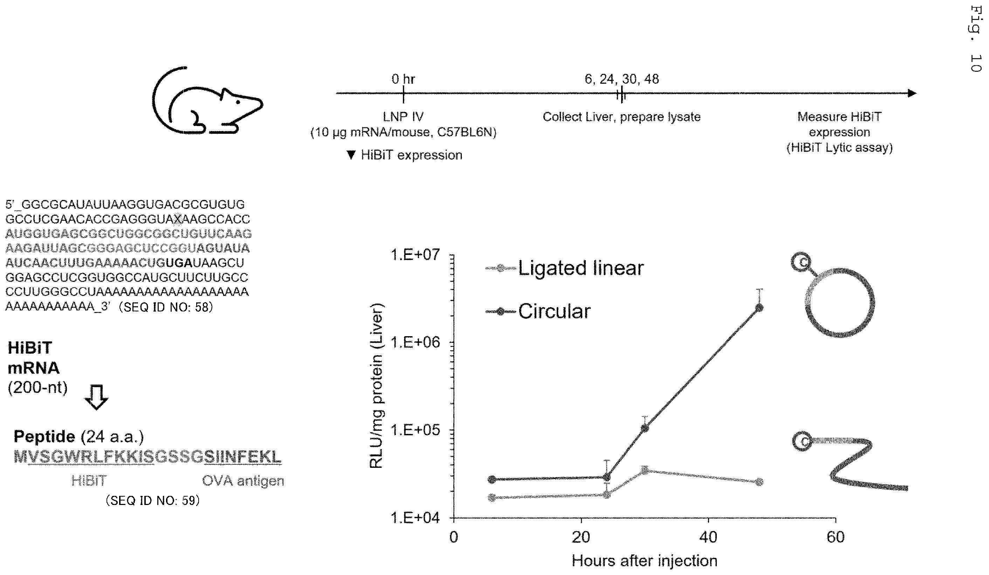

Fig. 10 shows the assay results of Test Example 7. The test scheme is shown in the upper section, the base sequence of the nucleic acid used is shown in the lower left section, and the graph of the assay results is shown in the lower right section. In the graph, the legends indicate the shapes of the chemically synthesized mRNAs used.

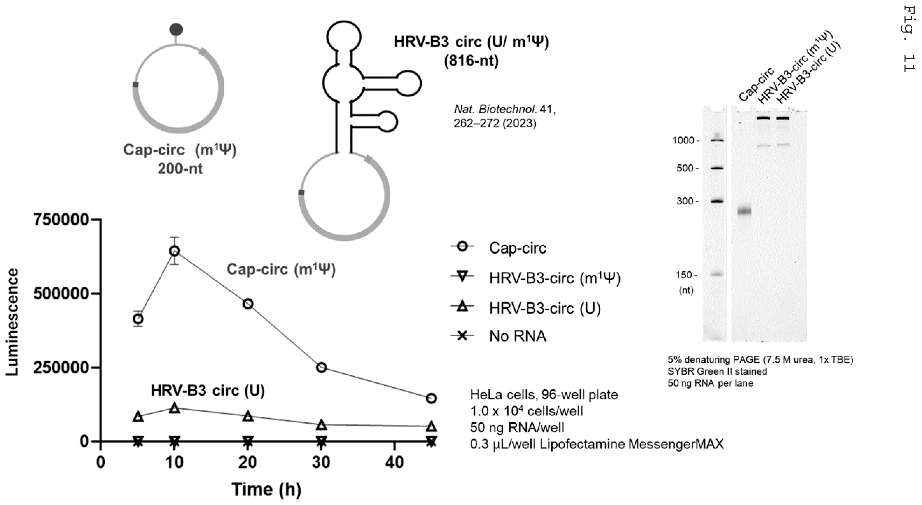

Fig. 11 shows the assay results of Test Example 8. A schematic diagram of the structures of the mRNAs used is shown in the upper left section, the results of denaturing electrophoresis of the mRNAs used are shown on the right side, and a graph of the assay results is shown in the lower left section.

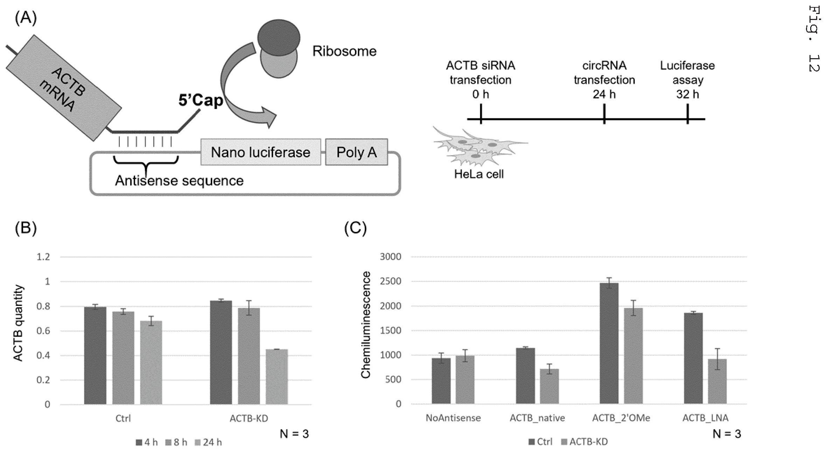

Fig. 12 shows a phenomenon of enhanced translation of circular mRNA by hybridization with endogenous mRNA (ACTB). (A) An experiment evaluating the translation activity of uncapped circular mRNA (circRNA) encoding NanoLuc luciferase (Nluc) in HeLa cells. The antisense sequence of human ACTB mRNA was introduced into the Antisense sequence region. (B) Transfection of ACTB siRNA into HeLa cells using a commercially available lipofection reagent reduced ACTB mRNA to nearly half after 24 hours. The cells were transfected with circRNAs, and Nluc expression levels were evaluated after 8 hours. (C) The circRNAs containing the antisense sequence of ACTB mRNA (ACTB_native, ACTB_2'OMe, ACTB_LNA) showed improved translation amounts of Nluc compared to that not containing the antisense sequence of ACTB mRNA (NoAntisense); in particular, the circRNAs (ACTB_2'OMe, ACTB_LNA) including the entire antisense sequence region modified with 2'OMe or LNA showed significantly increased translation amounts. Moreover, when ACTB mRNA was knocked down by siRNA, the circRNA containing no antisense sequence was not affected, but the translation amounts by the circRNAs containing the antisense sequence of ACTB mRNA decreased to at most nearly half.

### Description of Embodiments

As used herein, the terms "comprise" and "contain" include the concepts of "comprise," "contain," "essentially consist of," and "consist of."

As used herein, the polynucleotide is not specifically limited, and may be DNA, RNA, or the like, or may be subjected to known chemical modifications, examples of which are given below. In order to prevent degradation by hydrolases such as nucleases, the phosphate residue (phosphate) of each nucleotide can be substituted with, for example, a chemically modified phosphate residue such as phosphorothioate (PS), methylphosphonate, or phosphorodithioate. Moreover, the hydroxy group at the 2-position of the sugar (ribose) of each ribonucleotide may be substituted with -OR (R represents, for example, CH3 (2'-O-Me), CH2CH2OCH3 (2'-O-MOE), CH2CH2NHC(NH)NH2, CH2CONHCH3, or CH2CH2CN). Furthermore, a base moiety (pyrimidine or purine) may be chemically modified, and examples of such chemical modifications include introduction of a methyl group or a cationic functional group into the 5-position of a pyrimidine base, and substitution of the carbonyl group at the 2-position with thiocarbonyl. Other examples include, but are not limited to, phosphate and hydroxyl moieties modified with biotin, an amino group, a lower alkylamine group, an acetyl group, or the like.

As used herein, the polynucleotide may be linked to another molecule. Examples of the other molecule include a fluorescent label. Examples of the fluorescent label include fluorescein, Rhodamin, Texas Red, tetramethyl rhodamine, carboxyrhodamine, phycoerythrin, 6-FAM (trademark), Cy (registered trademark) 3, Cy (registered trademark) 5, and the Alexa Fluor (registered trademark) series.

As used herein, the bases constituting a nucleic acid include not only typical bases (such as adenine (A), thymine (T), uracil (U), guanine (G), and cytosine(C)) in natural nucleic acids such as RNA and DNA, but also other bases such as hypoxanthine (I) and modified bases. Examples of the modified bases include pseudouracil, 3-methyluracil, dihydrouracil, 5-alkylcytosines (for example, 5-methylcytosine), 5-alkyluracils (for example, 5-ethyluracil), 5-halouracil (5-bromouracil), 6-azapyrimidine, 6-alkylpyrimidines (6-methyluracil), 4-acetylcytosine, 5-(carboxyhydroxymethyl) uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2-aminopurine, isoguanine, indole, imidazole, xanthine, and cyanuric acid.

### 1. Method 1 for Producing or Expressing a Protein or a Peptide

This section corresponds to Items 1 to 7 described above and Test Examples 1 to 4 (Figs. 1 to 4) described below. However, the aspects of the present invention are not limited to the aspects of the test examples described below.

In one aspect, the present invention relates to
a method for producing or expressing a protein or a peptide (sometimes also referred to herein as "the method of the present invention"), the method comprising:
carrying out a translation reaction in a reaction system comprising:
a 5'-capped polynucleotide containing an arbitrary base sequence A; and
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure. This method will be described below.

The 5'-capped polynucleotide used in the method of the present invention is not specifically limited as long as it has a 5'-cap structure and is capable of binding to the single-stranded RNA via complementary base pairing. The 5'-capped polynucleotide is particularly preferably RNA. Examples of the 5'-cap structure include, but are not specifically limited to, cap-0, cap-1, and cap-2.

The 5'-capped polynucleotide can be single-stranded or double-stranded.

The single-stranded RNA used in the method of the present invention contains a protein-coding or peptide-coding sequence. The protein-coding or peptide-coding sequence is the coding sequence of the target protein or peptide to be produced or expressed in the method of the present invention. Examples of the protein or peptide include cancer antigens and microbial antigens. Examples of the cancer antigens include common antigens (tumor-associated antigens), such as differentiation antigens (gp100, MART-1, and the like), fetal proteins (CEA, AFP, and the like), glycoprotein/glycolipid/carbohydrate antigens (MUC-1, CA125, and the like), and overexpressed proteins (HER2, Survivin, WT-1, and the like); and specific antigens (neoantigens), such as (patient-specific) cancer antigens derived from driver mutations (KRAS, BRAF, EGFR, and the like) and passenger mutations. Examples of the microbial antigens include viral antigens and bacterial antigens. Examples of viruses from which the viral antigens are derived include, but are not specifically limited to, enveloped viruses (viruses with envelopes), such as influenza viruses (for example, type A, type B, and the like), rubella virus, Ebola virus, coronavirus, measles virus, varicella-zoster virus, herpes simplex virus, mumps virus, arbovirus, RS virus, SARS virus, hepatitis virus (for example, hepatitis B virus, hepatitis C virus, and the like), yellow fever virus, AIDS virus, rabies virus, hantavirus, dengue virus, Nipah virus, and lyssavirus; and non-enveloped viruses (viruses without envelopes), such as adenovirus, norovirus, rotavirus, human papillomavirus, poliovirus, enterovirus, coxsackievirus, human parvovirus, encephalomyocarditis virus, and rhinovirus. Examples of bacteria from which the bacterial antigens are derived include, but are not specifically limited to, *Bordetella pertussis, Tetanus bacillus, Corynebacterium diphtheriae, Salmonella enterica, Helicobacter pylori, Clostridium perfringens, Clostridium botulinum, Campylobacter, Escherichia coli, Staphylococcus aureus, Streptococcus pyogenes, B. cereus, Vibrio parahaemolyticus, Propionibacterium acnes, Enterococcus faecalis, Clostridium difficile, Streptococcus pneumoniae, Haemophilus influenzae, Moraxella, Klebsiella pneumoniae, Corynebacterium, Streptococcus, Pseudomonas aeruginosa, Staphylococcus, Mycoplasma, Candida* spp., and *Aspergillus* spp.

The 5'-capped polynucleotide contains an arbitrary base sequence A, and the single-stranded RNA contains a base sequence B capable of binding to the base sequence A via complementary base pairing. The base sequence A and the base sequence B allow the 5'-capped polynucleotide and the single-stranded RNA to bind to each other, which causes a translation complex necessary for translation of the single-stranded RNA to be located. It is noted that the base sequence A and the base sequence B are names for distinguishing them from each other; alternatively, the base sequence B may be the arbitrary base sequence, and the base sequence A may be the base sequence capable of binding to the base sequence B via complementary base pairing.

Complementary base pairing occurs when one base sequence is complementary to another. The term "complementary" includes not only a fully complementary relationship of the bases (fully complementary: for example, A and T or U, and G and C), but also a complementary relationship to the extent that the bases can be hybridized under stringent conditions. The stringent conditions can be determined based on the melting temperature (Tm) of a nucleic acid, as taught in Berger and Kimmel (1987, Guide to Molecular Cloning Techniques Methods in Enzymology, Vol. 152, Academic Press, San Diego CA). For example, post-hybridization washing conditions may be usually about 1 × SSC, 0.1% SDS, 37°C. The hybridized state is preferably maintained even after washing under such conditions. More stringent hybridization conditions may be, for example, about 0.5 × SSC, 0.1% SDS, 42°C, and still more stringent hybridization conditions may be, for example, about 0.1 × SSC, 0.1% SDS, 65°C, although not specifically limited thereto. Specifically, the base sequence B is a base sequence having, for example, 85% or more identity, preferably 90% or more identity, more preferably 95% or more identity, still more preferably 98% or more identity, even more preferably 99% or more identity, and particularly preferably 100% identity, to a base sequence fully complementary to the base sequence A.

As used herein, the term "identity" of base sequences refers to the degree of matching between two or more comparable base sequences. Therefore, the higher the match between two base sequences, the higher the identity or similarity of these sequences. The level of identity between base sequences is determined, for example, using a sequence analysis tool, FASTA, with default parameters. Alternatively, the identity level can be determined using the algorithm BLAST (Karlin S, Altschul SF. "Methods for assessing the statistical significance of molecular sequence features by using general scoring schemes" Proc Natl Acad Sci USA. 87:2264-2268 (1990), Karlin S, Altschul SF. "Applications and statistics for multiple high-scoring segments in molecular sequences. " Proc Natl Acad Sci USA. 90:5873-7 (1993)). A program called BLASTX has been developed based on the BLAST algorithm. Specific methods of these analysis methods are known, and reference may be made to the website of the National Center of Biotechnology Information (NCBI) (http://www.ncbi.nlm.nih.gov/).

The base sequences A and B have a length of, for example, 8 to 1000 bases, 10 to 500 bases, or 25 to 200 bases, although not specifically limited thereto.

The 5'-capped polynucleotide and the single-stranded RNA can be produced by or according to a known method for producing polynucleotides. A polynucleotide or RNA that is present in a living organism or a cell may be used as the 5'-capped polynucleotide or the single-stranded RNA. The 5'-capped polynucleotide can be preferably produced by the method of the present invention described below.

In one preferred aspect of the present invention, the 5'-capped polynucleotide is a polynucleotide endogenous to a living organism or a cell. For example, a lncRNA, an mRNA, or the like, preferably a lncRNA, can be used as the polynucleotide. By selecting a lncRNA or an mRNA that is specifically expressed in a specific cell or tissue, a protein encoded by the single-stranded RNA exogenously introduced into the living organism or cell can be expressed in a cell-specific or tissue-specific manner. This can also reduce the side effects and off-target effect. Moreover, by using the protein encoded by the single-stranded RNA as a reporter protein, the specific lncRNA or mRNA can be detected. Alternatively, the single-stranded RNA may be an RNA endogenous to a living organism or a cell. For example, a circular mRNA, a linear mRNA, or the like, preferably a circular mRNA, can be used as the polynucleotide.

The method of the present invention comprises carrying out a translation reaction in a reaction system comprising the 5'-capped polynucleotide and the single-stranded RNA.

The reaction system is not specifically limited as long as it contains substances necessary for the translation reaction (for example, ribosomes, amino acids, tRNA, and the like), and may be either an in vivo or in vitro reaction system. In the case of the in vivo reaction system, examples of the biological species include, but are not specifically limited to, various mammalian animals, such as humans, monkeys, mice, rats, dogs, cats, and rabbits; and animal cells. The type of cells is also not specifically limited, and examples include blood cells, hematopoietic stem cells/progenitor cells, gametes (spermatozoa, oocytes), fibroblasts, epithelial cells, vascular endothelial cells, nerve cells, hepatocytes, keratinocytes, muscle cells, epidermal cells, endocrine cells, ES cells, iPS cells, tissue stem cells, and cancer cells. In the case of the in vitro reaction system, a reconstituted system may be used, or an extract of any of the organisms or cells described above may be used.

The reaction conditions of the translation reaction are also not specifically limited, and can be set to appropriate conditions depending on the enzyme used and the like.

When the method of the present invention is carried out in vivo, the method can be carried out by introducing the 5'-capped polynucleotide and/or the single-stranded RNA into a subject (an organism or a cell). In one embodiment, when the method of the present invention is carried out in vivo, the method can be carried out by introducing the 5'-capped polynucleotide and the single-stranded RNA into the subject. In one embodiment, when the method of the present invention is carried out in vivo, the method can be particularly preferably carried out by introducing the 5'-capped polynucleotide or the single-stranded RNA into the subject. Moreover, when the method of the present invention is carried out in vitro, the method can be carried out by adding the 5'-capped polynucleotide and/or the single-stranded RNA (particularly preferably, the 5'-capped polynucleotide or the single-stranded RNA (i.e., either one)) to the reaction system. Therefore, in one aspect, the present invention relates to a composition (composition of the present invention) comprising:
a 5'-capped polynucleotide containing an arbitrary base sequence A; and/or
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure. That is, the 5'-capped polynucleotide and the single-stranded RNA when introduced or added are not necessarily bound to each other, and the 5'-capped polynucleotide and the single-stranded RNA may be introduced (or added) alone in a state in which they are not bound to each other. Alternatively, either one of the 5'-capped polynucleotide or the single-stranded RNA may be introduced and then bound to the 5'-capped polynucleotide or the single-stranded RNA endogenous to the subject or the reaction system.

In a particularly preferred embodiment of the present invention, the composition of the present invention preferably comprises the 5'-capped polynucleotide or the single-stranded RNA (i.e., only either one).

The composition of the present invention can be a pharmaceutical or a reagent. When the composition is a pharmaceutical, the composition can be used as, for example, a vaccine (for example, a vaccine against a microbial infection), or an agent for detecting the 5'-capped polynucleotide endogenous to a cell or a living organism, such as a lncRNA or an mRNA. The composition may optionally further contain other components. The other components are not specifically limited as long as they are pharmaceutically acceptable components, and examples include bases, carriers, solvents, dispersants, emulsifiers, buffers, stabilizers, excipients, binders, disintegrants, lubricants, thickeners, humectants, colorants, fragrances, and chelating agents. The composition may also be in the form of a kit in which two or more substances are contained in two or more separate containers.

Examples of the subject to which the composition of the present invention is applied include, but are not specifically limited to, mammalian animals, such as humans, monkeys, mice, rats, dogs, cats, rabbits, pigs, horses, cows, sheep, goats, and deer. Examples of the cell include animal cells. The type of cells is also not specifically limited, and examples include blood cells, hematopoietic stem cells/progenitor cells, gametes (spermatozoa, oocytes), fibroblasts, epithelial cells, vascular endothelial cells, nerve cells, hepatocytes, keratinocytes, muscle cells, epidermal cells, endocrine cells, ES cells, iPS cells, tissue stem cells, and cancer cells.

The composition of the present invention can take any dosage form, and examples of dosage forms include oral formulation forms, such as tablets (including orally disintegrating tablets, chewable tablets, effervescent tablets, lozenges, and jelly-like drops), pills, granules, fine granules, powders, hard capsules, soft capsules, dry syrups, liquids (including drinks, suspensions, and syrups), and jelly formulations; and parenteral formulation forms, such as injectable formulations (for example, drip injections (such as formulations for intravenous drip infusion), intravenous injections, intramuscular injections, subcutaneous injections, and intradermal injections), topical agents (for example, ointments, plasters, and lotions), suppositories, inhalants, ophthalmic formulations, ophthalmic ointments, nasal drops, and ear drops. Moreover, the active ingredient can be administered in a state complexed with particles (for example, lipid particles or exosomes), or in a state encapsulated in the particles.

The administration route of the composition of the present invention is not specifically limited as long as the desired effect can be obtained, and examples include oral administration; parenteral administration including enteral administration, such as tube-feeding and enema administration, intravenous administration, intraarterial administration, intramuscular administration, intracardiac administration, subcutaneous administration, intradermal administration, intraperitoneal administration, and nasal administration.

The content of the active ingredient in the composition of the present invention depends on the mode of use, the subject of application, the condition of the subject of application, and the like, and is not limited; for example, the content may be 0.0001 to 100% by weight, and preferably 0.001 to 50% by weight.

The dosage of the composition of the present invention for administration to an animal is not specifically limited as long as it is a pharmaceutically effective amount; usually, the dosage is 0.01 to 100 mg/kg body weight, and preferably 0.05 to 50 mg/kg body weight, per day in terms of the weight of the active ingredient. The dosage described above can also be increased or decreased as appropriate, depending on the age, disease state, symptoms, and the like.

The method of the present invention is characterized in that the 5'-capped polynucleotide and/or the single-stranded RNA has a translation efficiency enhancement structure.

The translation efficiency enhancement structure is preferably a structure for stabilizing the binding between the 5'-capped polynucleotide and the single-stranded RNA. The structure is not specifically limited as long as it can stabilize the binding between the 5'-capped polynucleotide and the single-stranded RNA. Examples of this structure include requirements (b) and (c) described below.

In addition to these structures, a structure in which a nucleotide constituting the base sequence A of the 5'-capped polynucleotide and/or the base sequence B of the single-stranded RNA is a modified nucleotide that further stabilizes the duplex formation between the base sequences A and B, as compared to the structure in which the nucleotide is a standard nucleotide (phosphodiester linkage, ribose/deoxyribose, basic nucleobase (A, T, G, C)) also corresponds to the stabilizing structure described above. A known nucleotide can be used as such a modified nucleotide, and examples include a 2' Ome-modified nucleotide at the sugar moiety, and an LNA. The number of such modified nucleotides is preferably 30% or more, more preferably 50% or more, still more preferably 70% or more, even more preferably 80% or more, particularly preferably 90% or more, and still particularly preferably 100%, relative to 100% by number of the nucleotides constituting the base sequence A or B.

The translation efficiency enhancement structure preferably satisfies at least one requirement selected from the group consisting of:
(a) the single-stranded RNA is a circular RNA;
(b) the 5'-capped polynucleotide and/or the single-stranded RNA has a nuclease-binding structure, and the reaction system contains the nuclease; and
(c) the 5'-capped polynucleotide and/or the single-stranded RNA has a structure allowing the 5'-capped polynucleotide and the single-stranded RNA to be covalently linked.

The aspect that satisfies requirement (a) corresponds to Test Example 4 (Fig. 4) described below. The aspect that satisfies requirement (b) corresponds to Test Example 1 (Fig. 1) and Test Example 2 (Fig. 2) described below. The aspect that satisfies requirement (c) corresponds to Test Example 3 (Fig. 3) described below. However, the aspects of the present invention are not limited to the aspects of the test examples described below.

When requirement (a) is satisfied, the circular RNA preferably contains no stop codon. This allows a rolling circle translation to occur, which can further improve the translation efficiency. In this case, the method of the present invention in which the 5'-capped polynucleotide binds to the circular RNA achieves unexpectedly improved translation efficiency, although it was assumed that the presence of the 5'-capped polynucleotide would serve to inhibit translation in the second round of translation by the ribosome.

When requirement (a) is satisfied, other preferred forms are as follows. The base sequence A preferably has a length of 10 to 100 bases, more preferably 12 to 50 bases, and particularly preferably 15 to 30 bases, from the viewpoint of translation efficiency. A base sequence between the base at the 5' end of the base sequence A and the 5' cap preferably has a length of 0 to 100 bases, more preferably 5 to 50 bases, and particularly preferably 10 to 30 bases, from the viewpoint of translation efficiency.

The nuclease in requirement (b) is not specifically limited as long as it is a nucleic acid-binding nuclease and is capable of binding to a target sequence in a sequence-specific manner. Examples of the nuclease include RISC (RNA-induced silencing complex) that functions in the RNA interference mechanism, and Cas that functions in a CRISPR/Cas system. When requirement (b) is satisfied, the nuclease is preferably RISC (corresponding to Test Example 1 (Fig. 1) described below) or Cas (corresponding to Test Example 2 (Fig. 2) described below). However, the aspects of the present invention are not limited to the aspects of the test examples described below. By using the nuclease, the association of the 5'-capped polynucleotide and the single-stranded RNA can be enhanced, and thus the binding between the 5'-capped polynucleotide and the single-stranded RNA can be enhanced, resulting in improved translation efficiency.

The nuclease-binding structure varies depending on the type of nuclease, and can be appropriately selected and designed according to the type of nuclease.

When the nuclease is RISC, the nuclease-binding structure requires that the 5'-capped polynucleotide be a siRNA or a miRNA.

The siRNA preferably has a length of, for example, 18 bases or more, 19 bases or more, 20 bases or more, or 21 bases or more. On the other hand, the siRNA preferably has a length of, for example, 25 bases or less, 24 bases or less, 23 bases or less, or 22 bases or less. It is contemplated that any upper limits and lower limits of the length of siRNA mentioned herein may be combined as desired. For example, contemplated combinations of lengths include a length from a lower limit of 18 bases to an upper limit of 25, 24, 23, or 22 bases; a length from a lower limit of 19 bases to an upper limit of 25, 24, 23, or 22 bases; a length from a lower limit of 20 bases to an upper limit of 25, 24, 23, or 22 bases; and a length from a lower limit of 21 bases to an upper limit of 25, 24, 23, or 22 bases.

The siRNA may be a small hairpin RNA (shRNA). ShRNA can be designed such that a portion thereof forms a stem-loop structure. The siRNA may contain additional bases at the 5' or 3' end. The additional bases are usually about 2 to 4 bases long. The siRNA may have an overhang sequence (overhang) at the 3' end, and specific examples include addition of dTdT (dT represents deoxythymidine). Alternatively, the siRNA may have a blunt end (blunt-ended) without terminal addition.

Rather than cleaving the target mRNA like a siRNA, the miRNA may pair with a 3'-untranslated region (UTR) of the target and inhibit its translation. The miRNA may be a pri-miRNA (primary miRNA), a pre-miRNA (precursor miRNA), or a mature miRNA. The length of the miRNA is not specifically limited; the pri-miRNA usually has a length of several hundred to several thousand bases, the pre-miRNA usually has a length of 50 to 80 bases, and the mature miRNA usually has a length of 18 to 30 bases.

The guide strand of each of the siRNA and miRNA has a 5'-cap structure and contains an arbitrary base sequence A. In order to suppress cleavage of the single-stranded RNA by RISC, the base sequence A preferably has 95% or less identity, and more preferably 90% or less identity, to a base sequence fully complementary to the base sequence B. In this aspect, more specifically, the base sequence A contains 1 to 8, 1 to 6, or 2 to 4 mismatched bases and/or unnatural bases with respect to the base sequence fully complementary to the base sequence B.

When the nuclease is Cas, the nuclease-binding structure contains a tracrRNA sequence in addition to the base sequence A or B of the 5'-capped polynucleotide and/or the single-stranded RNA.

The tracrRNA sequence is not specifically limited. Typically, the tracrRNA is an RNA consisting of a sequence having a length of about 50 to 100 bases that is capable of forming a plurality of (usually, three) stem-loops, and the sequence varies depending on the type of Cas protein used. The tracrRNA sequence is located on the 5' or 3' side of the base sequence A or B depending on the type of Cas protein used. Any of various known sequences can be used as the tracrRNA sequence, depending on the type of Cas protein used.

The Cas protein is not specifically limited as long as it is used in a CRISPR/Cas system. Cas proteins derived from various organisms are known, and examples include Cas9 proteins, Cas12 proteins, Cas13 proteins, and Cpf1 proteins (type V) derived from F. *novicida.* Information on the amino acid sequences of various Cas proteins and their coding sequences can be easily obtained on various databases, such as NCBI.

The Cas protein preferably contains an amino acid mutation to suppress cleavage of the single-stranded RNA. Mutations that eliminate or reduce nuclease activity are known or can be easily designed based on known information.

When requirement (b) is satisfied and the nuclease is Cas, preferably, the 5'-capped polynucleotide is a lncRNA or an mRNA, and the single-stranded RNA contains a tracrRNA sequence. By designing the base sequence B to be complementary to the sequence (base sequence A) in the lncRNA or mRNA, the lncRNA or mRNA can be used as a 5'-capped polynucleotide. When the method of the present invention is carried out in vivo, an endogenous RNA can be used as the lncRNA or mRNA, and, by selecting a lncRNA or an mRNA that is specifically expressed in a specific cell or tissue, a protein encoded by the single-stranded RNA can be expressed in a cell-specific or tissue-specific manner. This can also reduce the side effects and off-target effect. Moreover, by using the protein encoded by the single-stranded RNA as a reporter protein, the specific lncRNA or mRNA can be detected. When Cas is used as the nuclease, a subject containing a polynucleotide containing a Cas-coding sequence is used, the polynucleotide is introduced into the subject, or the polynucleotide is added to the reaction system.

When requirement (b) is satisfied and the nuclease is Cas, a base sequence between the base at the 5' end of the base sequence A and the 5' cap preferably has a length of 3 or more bases, more preferably 7 or more bases, still more preferably 12 or more bases, and even more preferably 20 or more bases, from the viewpoint of translation efficiency. The upper limit of the length in bases is not specifically limited, and is, for example, 100, 60, or 30.

When requirement (b) is satisfied, another preferred form is as follows. The base sequence A preferably has a length of 10 to 100 bases, more preferably 12 to 50 bases, and particularly preferably 15 to 30 bases, from the viewpoint of translation efficiency.

The phrase "structure allowing the 5'-capped polynucleotide and the single-stranded RNA to be covalently linked" in requirement (c) is not specifically limited as long as it is a structure possessed by one or both of the 5'-capped polynucleotide and the single-stranded RNA, and is a structure capable of covalently linking the 5'-capped polynucleotide and the single-stranded RNA. This structure is preferably a photocrosslinkable structure (a structure capable of forming a crosslinked structure by light irradiation). Preferred examples of the photocrosslinkable structure include psoralens, more specifically, 8-methoxypsoralen (methoxsalen) and 4,5',8-trimethylpsoralen (trioxsalen). For example, by using one or both of the 5'-capped polynucleotide and the single-stranded RNA in which a portion of the bases in the base sequence A/B is replaced with the structure described above, a covalent linkage can be formed with an opposing base (for example, uracil) upon complementary binding between the base sequences A and B, by light irradiation (for example, light irradiation at a wavelength of 320 to 380 nm). Through the formation of the covalent linkage, the effect of improving the translation efficiency by the 5'-capped polynucleotide can be more effectively demonstrated.

In one aspect of the present invention, the protein or peptide produced by the method of the present invention can be purified from the reaction system by or according to a known method.

### 1a. Method 2 for Producing or Expressing a Protein or a Peptide

This section corresponds to Items 8 to 10 described above and Test Examples 6 to 8 (Figs. 8 to 11) described below. However, the aspects of the present invention are not limited to the aspects of the test examples described below.

In one aspect, the present invention also relates to a method for producing or expressing a protein or a peptide, the method comprising carrying out a translation reaction in a reaction system comprising a single-stranded circular RNA comprising a 5'-capped polynucleotide linked thereto via a linker.

For the 5'-capped polynucleotide, reference can be made to the description in "1. Method 1 for Producing or Expressing a Protein or a Peptide" above, except that the limitation that the 5'-capped polynucleotide contains the base sequence A is not included.

The base sequence contained in the 5'-capped polynucleotide may have a length of, for example, 1 to 500 bases, preferably 2 to 100 bases, more preferably 2 to 50 bases, still more preferably 2 to 20 bases, even more preferably 2 to 10 bases, and particularly preferably 3 to 6 bases, although not specifically limited thereto.

The single-stranded circular RNA contains a protein-coding or peptide-coding sequence. For the single-stranded RNA, reference can be made to the description in "1. Method 1 for Producing or Expressing a Protein or a Peptide" above, except that the limitation that the single-stranded RNA contains the base sequence B is not included.

The length in bases of the single-stranded circular RNA is not specifically limited. The single-stranded circular RNA preferably has a relatively short length, for example, 500 or less bases, preferably 300 or less bases, more preferably 250 or less bases, and still more preferably 200 or less bases, from the viewpoint of translation efficiency and the like. In this embodiment, the single-stranded circular RNA does not require an IRES and thus, can be minimized in size, and thus high translation efficiency can be achieved.

In the single-stranded circular RNA, all the nucleotides may be linked via a typical internucleotide linkage, such as a phosphodiester linkage or a phosphorothioate linkage, or a portion of the nucleotides may be linked via a linker. In the latter case, the 5'-capped polynucleotide is preferably linked to the linker.

The linker is not specifically limited as long as it has a flexibly operable structure. The linker may consist of a chain structure, or may contain a chain structure and a ring structure. The chain structure may be linear or branched. For example, when the chain structure is a linear chain containing two ends (denoted as ends a and b for convenience), end a may be linked to a particular nucleotide (preferably a terminal nucleotide) of linear single-stranded RNA, end b may be linked to the 5'-capped polynucleotide (preferably the 3' end of the 5'-capped polynucleotide), and another nucleotide (preferably the other terminal nucleotide different from the terminal nucleotide mentioned above) of the linear single-stranded RNA may be linked to any site c of the linker structure that links ends a and b. In this example, end a, end b, and site c are interchangeable. As another example, when the chain structure is a branched chain containing three ends (denoted as ends A to C for convenience), ends A and B may link nucleotides (preferably the nucleotides at both ends) of linear single-stranded RNA, and end C may be linked to the 5'-capped polynucleotide (preferably the 3' end of the 5'-capped polynucleotide). These forms are also preferred from the viewpoint of translation efficiency.

More specifically, the linker is preferably a hydrocarbon chain in which at least one carbon atom constituting the main chain is optionally replaced with a heteroatom and/or a linking structure. When the at least one carbon atom constituting the main chain is replaced with a heteroatom and/or a linking structure, the number of replaced carbon atoms is, for example, 1 to 5.

The hydrocarbon chain is preferably an alkylene group, for example. The alkylene group may be either linear or branched, but is preferably linear. The number of carbon atoms constituting the main chain of the alkylene group is preferably 6 or more. The number of carbon atoms is more preferably 10 or more, and still more preferably 12 or more, while the number of carbon atoms is preferably 100 or less, more preferably 50 or less, and still more preferably 20 or less.

Examples of the heteroatom include oxygen, sulfur, and nitrogen atoms. When at least one carbon atom constituting the main chain of the alkylene group is replaced with an oxygen atom, specifically, -CH₂- in the main chain of the alkylene group is replaced with -O-, for example. When at least one carbon atom constituting the main chain of the alkylene group is replaced with a sulfur atom, specifically, -CH₂- in the main chain of the alkylene group is replaced with -S-, -S(=O)₂-, or -S(=O)-, for example. When at least one carbon atom constituting the main chain of the alkylene group is replaced with a nitrogen atom, specifically, -CH₂- in the main chain of the alkylene group is replaced with -NR- (R represents a hydrogen atom or a hydrocarbon group (preferably an alkyl group, and more preferably an alkyl group having 1 to 8 carbon atoms)), for example. The linking structure is not specifically limited as long as it is a divalent group that is a bonding structure formed by the reaction of two identical or different reactive groups. Examples of the reactive groups include amino, carboxy, hydroxy, ketone, ethynyl, vinyl, azide, epoxy, aldehyde, oxylamino, thiol, isocyanate, and isothiocyanate groups. Examples of reactions between the reactive groups are as follows:
It is known that an amino group reacts with a carboxy group (or a group resulting from esterification of the carboxy group with N-hydroxysuccinimide (NHS)) to form an amide linkage. It is known that an ethynyl group undergoes a 1,3-dipolar cycloaddition reaction with an azide group to form a 1,2,3-triazole ring. It is known that cyclooctyne undergoes a 1,3-dipolar cycloaddition reaction with an azide group to form a 1,2,3-triazole ring. An amino group reacts with a carboxy group to form an amide linkage. It is known that a carboxy group reacts with a hydroxy group to form an ester linkage. It is known that a carboxy group reacts with a thiol group to form a thioester linkage. It is known that a phosphate group reacts with a hydroxy group to form a phosphodiester linkage. A vinyl group reacts with a thiol group to form a linkage. An epoxy group reacts with an amino or thiol group to form a linkage. An aldehyde group reacts with an amino group to form a Schiff base, which upon reduction forms a linkage. An oxylamino group reacts with a ketone or aldehyde group to form an oxime.

The single-stranded circular RNA comprising a 5'-capped polynucleotide linked thereto via a linker can be obtained by chemical synthesis.

Except for the description above, reference can be made to the description in "1. Method 1 for Producing or Expressing a Protein or a Peptide" above.

### 2. Method for Producing 5'-Capped Polynucleotide

This section corresponds to Items 11 to 18 described above and Test Example 5 (Figs. 5 to 7) described below. However, the aspects of the present invention are not limited to the aspect of the test example described below.

In one aspect, the present invention relates to a method for producing a 5'-capped polynucleotide (sometimes also referred to herein as the "method of the present invention") comprising:
carrying out a transcription reaction in a reaction system comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side)-Y1-Y2-Y3, wherein Y1 and Y2 each represent a base in the base-inserted mutant sequence, and Y3 represents a base at the transcription initiation site; and
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2. This method will be described below.

The phage promoter is not specifically limited, and examples include T7 promoters, SP6 promoters, and T3 promoters. Of these, a T7 promoter is particularly preferred from the viewpoint of capping efficacy.

The phage promoter contains a base-inserted mutant sequence adjacent upstream of a transcription initiation site. That is, the phage promoter used in the method of the present invention is a mutant promoter of a wild-type phage promoter (for example, a T7, SP6, or T3 promoter) obtained by introducing a base insertion mutation adjacent upstream of the transcription initiation site. The transcription initiation site of the wild-type phage promoter can be easily determined based on known information.

In the double-stranded polynucleotide used in the method of the present invention, the antisense strand contains the base sequence Y: (upstream side)-Y2-Y3 or (upstream side)-Y1-Y2-Y3. In the base sequence Y, Y1 and Y2 each represent a base in the base-inserted mutant sequence, and Y3 represents a base at the transcription initiation site.

The double-stranded polynucleotide contains a coding sequence of the 5'-capped polynucleotide to be produced downstream of the phage promoter, i.e., contains the base sequence of the 5'-capped polynucleotide in the sense strand. The double-stranded polynucleotide is particularly preferably DNA.

The length in bases of the double-stranded polynucleotide is not specifically limited as long as it can be used as a template for transcription, and is, for example, 25 to 10000 bases, 25 to 5000 bases, 25 to 3000 bases, 25 to 2000 bases, 25 to 1000 bases, 25 to 500 bases, 25 to 300 bases, or 25 to 200 bases.

The 5'-cap analog is not specifically limited as long as it is a single-stranded polynucleotide containing a 5'-cap structure added thereto and can be used for the production of a 5'-capped polynucleotide.

The 5'-cap structure is as described above. The 5'-cap structure can contain a hydrophobic purification tag that can be removed with light (for example, Non-patent Literature 1). According to the method of the present invention, it is possible to produce a 5'-capped polynucleotide with high purity without using the hydrophobic purification tag, and also to produce a 5'-capped polynucleotide with higher purity by using the hydrophobic purification tag.

The polynucleotide of the 5'-cap analog preferably has a length of 2 to 5 bases, more preferably 2 to 4 bases, and particularly preferably 3 bases.

The polynucleotide of the 5'-cap analog contains the base sequence X: (cap side)-X2-X3 or (cap side)-X1-X2-X3. In the base sequence X, X3 is preferably the 3' end of the polynucleotide.

In the method of the present invention,
X3 is a base complementary to Y3, and
X1 is a base complementary to Y1, and/or X2 is a base complementary to Y2.

The capping efficiency can be improved by using the phage promoter having the base-inserted mutant sequence and the complementary relationship as described above. Particularly preferably, an antisense strand of the phage promoter contains the base sequence Y: (upstream side)-Y1-Y2-Y3, wherein X1 is a base complementary to Y1, and X2 is a base complementary to Y2, from the viewpoint of capping efficacy.

Taking ϕ 6.5, XY-inserted in Fig. 5 a) as an example, "XYG" in the 5'-cap analog (m7G-XYG) is the base sequence X (X1-X2-X3), the lower strand of the duplex below is the antisense strand, and "XYC" in the antisense strand corresponds to the base sequence Y (Y1-Y2-Y3).

The specific sequence of the base sequence X is not specifically limited, but is particularly preferably (cap side)-CUG, CAG, GUG, or GAG, and Y3 is C, from the viewpoint of the capping efficiency, production efficiency of the 5'-capped polynucleotide, and the like.

Moreover, the specific sequence of the base sequence X is particularly preferably (cap side)-UUG, UCG, UAG, CUG, CAG, AUG, or AAG, and Y3 is C, or (cap side)-UUA, GUA, CUA, or AUA, and Y3 is U/T, from the viewpoint of the translation efficiency of the 5'-capped polynucleotide to be produced.

The double-stranded polynucleotide and the 5'-cap analog can be produced by or according to known methods for producing polynucleotides and 5'-cap analogs. For the 5'-cap analog, more specifically, reference can be made to, for example, the synthesis methods in the Examples section provided below.

The reaction system is not specifically limited as long as it contains substances necessary for the translation reaction (for example, an RNA polymerase, various nucleoside triphosphates, and the like), and may be either an in vitro or in vivo reaction system.

In the present invention, it was found that the cap analog incorporation efficiency can be further improved by using a T7 RNA polymerase comprising a D130W mutation (Fig. 7). Therefore, in one aspect, the present invention relates to a T7 RNA polymerase comprising a D130W mutation. The term "D130W" refers to a mutation of the 130th amino acid D (aspartic acid) from the N-terminus to W (tryptophan) in the amino acid sequence of the T7 RNA polymerase
(MNTINIAKNDFSDIELAAIPFNTLADHYGERLAREQLALEHESYEMGEARFRKMFERQLKAGEV ADNAAAKPLITTLLPKMIARINDWFEEVKAKRGKRPTAFQFLQEIKPEAVAYITIKTTLACLTSA DNTTVQAVASAIGRAIEDEARFGRIRDLEAKHFKKNVEEQLNKRVGHVYKKAFMQVVEADMLSKG LLGGEAWSSWHKEDSIHVGVRCIEMLIESTGMVSLHRQNAGVVGQDSETIELAPEYAEAIATRAG ALAGISPMFQPCVVPPKPWTGITGGGYWANGRRPLALVRTHSKKALMRYEDVYMPEVYKAINIAQ NTAWKINKKVLAVANVITKWKHCPVEDIPAIEREELPMKPEDIDMNPEALTAWKRAAAAVYRKDR ARKSRRISLEFMLEQANKFANHKAIWFPYNMDWRGRVYAVSMFNPQGNDMTKGLLTLAKGKPIGK EGYYWLKIHGANCAGVDKVPFPERIKFIEENHENIMACAKSPLENTWWAEQDSPFCFLAFCFEYA GVQHHGLSYNCSLPLAFDGSCSGIQHFSAMLRDEVGGRAVNLLPSETVQDIYGIVAKKVNEILQA DAINGTDNEVVTVTDENTGEISEKVKLGTKALAGQWLAHGVTRSVTKRSVMTLAYGSKEFGFRQQ VLEDTIQPAIDSGKGPMFTQPNQAAGYMAKLIWESVSVTVVAAVEAMNWLKSAAKLLAAEVKDKK TGEILRKRCAVHWVTPDGFPVWQEYKKPIQTRLNLMFLGQFRLQPTINTNKDSEIDAHKQESGIA PNFVHSQDGSHLRKTVVWAHEKYGIESFALIHDSFGTIPADAANLFKAVRETMVDTYESCDVLAD FYDQFADQLHESQLDKMPALPAKGNLNLRDILESDFAFA (SEQ ID NO: 1)). The mutant T7 RNA polymerase can contain other amino acid mutations as long as the cap analog incorporation efficacy is not significantly impaired, and the number of such mutations is, for example, 80 or less, 50 or less, 30 or less, 20 or less, 10 or less, 5 or less, 2 or less, or 1 or less. The mutant T7 RNA polymerase may contain another amino acid sequence (for example, a sequence having an independent domain structure, such as a tag sequence or a fluorescent protein), as long as the cap analog incorporation efficacy is not significantly impaired.

The reaction conditions of the transcription reaction are also not specifically limited, and can be set to appropriate conditions depending on the enzyme used and the like.

The method of the present invention can be carried out by adding the double-stranded polynucleotide and/or the 5'-cap analog to the reaction system. Therefore, in one aspect, the present invention relates to a composition comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side)-Y1-Y2-Y3, wherein Y1 and Y2 each represent a base in the base-inserted mutant sequence, and Y3 represents a base at the transcription initiation site; and/or
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2.

The composition can be a pharmaceutical or a reagent. The composition may optionally further contain other components. The other components are not specifically limited as long as they are pharmaceutically acceptable components, and examples include bases, carriers, solvents, dispersants, emulsifiers, buffers, stabilizers, excipients, binders, disintegrants, lubricants, thickeners, humectants, colorants, fragrances, and chelating agents. The composition may also be in the form of a kit in which two or more substances are contained in two or more separate containers.

### Examples

The present invention will be described below in detail based on examples; however, the present invention is not limited to these examples.

### Test Example 1. Phenomenon of enhanced translation reaction of circular mRNA utilizing the RNA interference mechanism

A conceptual diagram of this experiment is shown in Fig. 1(a).

The base sequence of the circular RNA used in this experiment is as follows.
Circular Nluc mRNA (620-nt)
5' _GGCGCAUAUUAAGGUGACGCGUGUGGCCUCGAACACCGAGCGACCCUGCAGCGACCCGCUUA AAAGCUUGGCAAUCCGGUACUGUUGGUAAAGCCACCAUGGUCUUCACACUCGAAGAUUUCGUUGG GGACUGGCGACAGACAGCCGGCUACAACCUGGACCAAGUCCUUGAACAGGGAGGUGUGUCCAGUU UGUUUCAGAAUCUCGGGGUGUCCGUAACUCCGAUCCAAAGGAUUGUCCUGAGCGGUGAAAAUGGG CUGAAGAUCGACAUCCAUGUCAUCAUCCCGUAUGAAGGUCUGAGCGGCGACCAAAUGGGCCAGAU CGAAAAAAUUUUUAAGGUGGUGUACCCUGUGGAUGAUCAUCACUUUAAGGUGAUCCUGCACUAUG GCACACUGGUAAUCGACGGGGUUACGCCGAACAUGAUCGACUAUUUCGGACGGCCGUAUGAAGGC AUCGCCGUGUUCGACGGCAAAAAGAUCACUGUAACAGGGACCCUGUGGAACGGCAACAAAAUUAU CGACGAGCGCCUGAUCAACCCCGACGGCUCCCUGCUGUUCCGAGUAACCAUCAACGGAGUGACCG GCUGGCGGCUGUGCGAACGCAUUCUGGCGUAAUUCUAG_3' (SEQ ID NO: 6) (The 5' and 3' ends are linked).

Circular RNA was prepared as follows. First, 5'-end phosphorylated linear RNA as a pre-circularization starting material was prepared by a transcription reaction of the following composition: 5 ng/µL dsDNA (PCR product containing T7 promoter), 2 mM ATP, 2 mM UTP, 2 mM CTP, 2 mM GTP, 10 mM GMP, 40 mM Tris-HCl (pH of 8.0), 8 mM MgCl₂, 2 mM spermidine, 5 mM DTT, 0.002 U/µL pyrophosphatase, 4.7 ng/µL T7 RNA polymerase. The reaction mixture was incubated at 37°C for 2 hours, DNase I (Takara Bio Inc.) was then added to a final concentration of 0.1 U/µL, and the mixture was incubated at 37°C for 15 minutes. The reaction mixture was extracted with a mixture of equal amounts of TE-saturated phenol and chloroform and deproteinated, and then the transcribed RNA was recovered by alcohol precipitation. The resulting transcribed RNA as a crude product was purified by reversed-phase HPLC as previously reported (Nat. Commun., 14, 2657, 2023). The resulting 5'-end phosphorylated transcribed RNA was circularized by ligating the 5' and 3' ends using T4 RNA ligase 2. The composition of the ligase reaction mixture was as follows: 0.5 µM 5'-phosphorylated RNA, 1 µM template DNA oligo (5' AATATGCGCCCTAGAATTAC 3' (SEQ ID NO: 7)), 10% PEG8000, 50 mM Tris-HCl (pH of 7.5), 2 mM magnesium chloride, 1 mM DTT, 400 µM ATP, 0.1 µg/µL T4 RNA ligase 2. The reaction mixture was incubated at 37°C for 1 hour and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis.

The sequences of the RNA oligonucleotides used in this experiment are as follows.
Passenger strand
   5' GACGCGUGUCUACUCGAACAUA 3' (SEQ ID NO: 8)
Uncapped guide strand
   5' p-UGUUCGAGUAGACACGCGUCAC-p 3' (SEQ ID NO: 9) Capped guide strand
   5' p-UGUUCGAGUAGACACGCGUCAC(3')ppp(5')m⁷G 3' (SEQ ID NO: 10).

A capped RNA oligonucleotide was prepared as previously reported (ACS Chem Biol 2022, 17, 1308-1314). A 5'-phosphorylated RNA oligonucleotide sequence was synthesized using a commercially available phosphoramidite compound (manufactured by ChemGenes) with an automated nucleic acid synthesizer NTS T-8-A20-R8NC (manufactured by Nihon Techno Service Co., Ltd.). After completion of the synthesis, a 1:1 mixture of 40% aqueous methylamine solution and 28% concentrated aqueous ammonia was added to the CPG solid support, and the mixture was incubated at 65°C for 60 minutes to cleave the oligonucleotide from the support and deprotect the phosphate and base moieties. The mixture was evaporated to dryness under reduced pressure, a 1 M TBAF/THF solution was added and the mixture was incubated overnight at room temperature, and then desalted using a NAP-25 gel filtration column (Cytiva) and alcohol-precipitated to obtain an RNA oligonucleotide as a precipitate. The oligonucleotide was further purified by reversed-phase HPLC. The resulting 5'-phosphorylated RNA oligonucleotide was reacted with m7GDP imidazolide (Im-m7GDP) to introduce a capped structure at the 5' end (ACS Chem Biol 2022, 17, 1308-1314). The progress of the capping reaction was confirmed by gel shift of the RNA band by denaturing PAGE and molecular weight measurement using LC-MS analysis. The passenger strand and the uncapped guide strand or capped guide strand were mixed in a buffer (10 mM Tris-HCl (pH of 7.5), 50 mM NaCl, 1 mM EDTA (pH of 8.0)) to a final concentration of 1 µM, and the mixture was heated at 80°C for 5 minutes and then slowly cooled to prepare a 1 µM double-stranded RNA oligonucleotide solution.

The effect of the capped double-stranded RNA oligonucleotide on the translation reaction of circular Nluc mRNA was evaluated using a human-derived cultured cell strain HeLa (Riken Cell Bank, RCB0007). HeLa cells were cultured in DMEM (Wako Pure Chemical Industries, Ltd.) containing 10% FBS (Thermo Fisher) at a CO₂ concentration of 5% and 37°C. The HeLa cells were seeded in a 96-well multiwell plate for cell culture at 1.5 × 10E4 cells/well on the day before transfection. The circular Nluc mRNA (30 ng/well) and the double-stranded RNA oligonucleotide (0.3 pmol/well, 1.5 pmol/well, or 3 pmol/well) were separately mixed with Lipofectamine MessengerMAX (0.15 µL) and 10 µL of Opti-MEM^{™} I Reduced Serum Medium (Thermo Fisher). These mixtures were added to the wells containing the HeLa cells in which the growth medium had been replaced with Opti-MEM^{™} I Reduced Serum Medium (20 µL) to transfect the cells with the RNA. After 3 hours, the supernatant was replaced with the growth medium, and after culturing for a total of 6 hours, the amount of the Nluc protein contained in the cell lysate was evaluated using the Nano-Glo (registered trademark) Luciferase Assay System (Promega). The TriStar5 multimode plate reader (Berthold) was used for measuring the amount of chemiluminescence.

The results are shown in Fig. 1. It was demonstrated that the translation reaction can be enhanced by using a system to which RISC can bind.

### Test Example 2. Phenomenon of enhanced translation of circular mRNA utilizing hybridization with long non-coding RNA (lncRNA) and stabilization thereof by dCas13 protein

A conceptual diagram of this experiment is shown in Figs. 2(a) and (c).

The base sequences of the RNAs used in this experiment are as follows.
HULC RNA (500-nt): 
5'_GAUGGGGGUGGAACUCAUGAUGGAAUUGGAGCCUUUACAAGGGAAUGAAGAGACAAGAGCUC
UCUUUAUGCCACGUGAGGAUACAGCAAGGCCCCAAUCUGCAAGCCAGGAAGAGUCGUCACGAGAA
CCAGACCAUGCAGGAACUCUGAUCGUGGACAUUUCAACCUCCAGAACUGUGAUCCAAAAUGCAUA
UGUAUCUUUGGAAGAAACUCUGAAGUAAAGGCCGGAAUAUUCUUUGUUUAAAACAUUAAAAACAA
AACAGACCAAAGCAUCAAGCAAGAAGUUUCCUGGCAAUAAACUAAGCACAGCAUUAUUUUUUAAG
GAACACAAAUUAAGUGUUCAACCUGUGGCAAAUUUGUACUUUCUCCCUGAAUUAUGUUGUUAUCA
AAGAAAAAAAUUGGGAAGCAUGGCAAAAUAUCAUCAAAACUGAAACUAGAAUUAAACAAAACUAA
AUUAAAAUGAAAUAAAAUGAUGUCCAUUCUUAAAAAAAAAAAAAAAAAAA_3' (SEQ ID NO:
11).
UCA1 RNA (1678 nt): 
5'_GUGACAUUCUUCUGGACAAUGAGUCCCAUCAUCUCUCCACCAUGCACCUUGUGACUCCCUCC
UCUGCUGACAACAGAUAACCACCUUUAACUGUAACUUUCCACAGCCUACCCCAGCCCUAUAAAGC
UGCCCCUCUCCUAUCUCCCUUCGCUGACUCUCUUUUCAGACUCAGCCCACUUGCACCCAAGUGAA
UUAACAGCCUUGUUGCUCACACAAAGCCUGUUUAGGUGGUCUUCUAUACGGACAUGCUUGACACU
UGGUGCCAAAAUCUGGGCCAGGGGGACUCCUUCGUGAGACCGGCCCCCUGUCCUGGCCCUCAUUC
CGUGAAGAGAUCCACCUGCGACCUCGGGUCCUCAGACCAGCCCAAGGAACAUCUCACCAAUUUCA
AAUCGGAUCUCCUCGGCUUAGUGGCUGAAGACUGAUGCUGCCCGAUCGCCUCAGAAGCCCCUUGG
ACCAUCACAGAUGCCGAGCUUCGGGUAACUCUUACGGUGGAGGGAUCUGCAAUCAGAACUAUUGA
ACUUCUCCAUUCAGACCGCCACUCACACCUAUGGGAAAAGGGUAAUGUAUCAUCGGCUUAGCAAC
AGGGAAUACUAUUCGUAUGAUGGAAAAUGGGGACAAAAGGCUUUGGUACAUAAAACAUUAUUCCU
 
UCCUUGGCCUAAAAACUCAUCGCCACCUACAUUAAAGCUAAUAUGCCUGAUUACUGUUUUUAGAG
AACUUAUUUUAUUAGGGCAGUUCCAAGCUCAAAAAUACGCUAACUGGCACCUUGUUAGCUACAUA
AAAAUGCACCCUAGACCCGAAACUUACUAGACUCAUUAUAAAAUUUUCUUUAAGGUGUCCACGCA
GUCCCUGGUCACACUUGAAGCAGUCCGGAGAAAUAUCAGCCCUACCCCAGUAAUCCCCAGAAGGA
ACUUACACUUUUUUUUAAUCUUUUCCUACAACUUCAUAUUUUAUAAAUAAAAAGACAAAAAUGUC
AGGCCUGUGAGCUGAAGCUUAGCCAUUGUAACCCCUGUGACCUGCACAUAUCCGUCCAGGUGGCC
UGCAGGAGCCAAGAAGUCUGGAGCAGCCGAAAAACCACAAAGAAGUGAAACAGCCAGUUCCUGCC
UUAACUAAUUAACCCACCUUACGACAUUCCACCAUUAUGACUUGUCCACCAUUAUGACUUGUUCC
UGCCCUGCCCCAACUGAUCAAUCAACCCUGUGACAUUCUUCUCCUGGACAAUGAGUCCCAUCAUC
UCUCCACCAUGCACCUUGUGACCCCCUCCUCUGCUGAGGAUAACCACCUUUAACUGUAACUUUCC
ACGCCUACCCAAGCCCUAUAAAGCUGCCCCUCUCCUAUCUCCCUUCACUGACUCUCUUUUCGGAC
UCAGCCCACUUGCACCCAAGUGAAUUAACAGCCUUGUUGCUCACACAAAGCCUGAUUGGGUGUCU
UCUAUACGGACACGCGUGACAGGAACCUCAACCCAAAGGCAGUCUGAUGAGGUGUCUAAGAUAAA
AGUAGCGGCACAAAGGCUUUUGUAAACAGAGGCGUUUCAUGUGGUUUUCCUUUCCUUUCCUUAUA
UGUGAAAAGGUGACAGAAAAGAAAUCUUCCUAAAAGAGUCAGCCACUGCUCUGUGGAACGAUGAU
CAUUUAAAUGCCACGUCGAUUUGAAACGUACAACAAAGGGACUAACAGUUGAGAACC_3' (SEQ
ID NO: 56).
dRfxCas13d mRNA (3184-nt): 
5'_ACAUUUGCUUCUGACACAACUGUGUUCACUAGCAACCUCAAACAGACACCAUGGGCGUGAAG
UCCACACUCGUGUCCGGCUCCAAAGUGUACAUGACAACCUUCGCCGAAGGCAGCGACGCCAGGCU
GGAAAAGAUCGUGGAGGGCGACAGCAUCAGGAGCGUGAAUGAGGGCGAGGCCUUCAGCGCUGAAA
UGGCCGAUAAAAACGCCGGCUAUAAGAUCGGCAACGCCAAAUUCAGCCAUCCUAAGGGCUACGCC
GUGGUGGCUAACAACCCUCUGUAUACAGGACCCGUCCAGCAGGAUAUGCUCGGCCUGAAGGAAAC
UCUGGAAAAGAGGUACUUCGGCGAGAGCGCUGAUGGCAAUGACAAUAUUUGUAUCCAGGUGAUCC
AUAACAUCCUGGACAUUGAAAAAAUCCUCGCCGAAUACAUUACCAACGCCGCCUACGCCGUCAAC
AAUAUCUCCGGCCUGGAUAAGGACAUUAUUGGAUUCGGCAAGUUCUCCACAGUGUAUACCUACGA
CGAAUUCAAAGACCCCGAGCACCAUAGGGCCGCUUUCAACAAUAACGAUAAGCUCAUCAACGCCA
UCAAGGCCCAGUAUGACGAGUUCGACAACUUCCUCGAUAACCCCAGACUCGGCUAUUUCGGCCAG
GCCUUUUUCAGCAAGGAGGGCAGAAAUUACAUCAUCAAUUACGGCAACGAAUGCUAUGACAUUCU
GGCCCUCCUGAGCGGACUGGCGCACUGGGUGGUCGCUAACAACGAAGAAGAGUCCAGGAUCUCCA
GGACCUGGCUCUACAACCUCGAUAAGAACCUCGACAACGAAUACAUCUCCACCCUCAACUACCUC
UACGACAGGAUCACCAAUGAGCUGACCAACUCCUUCUCCAAGAACUCCGCCGCCAACGUGAACUA
UAUUGCCGAAACUCUGGGAAUCAACCCUGCCGAAUUCGCCGAACAAUAUUUCAGAUUCAGCAUUA
UGAAAGAGCAGAAAAACCUCGGAUUCAAUAUCACCAAGCUCAGGGAAGUGAUGCUGGACAGGAAG
 
GAUAUGUCCGAGAUCAGGAAAAAUCAUAAGGUGUUCGACUCCAUCAGGACCAAGGUCUACACCAU
GAUGGACUUUGUGAUUUAUAGGUAUUACAUCGAAGAGGAUGCCAAGGUGGCUGCCGCCAAUAAGU
CCCUCCCCGAUAAUGAGAAGUCCCUGAGCGAGAAGGAUAUCUUUGUGAUUAACCUGAGGGGCUCC
UUCAACGACGACCAGAAGGAUGCCCUCUACUACGAUGAAGCUAAUAGAAUUUGGAGAAAGCUCGA
AAAUAUCAUGCACAACAUCAAGGAAUUUAGGGGAAACAAGACAAGAGAGUAUAAGAAGAAGGACG
CCCCUAGACUGCCCAGAAUCCUGCCCGCUGGCCGUGAUGUUUCCGCCUUCAGCAAACUCAUGUAU
GCCCUGACCAUGUUCCUGGAUGGCAAGGAGAUCAACGACCUCCUGACCACCCUGAUUAAUAAAUU
CGAUAACAUCCAGAGCUUCCUGAAGGUGAUGCCUCUCAUCGGAGUCAACGCUAAGUUCGUGGAGG
AAUACGCCUUUUUCAAAGACUCCGCCAAGAUCGCCGAUGAGCUGAGGCUGAUCAAGUCCUUCGCU
AGAAUGGGAGAACCUAUUGCCGAUGCCAGGAGGGCCAUGUAUAUCGACGCCAUCCGUAUUUUAGG
AACCAACCUGUCCUAUGAUGAGCUCAAGGCCCUCGCCGACACCUUUUCCCUGGACGAGAACGGAA
ACAAGCUCAAGAAAGGCAAGCACGGCAUGAGAAAUUUCAUUAUUAAUAACGUGAUCAGCAAUAAA
AGGUUCCACUACCUGAUCAGAUACGGUGAUCCUGCCCACCUCCAUGAGAUCGCCAAAAACGAGGC
CGUGGUGAAGUUCGUGCUCGGCAGGAUCGCUGACAUCCAGAAAAAACAGGGCCAGAACGGCAAGA
ACCAGAUCGACAGGUACUACGAAACUUGUAUCGGAAAGGAUAAGGGCAAGAGCGUGAGCGAAAAG
GUGGACGCUCUCACAAAGAUCAUCACCGGAAUGAACUACGACCAAUUCGACAAGAAAAGGAGCGU
CAUUGAGGACACCGGCAGGGAAAACGCCGAGAGGGAGAAGUUUAAAAAGAUCAUCAGCCUGUACC
UCACCGUGAUCUACCACAUCCUCAAGAAUAUUGUCAAUAUCAACGCCAGGUACGUCAUCGGAUUC
CAUUGCGUCGAGCGUGAUGCUCAACUGUACAAGGAGAAAGGCUACGACAUCAAUCUCAAGAAACU
GGAAGAGAAGGGAUUCAGCUCCGUCACCAAGCUCUGCGCUGGCAUUGAUGAAACUGCCCCCGAUA
AGAGAAAGGACGUGGAAAAGGAGAUGGCUGAAAGAGCCAAGGAGAGCAUUGACAGCCUCGAGAGC
GCCAACCCCAAGCUGUAUGCCAAUUACAUCAAAUACAGCGACGAGAAGAAAGCCGAGGAGUUCAC
CAGGCAGAUUAACAGGGAGAAGGCCAAAACCGCCCUGAACGCCUACCUGAGGAACACCAAGUGGA
AUGUGAUCAUCAGGGAGGACCUCCUGAGAAUUGACAACAAGACAUGUACCCUGUUCGCAAACAAG
GCCGUCGCCCUGGAAGUGGCCAGGUAUGUCCACGCCUAUAUCAACGACAUUGCCGAGGUCAAUUC
CUACUUCCAACUGUACCAUUACAUCAUGCAGAGAAUUAUCAUGAAUGAGAGGUACGAGAAAAGCA
GCGGAAAGGUGUCCGAGUACUUCGACGCUGUGAAUGACGAGAAGAAGUACAACGAUAGGCUCCUG
AAACUGCUGUGUGUGCCUUUCGGCUACUGUAUCCCCAGGUUUAAGAACCUGAGCAUCGAGGCCCU
GUUCGAUAGGAACGAGGCCGCCAAGUUCGACAAGGAGAAAAAGAAGGUGUCCGGCAAUUCCACCG
UGGAACGGAUCAUUCAGGCCCUGCCUCCACUUGAAAGACUGACACUGUAAGCUCGCUUUCUUGCU
GUCCAAUUUCUAUUAAAGGUUCCUUUGUUCCCUAAGUCCAACUACUAAACUGGGGGAUAUUAUGA
AGGGCCUUGAGCAUCUGGAUUCUGCCUAAUAAAAAACAUUUAUUUUCAUUGCAAAAAAAAAAAAA
AAAAAAAAAAAAAAAAAAAAAAAAAAAAAAAAAAAAAAAAAAAAAAAAAAAAAAAAAAAAAAAAA
AA_3' (SEQ ID NO: 12).
Linear Nluc mRNA (705-nt) with a 5-base-long linker:
   5' _GGUCCCAGGUCCACUUCAAGUAAACCCCUACCAACUGGUCGGGGUUUGAAACCCAAUUCCAU CAUGAGUUCCACCGCGUGUGGCCUCGAACACCGAGCGACCCUGCAGCGACCCGCUUAAAAGCUUG GCAAUCCGGUACUGUUGGUAAAGCCACCAUGGUCUUCACACUCGAAGAUUUCGUUGGGGACUGGC GACAGACAGCCGGCUACAACCUGGACCAAGUCCUUGAACAGGGAGGUGUGUCCAGUUUGUUUCAG AAUCUCGGGGUGUCCGUAACUCCGAUCCAAAGGAUUGUCCUGAGCGGUGAAAAUGGGCUGAAGAU CGACAUCCAUGUCAUCAUCCCGUAUGAAGGUCUGAGCGGCGACCAAAUGGGCCAGAUCGAAAAAA UUUUUAAGGUGGUGUACCCUGUGGAUGAUCAUCACUUUAAGGUGAUCCUGCACUAUGGCACACUG GUAAUCGACGGGGUUACGCCGAACAUGAUCGACUAUUUCGGACGGCCGUAUGAAGGCAUCGCCGU GUUCGACGGCAAAAAGAUCACUGUAACAGGGACCCUGUGGAACGGCAACAAAAUUAUCGACGAGC GCCUGAUCAACCCCGACGGCUCCCUGCUGUUCCGAGUAACCAUCAACGGAGUGACCGGCUGGCGG CUGUGCGAACGCAUUCUGGCGUAAUUCUAAAAAAAAAAAAAAAAAAAAAAAAAAAAAA_3' (SEQ ID NO: 13) (complementary sequence to HULC is underlined).
Linear Nluc mRNA (705-nt) with a 10-base-long linker:
   5' _GGUCCCAGGUCCACUUCAAGUAAACCCCUACCAACUGGUCGGGGUUUGAAACAGGCUCCAAU UCCAUCAUGAGUUGCGUGUGGCCUCGAACACCGAGCGACCCUGCAGCGACCCGCUUAAAAGCUUG GCAAUCCGGUACUGUUGGUAAAGCCACCAUGGUCUUCACACUCGAAGAUUUCGUUGGGGACUGGC GACAGACAGCCGGCUACAACCUGGACCAAGUCCUUGAACAGGGAGGUGUGUCCAGUUUGUUUCAG AAUCUCGGGGUGUCCGUAACUCCGAUCCAAAGGAUUGUCCUGAGCGGUGAAAAUGGGCUGAAGAU CGACAUCCAUGUCAUCAUCCCGUAUGAAGGUCUGAGCGGCGACCAAAUGGGCCAGAUCGAAAAAA UUUUUAAGGUGGUGUACCCUGUGGAUGAUCAUCACUUUAAGGUGAUCCUGCACUAUGGCACACUG GUAAUCGACGGGGUUACGCCGAACAUGAUCGACUAUUUCGGACGGCCGUAUGAAGGCAUCGCCGU GUUCGACGGCAAAAAGAUCACUGUAACAGGGACCCUGUGGAACGGCAACAAAAUUAUCGACGAGC GCCUGAUCAACCCCGACGGCUCCCUGCUGUUCCGAGUAACCAUCAACGGAGUGACCGGCUGGCGG CUGUGCGAACGCAUUCUGGCGUAAUUCU_3' (SEQ ID NO: 14) (complementary sequence to HULC is underlined).
Linear and circular Nluc mRNAs (705-nt) with a 15-base-long linker:
   5' _GGUCCCAGGUCCACUUCAAGUAAACCCCUACCAACUGGUCGGGGUUUGAAACUGUAAAGGCU CCAAUUCCAUCAUGCGUGUGGCCUCGAACACCGAGCGACCCUGCAGCGACCCGCUUAAAAGCUUG GCAAUCCGGUACUGUUGGUAAAGCCACCAUGGUCUUCACACUCGAAGAUUUCGUUGGGGACUGGC GACAGACAGCCGGCUACAACCUGGACCAAGUCCUUGAACAGGGAGGUGUGUCCAGUUUGUUUCAG AAUCUCGGGGUGUCCGUAACUCCGAUCCAAAGGAUUGUCCUGAGCGGUGAAAAUGGGCUGAAGAU CGACAUCCAUGUCAUCAUCCCGUAUGAAGGUCUGAGCGGCGACCAAAUGGGCCAGAUCGAAAAAA UUUUUAAGGUGGUGUACCCUGUGGAUGAUCAUCACUUUAAGGUGAUCCUGCACUAUGGCACACUG GUAAUCGACGGGGUUACGCCGAACAUGAUCGACUAUUUCGGACGGCCGUAUGAAGGCAUCGCCGU GUUCGACGGCAAAAAGAUCACUGUAACAGGGACCCUGUGGAACGGCAACAAAAUUAUCGACGAGC GCCUGAUCAACCCCGACGGCUCCCUGCUGUUCCGAGUAACCAUCAACGGAGUGACCGGCUGGCGG CUGUGCGAACGCAUUCUGGCGUAAUUCU_3' (SEQ ID NO: 15) (complementary sequence to HULC is underlined; the 5' and 3' ends of the circular Nluc mRNA are linked).
Linear and circular Nluc mRNAs (705-nt) with a 15-base-long linker:
   5' _GGUCCCAGGUCCACUUCAAGUAAACCCCUACCAACUGGUCGGGGUUUGAAACUGGAGAGAUG AUGGGACUCAUUGGCGUGUGGCCUCGAACACCGAGCGACCCUGCAGCGACCCGCUUAAAAGCUUG GCAAUCCGGUACUGUUGGUAAAGCCACCAUGGUCUUCACACUCGAAGAUUUCGUUGGGGACUGGC GACAGACAGCCGGCUACAACCUGGACCAAGUCCUUGAACAGGGAGGUGUGUCCAGUUUGUUUCAG AAUCUCGGGGUGUCCGUAACUCCGAUCCAAAGGAUUGUCCUGAGCGGUGAAAAUGGGCUGAAGAU CGACAUCCAUGUCAUCAUCCCGUAUGAAGGUCUGAGCGGCGACCAAAUGGGCCAGAUCGAAAAAA UUUUUAAGGUGGUGUACCCUGUGGAUGAUCAUCACUUUAAGGUGAUCCUGCACUAUGGCACACUG GUAAUCGACGGGGUUACGCCGAACAUGAUCGACUAUUUCGGACGGCCGUAUGAAGGCAUCGCCGU GUUCGACGGCAAAAAGAUCACUGUAACAGGGACCCUGUGGAACGGCAACAAAAUUAUCGACGAGC GCCUGAUCAACCCCGACGGCUCCCUGCUGUUCCGAGUAACCAUCAACGGAGUGACCGGCUGGCGG CUGUGCGAACGCAUUCUGGCGUAAUUCU_3' (SEQ ID NO: 57) (complementary sequence to UCA1 is underlined; the 5' and 3' ends of the circular Nluc mRNA are linked).

Each RNA was synthesized by transcription using DNA encoding the corresponding sequence as a template and T7 RNA polymerase. The composition of the transcription reaction was as follows: 10 ng/µL dsDNA (PCR product containing T7 promoter), 2 mM ATP, 2 mM UTP, 2 mM CTP, 2 mM GTP, 40 mM Tris-HCl (pH of 8.0), 8 mM MgCl₂, 2 mM spermidine, 5 mM DTT, 0.002 U/µL pyrophosphatase (New England Biolabs), 2 U/µL T7 RNA polymerase (Takara Bio Inc). The reaction mixture was incubated at 37°C for 2 hours. In the preparation of HULC RNA, dRfxCas13d mRNA, and UCA1 RNA, a hydrophobic tag-containing cap analog compound DiPure (Nat. Commun. 14, 2657, 2023) was added to a final concentration of 2 mM, and capped mRNAs were isolated and purified as previously reported. After the transcription reaction, DNase I (Takara Bio Inc) was added to a final concentration of 0.1 U/µL, and the mixture was incubated at 37°C for 15 minutes. An aqueous 7.5 M lithium chloride solution was added to the reaction mixture to give a final concentration of 2.5 M, and the mixture was cooled at -30°C for 30 minutes and then centrifuged (20,000Xg, 20 min) to recover the transcribed RNA as a precipitate. The resulting transcribed RNA as a crude product was purified by reversed-phase HPLC as previously reported (Nat. Commun., 14, 2657, 2023). Circular Nluc mRNA was prepared by ligating the 5' and 3'ends of 5'-phosphorylated RNA, which was synthesized by transcription by adding GMP to the transcription reaction mixture described above to a final concentration of 10 mM, using T4 RNA ligase 2 in the presence of a template DNA oligonucleotide (30-nt, 5'AGTGGACCTGGGACCTTTTTTTTTTTTTTT3' (SEQ ID NO: 16)) complementary to both ends of the RNA. The composition of the ligase reaction mixture was as follows: 1 µM 5'-phosphorylated RNA, 2 µM DNA oligonucleotide, 10% PEG8000, 50 mM Tris-HCl (pH of 7.5), 2 mM MgCl₂, 1 mM DTT, 400 µM ATP, 0.1 µg/µL T4 RNA ligase 2. The reaction mixture was incubated at 37°C for 1 hour and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis (Sci. Rep., 5, 16435, 2015).

An experiment evaluating the expression level of the Nluc protein was carried out using a human-derived cultured cell strain HeLa (Riken Cell Bank, RCB0007). HeLa cells were cultured in DMEM (Wako Pure Chemical Industries, Ltd.) containing 10% FBS (Thermo Fisher) at a CO₂ concentration of 5% and 37°C. The HeLa cells were seeded in a 96-well multiwell plate at 1 × 10E4 cells/well on the day before transfection.

dCasRNA (20 ng/well), HULC RNA (160 ng/well), and linear Nluc RNA (20 ng/well) were mixed with 0.015 µL/well, 0.12 µL/well, or 0.015 µL/well, respectively, of Lipofectamine MessengerMAX (Thermo Fisher), the mixtures were incubated for 5 minutes and then added to the wells containing the HeLa cells in which the growth medium had been replaced with Opti-MEM^{™} I Reduced Serum Medium (Thermo Fisher) to transfect the cells with the RNA. After 4 hours, the supernatant was replaced with the growth medium, and, after culturing for a total of 24 hours, the expression level of Nluc in the cells was evaluated using the Nano-Glo (registered trademark) Luciferase Assay System (Promega). The TriStar5 multimode plate reader (Berthold) was used for measuring the amount of chemiluminescence. In the experiment using circular Nluc mRNA, 0.1 µM HULC lncRNA and 0.2 µM Nluc RNA were annealed in TE buffer containing 50 mM NaCl, the annealed product was similarly transfected into the HeLa cells using Lipofectamine MessengerMAX, and, after 4.5 hours, the expression level of Nluc was measured using the Nano-Glo (registered trademark) Luciferase Assay System.

In addition, an experiment evaluating the expression level of the Nluc protein was carried out using a human-derived cultured cell strain HeLa (Riken Cell Bank, RCB0007). HeLa cells were cultured in DMEM (Wako Pure Chemical Industries, Ltd.) containing 10% FBS (Thermo Fisher) at a CO₂ concentration of 5% and 37°C. The HeLa cells were seeded in a 96-well multiwell plate at 1 × 10E4 cells/well on the day before transfection. In TE buffer containing 50 mM NaCl, 0.1 µM Nluc RNA and 0.2 µM HULC RNA or UCA1 RNA were mixed, and hybridization was carried out by slow cooling from heating. For each well, 1 µL of the RNA hybridization product and 0.15 µL of Lipofectamine MessengerMAX (Thermo Fisher) were prepared and were each diluted with 5 µL of Opti-MEM (trademark) I Reduced Serum Medium (Thermo Fisher) and then mixed, and the mixture was treated at room temperature for 5 minutes to form a liposome. The medium in the 96-well multiplate containing the HeLa cells prepared on the previous day was replaced with fresh DMEM, and then the liposome describe above was added to transfect the cells with the RNA. After 4.5 hours, Nluc expression levels in the cells were evaluated using the Nano-Glo (registered trademark) Luciferase Assay System (Promega). The TriStar5 multimode plate reader (Berthold) was used for measuring the amount of chemiluminescence.

The results are shown in Fig. 2. It was demonstrated that the translation reaction can be enhanced by the hybridization with lncRNA and also using a system to which the Cas protein can bind.

### Test Example 3. Phenomenon in which a photocrosslinkable capped RNA probe enhances the translation reaction of circular mRNA in a manner dependent on light irradiation

A conceptual diagram of this experiment is shown in Fig. 3(a).

The sequences of the chemically synthesized RNAs used in this experiment are as follows.
Linear 24-nt RNA
   5' _AAAAGGCGCAUAUUAAGGUGACGC_3' (SEQ ID NO: 17)
Probe 1 (50-nt RNA)
   5' _m7G-ppp-GCGUCAXCCUUAAUAUGCGCCAAAAAAAAAAAAAAAAAAAAAAAAAAAAAA_3 ' (SEQ ID NO: 18)
Probe 2 (50-nt RNA)
   5' _m7G-ppp-GCGUCACCUUAAUAXUGCGCCAAAAAAAAAAAAAAAAAAAAAAAAAAAAAA_3 ' (SEQ ID NO: 19)
Probe 3 (50-nt RNA)
   5'_m₂^{7,2'O}G-ppp-
   GCGUCACCUUAAUAXUGCGCCAAAAAAAAAAAAAAAAAAAAAAAAAAAAAA_3' (SEQ ID NO: 20)
Probe 4 (50-nt RNA)
   5' _m⁷G-ppp-GCGUCACCUUAAUAUGCGCCAAAAAAAAAAAAAAAAAAAAAAAAAAAAAA_3' (SEQ ID NO: 21)

X contained in each of the sequences of the probes 1 and 2 represents an inserted trioxsalen structure (derived from reagent CLP-6644 manufactured by ChemGenes).

The sequence of the circular RNA used in this experiment is as follows.
Circular Nluc mRNA (650-nt)
5' _GGCGCAUAUUAAGGUGACGCGUGUGGCCUCGAACACCGAGCGACCCUGCAGCGACCCGCUUA AAAGCUUGGCAAUCCGGUACUGUUGGUAAAGCCACCAUGGUCUUCACACUCGAAGAUUUCGUUGG GGACUGGCGACAGACAGCCGGCUACAACCUGGACCAAGUCCUUGAACAGGGAGGUGUGUCCAGUU UGUUUCAGAAUCUCGGGGUGUCCGUAACUCCGAUCCAAAGGAUUGUCCUGAGCGGUGAAAAUGGG CUGAAGAUCGACAUCCAUGUCAUCAUCCCGUAUGAAGGUCUGAGCGGCGACCAAAUGGGCCAGAU CGAAAAAAUUUUUAAGGUGGUGUACCCUGUGGAUGAUCAUCACUUUAAGGUGAUCCUGCACUAUG GCACACUGGUAAUCGACGGGGUUACGCCGAACAUGAUCGACUAUUUCGGACGGCCGUAUGAAGGC AUCGCCGUGUUCGACGGCAAAAAGAUCACUGUAACAGGGACCCUGUGGAACGGCAACAAAAUUAU CGACGAGCGCCUGAUCAACCCCGACGGCUCCCUGCUGUUCCGAGUAACCAUCAACGGAGUGACCG GCUGGCGGCUGUGCGAACGCAUUCUGGCGUAAUUCUAGAAAAAAAAAAAAAAAAAAAAAAAAAA AAA_3' (SEQ ID NO: 22) (The 5' and 3' ends are linked).

An unmodified RNA oligonucleotide and trioxsalen-containing capped RNA oligonucleotides were prepared as previously reported (ACS Chem Biol 2022, 17, 1308-1314). A 5'-phosphorylated RNA oligonucleotide sequence was synthesized using a commercially available phosphoramidite compound (manufactured by ChemGenes) with an automated nucleic acid synthesizer NTS T-8-A20-R8NC (manufactured by Nihon Techno Service Co., Ltd.). (5'-phosphate group CLP-1544; trioxsalen structure, CLP-6644). After completion of the synthesis, a 1:1 mixture of 40% aqueous methylamine solution and 28% concentrated aqueous ammonia was added to the CPG solid support, and the mixture was incubated at 65°C for 15 minutes to cleave the oligonucleotide from the support and deprotect the phosphate and base moieties. The mixture was evaporated to dryness under reduced pressure, a 1 M TBAF/THF solution was added and the mixture was incubated overnight at room temperature, and then desalted using a NAP-25 gel filtration column (Cytiva) and alcohol-precipitated to obtain an RNA oligonucleotide as a precipitate. The oligonucleotide was further purified by reversed-phase HPLC. The resulting 5'-phosphorylated RNA oligonucleotide was reacted with m7GDP imidazolide (Im-m7GDP) to introduce a capped structure at the 5' end (ACS Chem Biol 2022, 17, 1308-1314). The progress of the capping reaction was confirmed by gel shift of the RNA band by denaturing PAGE and molecular weight measurement using LC-MS analysis.

The circular RNA used in this experiment was prepared as follows. First, 5'-end phosphorylated linear RNA as a pre-circularization starting material was prepared by a transcription reaction of the following composition: 5 ng/µL dsDNA (PCR product containing T7 promoter), 2 mM ATP, 2 mM UTP, 2 mM CTP, 2 mM GTP, 10 mM GMP, 40 mM Tris-HCl (pH of 8.0), 8 mM MgCl₂, 2 mM spermidine, 5 mM DTT, 0.002 U/µL pyrophosphatase, 4.7 ng/µL T7 RNA polymerase. The reaction mixture was incubated at 37°C for 2 hours, DNase I (Takara Bio Inc.) was then added to a final concentration of 0.1 U/µL, and the mixture was incubated at 37°C for 15 minutes. The reaction mixture was extracted with a mixture of equal amounts of TE-saturated phenol and chloroform and deproteinated, and then the transcribed RNA was recovered by alcohol precipitation. The resulting transcribed RNA as a crude product was purified by reversed-phase HPLC as previously reported (Nat. Commun., 14, 2657, 2023). The resulting 5'-end phosphorylated transcribed RNA was circularized by ligating the 5' and 3' ends using T4 RNA ligase 2. The composition of the ligase reaction mixture was as follows: 0.5 µM 5'-phosphorylated RNA, 1 µM template DNA oligo (5' ACCTTAATATGCGCCTTTTTTTTTTTTTTT 3' (SEQ ID NO: 23)), 10% PEG8000, 50 mM Tris-HCl (pH of 7.5), 2 mM magnesium chloride, 1 mM DTT, 400 µM ATP, 0.1 µg/µL T4 RNA ligase 2. The reaction mixture was incubated at 37°C for 1 hour and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis.

A crosslinking experiment of probe 1 or 2 to the linear 24-nt RNA was carried out as follows. A mixed solution of the RNA and probe (1 µM linear 24-nt RNA, 3, 15, or 30 µM probe 1 or probe 2, 10 mM Tris-HCl (pH of 7.5), 50 mM NaCl (pH of 7.5), 1 mM EDTA (pH of 8.0)) was prepared, heated at 90°C for 3 minutes, and then slowly cooled to room temperature. The solution was subsequently irradiated with 365-nm light using a 300 W xenon light source MAX-350 at an intensity of 4.0 mW/cm² for 30 minutes under ice cooling. The reaction solution was analyzed by 20% denaturing PAGE to investigate the efficiency of the crosslinking reaction. The RNA band was stained with SYBR^{™} Green II Nucleic Acid Gel Stain (Takara Bio Inc), imaged on a ChemiDoc MP imager (Bio-Rad) and visualized.

The circular Nluc mRNA and the photocrosslinkable probe (probe 3) or non-crosslinkable probe (probe 4) were heated in a mixed solution (8.8 µL: containing 25 ng/µL circular Nluc mRNA, 0, 0.35 or 1.75 µ probe 3 or probe 4, 10 mM Tris-HCl (pH of 7.5), 50 mM NaCl (pH of 7.5), and 1 mM EDTA (pH of 8.0)) at 90°C for 3 minutes, and then slowly cooled to room temperature. The solution was subsequently irradiated with 365-nm light using a 300 W xenon light source MAX-350 at an intensity of 4.0 mW/cm² for 30 minutes under ice cooling. The resulting solution was transfected into HeLa cells, seeded on a 96-well multiwell plate at 1.0 × 10⁴ cells/well on the previous day, at 10 ng mRNA/well using the Lipofectamine (registered trademark) Messenger MAX^{™} Reagent, and the cells were cultured for a total of 24 hours and then lysed. The amount of the Nluc protein contained in the cell lysate was evaluated using the Nano-Glo (registered trademark) Luciferase Assay System (Promega). The TriStar5 multimode plate reader (Berthold) was used for measuring the amount of chemiluminescence.

The results are shown in Fig. 3. It was demonstrated that the translation reaction can be enhanced by photocrosslinking.

The above results in Test Examples 1 to 3 demonstrate that, in a particularly preferred embodiment of the composition, the 5'-capped polynucleotide and single-stranded RNA are firmly linked and stabilized by each of RISC, the Cas protein, and photocrosslinking, which allows the translation reaction to be enhanced.

### Test Example 4. Enhanced rolling circle translation reaction by capped RNA

A conceptual diagram of this experiment is shown in Fig. 4a).

The base sequence of the circular RNA used in this experiment is as follows.
Linear/Circular FLAG-EGF (264-nt)
5' _GGGAGCCACCAUGGACUACAAGGACGACGACGACAAGAUCAUCGACUAUAAAGACGACGACG AUAAAGGUGGCGACUAUAAGGACGACGACGACAAAGCCAUCAACAGCGACAGCGAGUGCCCCCUG AGCCACGACGGCUACUGCCUGCACGACGGCGUGUGCAUGUACAUCGAGGCCCUGGACAAGUACGC CUGCAACUGCGUGGUGGGCUACAUCGGCGAGAGAUGCCAGUACAGAGACCUGAAGUGGUGGGAGC UGAGACU_3' (SEQ ID NO: 2) (The 5' and 3' ends of the circular are linked).

The base sequences of the 5'-phosphorylated RNA oligonucleotides and 5'-capped RNA oligonucleotides used in this experiment are as follows.
5'-capped probe 1
   5'_m7G_ppp_GCCACCUCUCUGUACUGGCAUCUCUC_3' (SEQ ID NO: 3) 5'-capped probe 2
5'_m7G_ppp_GACAGUCUGCUCGAAGCGGCCGCUCUAGAAUCUCUGUACUGGCAUCUCUC_3' (SEQ ID NO: 4) 5'-capped probe 3
5'_m7G_ppp_GACAGUCUGCUCGAAGCGGCCGCUCUAGAACCGCGUAUAAUUCCACUGCG_3' (SEQ ID NO: 5).

Circular RNA (Sci Rep 2015, 5, 16435) was prepared as follows. First, 5'-end phosphorylated linear RNA as a pre-circularization starting material was prepared by a transcription reaction of the following composition: 5 ng/µL dsDNA (PCR product containing T7 promoter), 2 mM ATP, 2 mM UTP, 2 mM CTP, 2 mM GTP, 10 mM GMP, 40 mM Tris-HCl (pH of 8.0), 8 mM MgCl₂, 2 mM spermidine, 5 mM DTT, 0.002 U/µL pyrophosphatase, 4.7 ng/µL T7 RNA polymerase. The reaction mixture was incubated at 37°C for 2 hours, DNase I (Takara Bio Inc.) was then added to a final concentration of 0.1 U/µL, and the mixture was incubated at 37°C for 15 minutes. An aqueous 7.5 M lithium chloride solution was added to the reaction mixture to give a final concentration of 2.5 M, and the mixture was cooled at -30°C for 30 minutes and then centrifuged (20,000Xg, 20 min) to recover the transcribed RNA as a precipitate. The resulting transcribed RNA as a crude product was purified by reversed-phase HPLC as previously reported (Nat. Commun., 14, 2657, 2023). The resulting 5'-end phosphorylated transcribed RNA was circularized by ligating the 5' and 3' ends using T4 RNA ligase 2. The composition of the ligase reaction mixture was as follows: 1 µM 5'-phosphorylated RNA, 10% PEG6000, 50 mM Tris-HCl (pH of 7.5), 2 mM MgCl2, 1 mM DTT, 400 µM ATP, 0.1 µg/µL T4 RNA ligase 2. The reaction mixture was incubated at 37°C for 1 hour and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis.

A capped RNA oligonucleotide was prepared as previously reported (ACS Chem Biol 2022, 17, 1308-1314). A 5'-phosphorylated RNA oligonucleotide sequence was synthesized using a commercially available phosphoramidite compound (manufactured by ChemGenes) with an automated nucleic acid synthesizer NR-2A_7MX (manufactured by Nihon Techno Service Co., Ltd.). After completion of the synthesis, a 1:1 mixture of 40% aqueous methylamine solution and 28% concentrated aqueous ammonia was added to the CPG solid support, and the mixture was incubated at 65°C for 30 minutes to cleave the oligonucleotide from the support and deprotect the phosphate and base moieties. The mixture was evaporated to dryness under reduced pressure, a 1 M TBAF/THF solution was added and the mixture was incubated overnight at room temperature to deprotect the TOM protecting group, and then desalted using a NAP-25 gel filtration column (Cytiva) and alcohol-precipitated to obtain an RNA oligonucleotide as a precipitate. The oligonucleotide was further purified by reversed-phase HPLC. The resulting 5'-phosphorylated RNA oligonucleotide was reacted with m7GDP imidazolide (Im-m7GDP) to introduce a capped structure at the 5' end. The capping reaction mixture (20 µM phosphorylated RNA oligonucleotide, 10 mM Im-m7GDP, 1 M 1-methylimidazole, DMSO solution) was incubated at 55°C for 3 hours, then RNA was recovered by alcohol precipitation, and the desired RNA was separated and purified by reversed-phase HPLC. The progress of the capping reaction was confirmed by gel shift of the RNA band by denaturing PAGE and molecular weight measurement using LC-MS analysis [5'-capped probe 1; Calcd 8632.0, Found 8633.06, (+1.1): 5'-capped probe 2; Calcd 16425.7, Found; 16428.8 (+3.1): 5'-capped probe 3; Calcd 16511.8, Found; 16512.0 (+0.2)].

The translation reaction of the circular RNA using rabbit reticulocyte lysate (Promega) was carried out as follows. A translation mixture (2 µM circular RNA, 4 µM RNA probe, 70 (v/v) % Reticulocyte Lysate, Nuclease Treated, 10 µM Amino Acid Mixture Minus Cysteine, 10 µM Amino Acid Mixture Minus Leucine, 0.8 units/µL murine RNase Inhibitor (New England Biolabs)) was incubated at 30°C for 4 hours, and an aliquot (4 µL) taken from the reaction mixture was analyzed by Western blotting. After electrophoresis on 5-20% gradient SDS-polyacrylamide (ATTO Corporation), the protein or peptide was transferred onto an Immobilon-P membrane (Merck) by the semi-dry method, and reacted with anti-FLAG M2 antibody (Sigma-Aldrich) and anti-mouse IgG-HRP conjugate (Sigma-Aldrich) in this order. Chemiluminescence generated by using the SuperSignal^{™} West Femto Maximum Sensitivity Substrate (Thermo Fisher) was imaged on a ChemiDoc MP imager (Bio-Rad).

The results are shown in Fig. 4. In the presence of the capped oligo RNA probes containing a region complementary to the circular RNA sequence, a significant increase in the translation products was confirmed.

### Test Example 5: Development of a highly efficient method of synthesizing Cap-2 mRNAs using PureCap analogs and modified promoters

### Test Example 5-1. Synthesis of PureCap Analogs

PureCap analogs synthesized are shown below.

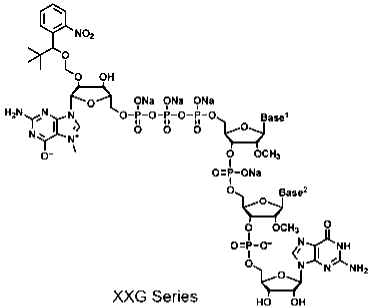

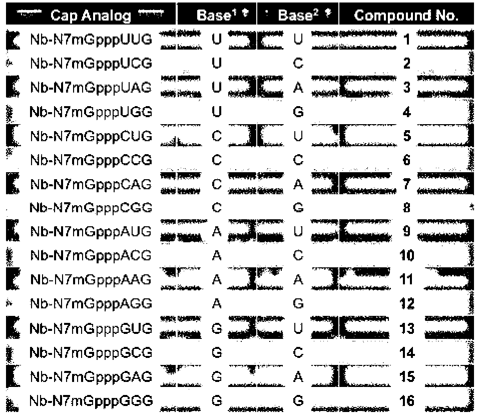

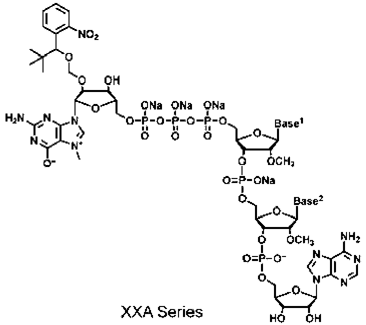

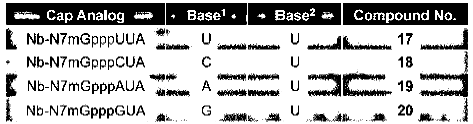

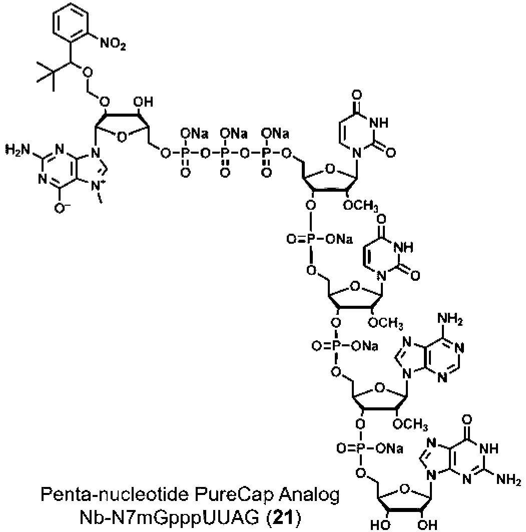

### Liquid-Phase Synthesis of Trinucleotides

### Synthesis Scheme of Trinucleotide, pAGG (33)

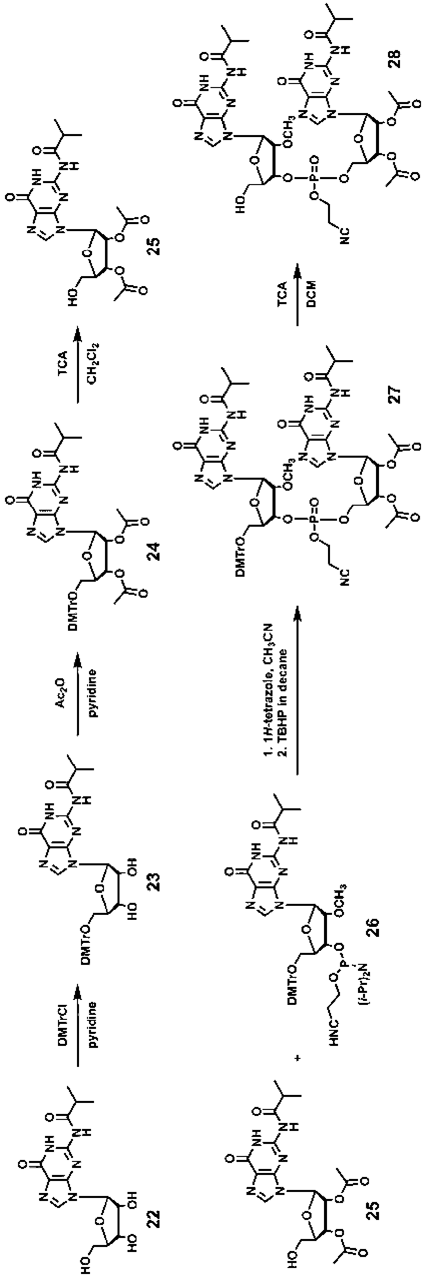

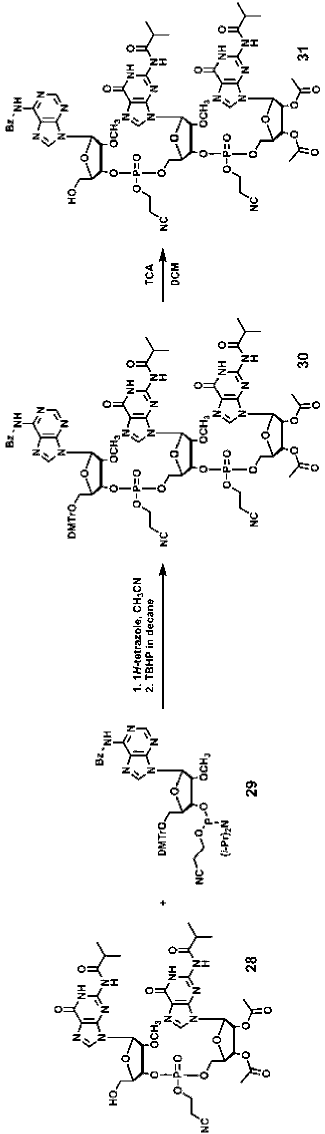

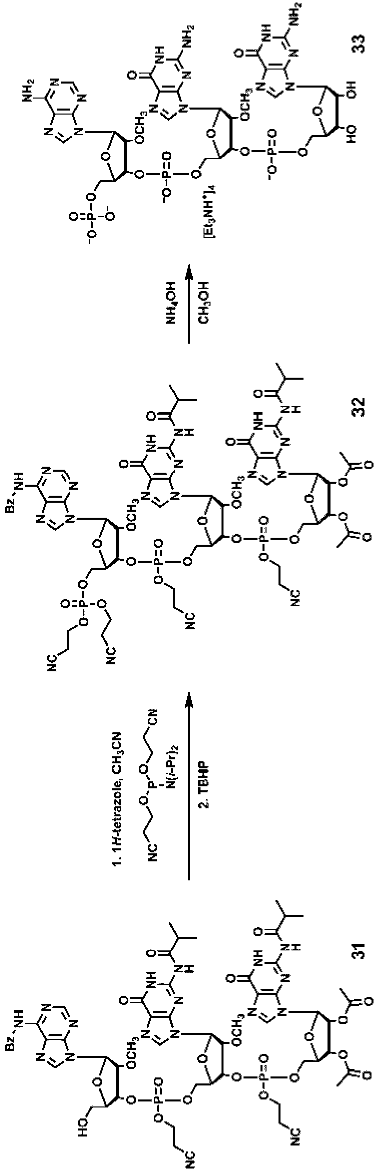

### DMTr Protection, Synthesis of Compound (23)

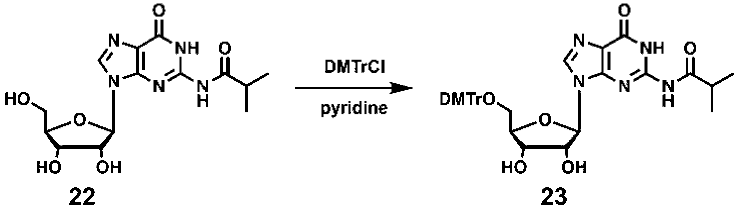

DMTrCl (11.5 g, 33.98 mmol) in pyridine (100 mL) was added to a mixture of N²-isobutyrylguanosine (22) (10.0 g, 28.3 mmol) in pyridine (200 mL). The mixture was stirred at room temperature for 15 hours. The reaction was monitored by TLC and quenched with water after completion of the reaction. Pyridine was removed from the reaction mixture, and the mixture was diluted with water. The mixture was extracted with EtOAc and dried over Na₂SO₄. The solvent was removed on a rotary evaporator, and the residue was purified by silica gel column chromatography using 5% MeOH in DCM (1% TEA) to give compound 23 as a magenta solid (19.0 g, quantitative). ¹H-NMR (400 MHz, DMSO-d₆) δ 11.57 (s, 1H), 8.08 (d, J = 2.0 Hz, 1H), 7.31 (d, J = 7.6 Hz, 2H), 7.25-7.14 (m, 7H), 6.81-6.76 (m, 4H), 5.83 (q, J = 2.2 Hz, 1H), 5.64 (s, 1H), 5.24 (d, J = 15.9 Hz, 1H), 4.50 (t, J = 4.7 Hz, 1H), 4.19-4.00 (m, 2H), 3.68 (s, 6H), 3.25-3.13 (m, 2H), 2.77-2.67 (m, 1H), 2.46 (d, J = 1.8 Hz, 1H), 1.13-1.02 (m, 6H) ppm.

### Acetyl Protection, Synthesis of Compound (24)

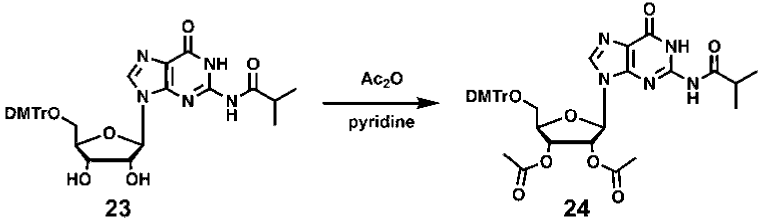

Acetic anhydride (27.4 mL, 290 mmol) was added to a solution of compound 23 (19.0 g, 29.0 mmol) in pyridine (200 mL). The mixture was stirred at room temperature for 15 hours. The reaction was monitored by TLC and quenched with water after completion of the reaction. Pyridine was removed from the reaction mixture, and the mixture was diluted with water. The mixture was extracted with EtOAc and dried over Na₂SO₄. The solvent was removed on a rotary evaporator, and the residue was purified by silica gel column chromatography using 5% MeOH in DCM (1% TEA) to give compound 24 as a light brown solid (23.1 g, quantitative). ¹H-NMR (400 MHz, CDCl₃) δ 7.77 (d, J = 5.6 Hz, 1H), 7.54-7.47 (m, 2H), 7.43-7.29 (m, 4H), 7.25-7.15 (m, 3H), 6.83-6.70 (m, 4H), 6.61-6.51 (m, 1H), 5.97-5.82 (m, 2H), 5.64 (s, 1H), 5.29 (s, 1H), 4.28-4.20 (m, 1H), 3.80-3.69 (m, 6H), 3.63 (q, J = 7.3 Hz, 1H), 3.15-3.07 (m, 1H), 2.14-2.02 (m, 6H), 1.50-1.43 (m, 1H), 0.93-0.82 (m, 3H), 0.64 (td, J = 14.9, 7.0 Hz, 3H) ppm.

### Detritylation, Synthesis of Compound (25)

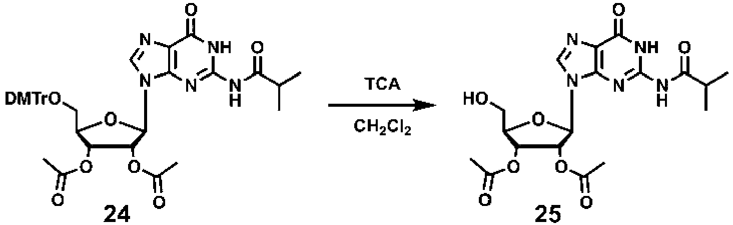

A solution of trichloroacetic acid (25.5 g, 156 mmol) in dichloromethane (78.0 mL) was poured into a solution of compound 24 (23.1 g, 31.2 mmol) in dichloromethane (100 mL) at 0°C. After the addition, the reaction mixture was warmed to room temperature and stirred overnight. After completion of the reaction, methanol (15.0 mL) was added to the reaction mixture to quench the reaction. The mixture was then washed with saturated aqueous NaHCO solution (₃) and extracted with dichloromethane. The combined organic layer was concentrated and purified by silica gel column chromatography using 0-4% MeOH/DCM to give compound 25 as a white solid (3.90 g, 70.0% yield). ¹H-NMR (400 MHz, CDCl₃) δ 8.32 (s, 1H), 7.23-7.11 (m, 2H), 6.19 (d, J = 6.1 Hz, 1H), 5.82 (t, J = 5.8 Hz, 1H), 5.59-5.50 (m, 1H), 4.29 (q, J = 3.1 Hz, 1H), 3.84 (ddd, J = 26.0, 12.3, 3.0 Hz, 2H), 2.77-2.70 (m, 1H), 2.15 (d, J = 8.3 Hz, 3H), 2.02 (d, J = 5.8 Hz, 3H), 1.26-1.22 (m, 6H) ppm.

### Synthesis of Dinucleotide Form G(2'OMe)pG(2'OMe) (28)

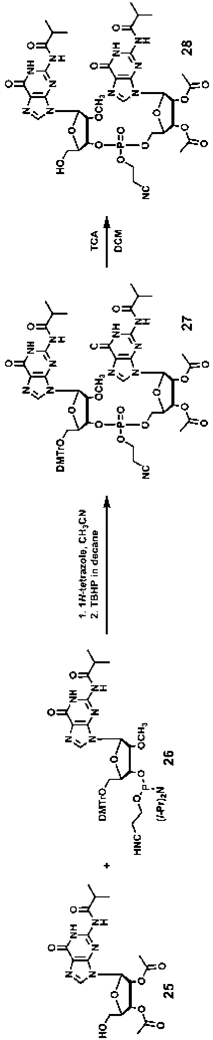

Compound 25 (1.00 g, 2.29 mmol), compound 26 (2.58 g, 2.97 mmol), and molecular sieve 3A (2.60 g) were dissolved in dry acetonitrile (23.0 mL). After stirring for 20 minutes, 1H-tetrazole (722 mg, 10.3 mmol) was added and stirred for 4 hours. After complete conversion of compound 25, 5-6 M TBHP (600 µL, 2.97 mmol) in decane was added to the reaction mixture and stirred for 1 hour. The reaction mixture was then diluted with dichloromethane and washed with saturated aqueous NaHCO₃ and brine. The organic layer was dried over Na₂SO₄, concentrated, and used in the next step without purification. (3.48 g of a white solid) was obtained. Crude product 27 (3.48 g, 2.85 mmol) was dissolved in dichloromethane (32.0 mL) and cooled to 0°C. A solution of trichloroacetic acid (0.2 M, 71.2 mL, 14.2 mmol) in dichloromethane was added to the mixture and stirred at room temperature for 3 hours. The reaction was monitored by TLC, and after complete conversion, the reaction mixture was quenched with a saturated aqueous NaHCO₃ solution, washed twice with a saturated aqueous NaHCO₃ solution, and extracted with dichloromethane. The solvent was removed on a rotary evaporator, and the residue was purified by silica gel column chromatography using 0-5% MeOH/dichloromethane to give compound 28 as a white solid (1.10 g, yield 53.0%, over two steps). ¹H-NMR (400 MHz, DMSO-d₆) δ 12.08-12.05 (m, 2H), 11.62 (s, 1H), 11.54 (d, J = 3.1 Hz, 1H), 8.30-8.26 (m, 1H), 8.24 (s, 1H), 6.09 (d, J = 6.7 Hz, 1H), 5.88-5.84 (m, 1H), 5.77 (t, J = 6.3 Hz, 1H), 5.47 (t, J = 2.9 Hz, 1H), 5.29 (q, J = 5.1 Hz, 1H), 5.09-5.03 (m, 1H), 4.56 (dd, J = 12.6, 7.9 Hz, 1H), 4.43-4.33 (m, 3H), 4.23-4.17 (m, 3H), 3.59-3.49 (m, 2H), 3.28 (dd, J = 10.7, 10.0 Hz, 5H), 2.91 (dt, J = 16.2, 5.4 Hz, 2H), 2.76-2.68 (m, 2H), 2.09 (d, J = 4.9 Hz, 3H), 1.98-1.95 (m, 3H), 1.09-1.06 (m, 12H) ppm.

### Synthesis of Trinucleotide Form A(2'OMe)pG(2'OMe) (31)

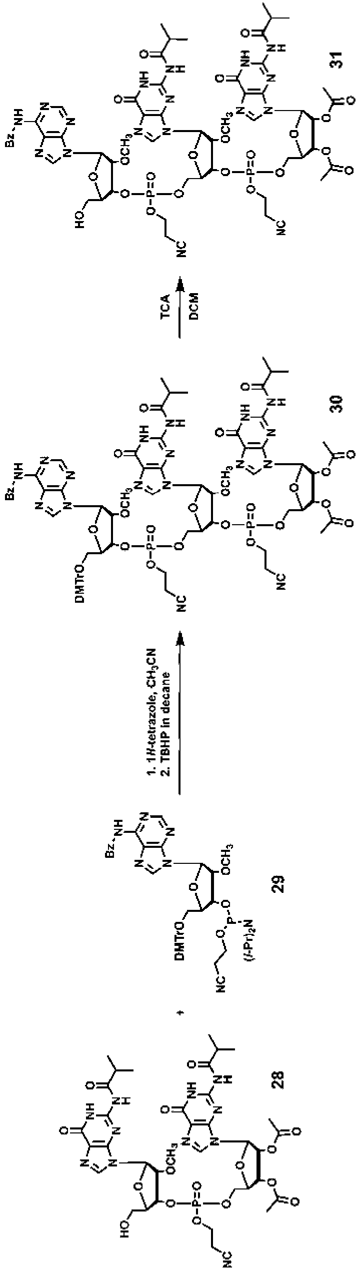

Compound 28 (100 mg, 0.108 mmol), compound 29 (126 mg, 0.140 mmol), and molecular sieve 3A (125 mg) were suspended in dry acetonitrile (1.00 mL). After stirring at room temperature for 20 minutes, 1H-tetrazole (34.0 mg, 0.486 mmol) was added and stirred for 4 hours. After complete conversion of compound 28, 5-6 M TBHP (28.0 µL, 0.140 mmol) was added to the mixture and stirred for 1 hour.

The reaction mixture was thereafter diluted with dichloromethane and washed with saturated aqueous NaHCO₃ and brine. The organic layer was dried over Na₂SO₄, concentrated, and used in the next step without purification. Crude product 30 (276 mg, 0.160 mmol) was dissolved in dichloromethane (3.20 mL) and cooled to 0°C. A solution of trichloroacetic acid (0.2 M, 4.00 mL, 0.800 mmol) in dichloromethane was added to the solution and stirred at room temperature for 3 hours. The reaction was monitored by TLC, and after complete conversion, the reaction mixture was quenched with a saturated aqueous NaHCO₃ solution, washed twice with a saturated aqueous NaHCO₃ solution, and extracted with dichloromethane. The solvent was removed on a rotary evaporator, and the residue was purified by silica gel column chromatography using 0-5% MeOH/dichloromethane to give compound 31 as a white solid (125 mg, yield 81%, over two steps). ¹H-NMR (400 MHz, DMSO-d₆) δ 12.09-12.06 (m, 2H), 11.55 (d, J = 6.1 Hz, 2H), 11.22 (d, J = 6.1 Hz, 1H), 8.71 (dt, J = 11.4, 2.6 Hz, 2H), 8.26-8.23 (m, 2H), 8.01 (d, J = 8.3 Hz, 2H), 7.64-7.50 (m, 3H), 6.17-6.07 (m, 2H), 5.94-5.90 (m, 1H), 5.79-5.74 (m, 1H), 5.47 (dt, J = 9.1, 3.4 Hz, 1H), 5.36 (t, J = 5.4 Hz, 1H), 5.16 (t, J = 11.6 Hz, 2H), 4.82-4.75 (m, 1H), 4.65-4.59 (m, 1H), 4.49-4.18 (m, 11H), 3.60 (t, J = 4.6 Hz, 2H), 3.35-3.26 (m, 28H), 2.95-2.88 (m, 4H), 2.74-2.63 (m, 2H), 2.28 (d, J = 2.0 Hz, 0H), 2.08 (dd, J = 6.1, 1.6 Hz, 3H), 1.98 (s, 3H), 1.09-1.07 (m, 12H) ppm.

### Synthesis of Trinucleotide Form pA(2'OMe)pG(2'OMe) (33)

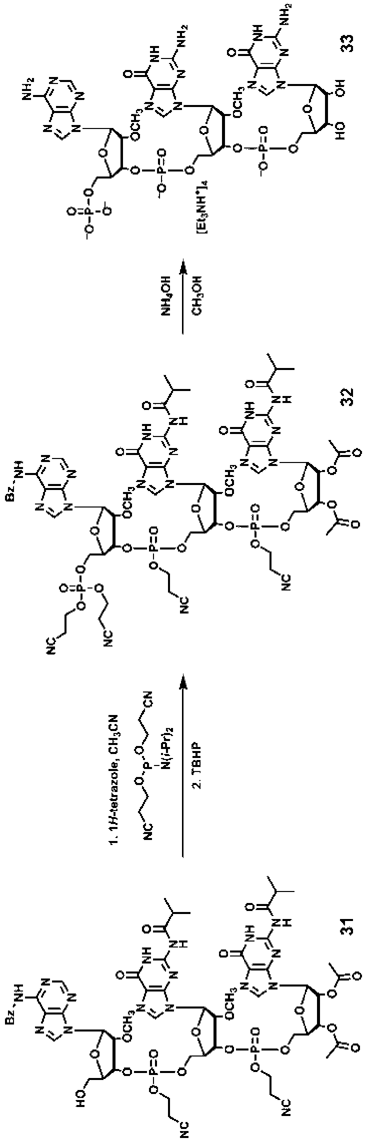

Compound A (30 mg, 0.02 mmol, 1 eq), compound B (22.85 mg, 0.084 mmol, 4 eq) and molecular sieve 3A (30 mg) were dissolved in dry ACN (1 mL). After stirring for 20 minutes, 0.4 M 1H-tetrazole (0.2 mL, 0.086 mmol, 4.1 eq) was added at rt and stirred for 2 hours. After complete conversion of the SM A, 5 M TBHP (6 µL, 0.03 mmol, 1.5 eq) was added and stirred for an additional hour. The reaction product was thereafter diluted with DCM and washed with NaHCO3 and brine. The organic portion was dried over Na₂SO₄, the solvent was removed in vacuum, and the residue was used in the next step. Compound A (44 mg, 0.04 mmol, 1 eqw) was dissolved in MeOH (0.5 mL), and aq NH4OH (1 mL) was added thereto at rt and stirred for 20 hours. After complete conversion of the SM, the reaction mixture was diluted with MeOH and evaporated three times on a rotary evaporator. The crude product was thereafter dissolved in MQ and purified on a DEAE column with a 270 minute linear gradient. After evaporation of the solvent, 5 mg of a pure white solid compound was obtained. ¹H-NMR (392 MHz, DMSO-D6) δ 8.45 (s, 1H), 8.10-7.90 (m, 3H), 7.25 (s, 2H), 6.72 (d, J = 13.7 Hz, 4H), 5.99 (d, J = 6.7 Hz, 1H), 5.80 (d, J = 7.9 Hz, 1H), 5.64 (d, J = 6.3 Hz, 1H), 4.82-4.74 (m, 3H), 4.54-4.42 (m, 5H), 4.30-4.20 (m, 3H), 3.98-3.79 (m, 8H), 3.26-3.17 (m, 6H), 2.81 (d, J = 33.2 Hz, 3H). 31P-NMR (159 MHz, DMSO-D6) δ -0.6 (s, 1P), -1.4 (s, 2P) ppm.

### Synthesis Scheme of Trinucleotide pUGG (38)

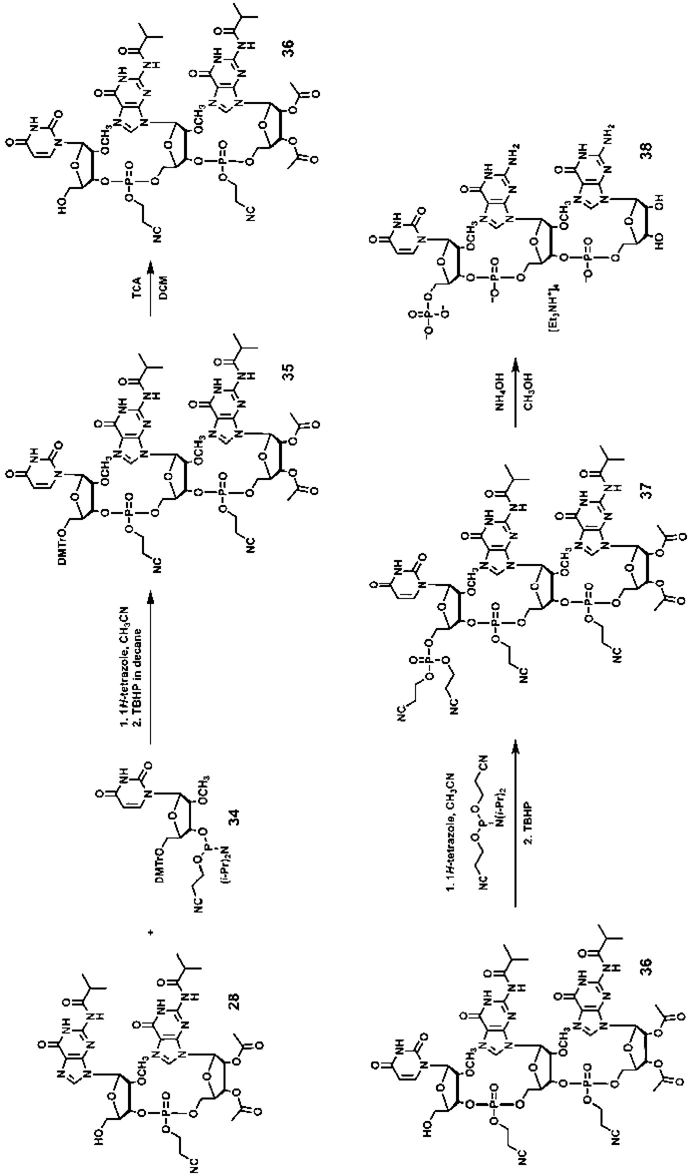

### Synthesis of Trinucleotide Form UGG (36)

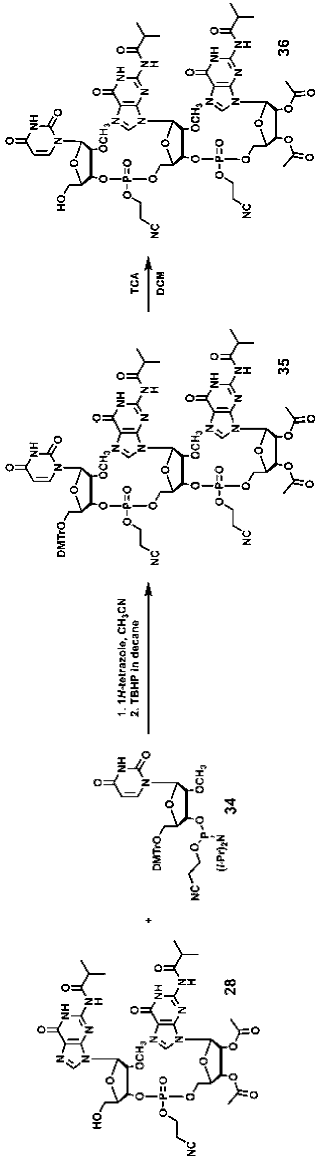

Compound 18-4 (343.3 mg, 0.37 mmol, 1 eq) was co-evaporated with toluene to remove water, then 2'-OMe-U-CE phosphoramidite (368.96 mg, 0.48 mmol, 1.3 eq) was added to the flask. 8 mL of acetonitrile was added to dissolve the compound, and then 180 mg of molecular sieve 3 Å was added. After stirring for 5 minutes, 1H-tetrazole (119 mg, 1.7 mmol, 4.5 eq) was added to the mixture. The mixture was then stirred under an argon atmosphere for 4 hours, and then a tert-butyl hydroperoxide solution (5-6 M in decane, 0.12 mL, 0.6 mmol, 1.6 eq) was added to oxidize the resulting compound. The mixture was thereafter stirred for an additional 60 minutes, and 5 mL of dichloromethane was added to dilute the reaction mixture. After filtering MS 3 Å through filter paper, the mixture was washed with 15 mL of dichloromethane and then washed with 40 mL of water. The organic layer was collected, and trichloroacetic acid (372 mg, 3.2 mmol, 8.8 eq) was added under an ice bath. The mixture was thereafter stirred for 30 minutes. After complete conversion, dichloroethane was added to the mixture, and the mixture was washed with NaHCO₃. The organic layer was dried (Na₂SO₄) and concentrated. Column chromatography was used for purification (silica gel, dichloromethane:methanol = 19:1 to 9:1). 280 mg of a white solid was obtained (yield: 58%). ¹H-NMR (600 MHz, DMSO-D6) δ 12.11-12.09 (m, 2H), 11.58 (s, 2H), 11.42 (d, J = 6.0 Hz, 1H), 8.30-8.24 (m, 2H), 7.89-7.85 (m, 1H), 6.12 (t, J = 6.6 Hz, 1H), 5.95-5.91 (m, 2H), 5.80 (q, J = 6.6 Hz, 1H), 5.71 (dd, J=11.4, 8.4, 1H), 5.52-5.50 (m, 1H), 5.35 (s, 1H), 5.21-5.19 (m, 1H), 4.99-4.96 (m, 1H), 4.67-4.61 (m, 1H), 4.50-4.19 (m, 10H), 4.18-4.16 (m, 1H), 4.09 (td, J = 16.82, 5.4 Hz, 1H), 3.57 (s, 2H), 3.37-3.30 (m, 6-H), 2.97-2.91 (m,4H), 2.79-2.72 (m, 2H), 2.13 (d, J = 10.8 Hz, 3H), 2.02 (d, J = 1.2 Hz, 3H), 1.13-1.11 (m, 12H) ppm. ¹³C-NMR (100MHz, ACETONITRILE-D3) δ 179.1, 168.9, 168.8, 168.5, 163.1, 154.7, 149.6, 147.6, 147.3, 139.5, 139.2, 137.8, 137.4, 122.9, 119.5, 119.3, 116.1, 116.1, 116.0, 100.6, 100.5, 85.1, 84.9, 80.4, 74.2, 71.5, 71.3, 69.2, 65.5, 62.1, 62.0, 58.8, 56.8, 56.7, 56.5, 52.1, 47.2, 47.0, 46.7, 46.5, 46.3, 46.1, 45.9, 45.7, 34.3, 17.9, 17.6, 16.8, 16.7, 16.6 ppm. ³¹P-NMR (240 MHz, DMSO-D6) δ -1.6, -1.9 ppm. HRMS (ESI) Calcd. For C₄₉H₆₂N₁₄NaO₂₄P⁺( [M+Na] ⁺): 1315.3429. Obsd.1315.3428

### Synthesis of Trinucleotide Form pUGG (38)

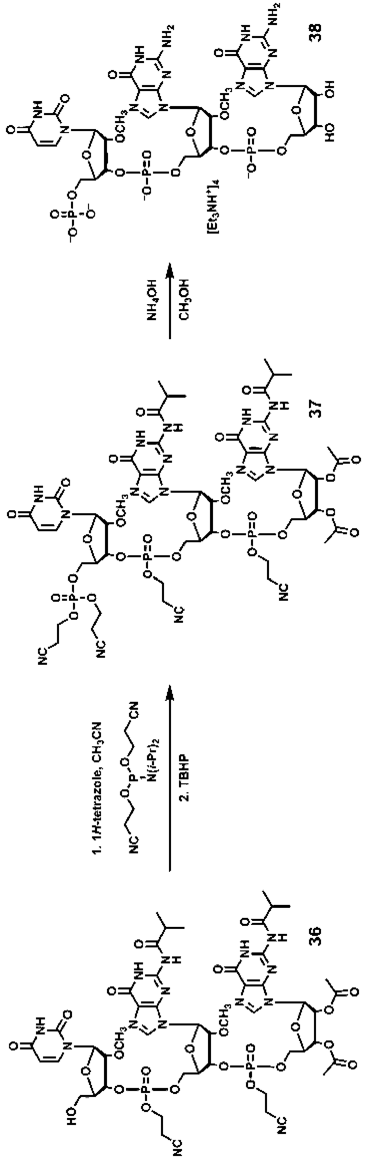

Compound 18-6-1 (170 mg, 0.13 mmol, 1 eq) was co-evaporated once with toluene to remove water, and bis(2-cyanoethyl) diisopropylphosphoramidite (144 mg, 0.52 mmol, 4 eq), 135 mg of molecular sieve 3 Å, and 5 mL of acetonitrile were added thereto. After stirring for 9 minutes under an argon atmosphere, 1H-tetrazole (45 mg, 0.64 mmol, 5 eq) was added. The mixture was stirred at ambient temperature for 4 hours, and TBHP (5-6 M in decane, 52 µL, 0.26 mmol, 2 eq) was added dropwise. The reaction mixture was diluted with dichloromethane after 1 hour and washed with water. The organic layer was dried (Na₂SO₄) and concentrated. The reaction crude was used as is for deprotection. The resulting solid was dissolved in 1.8 mL of methanol, and 1.7 mL of a NH₄OH solution (28%) was added thereto. After stirring for days, the reaction mixture was transferred to 55°C to achieve complete conversion of the starting compound. The solution was thereafter removed by rotary evaporation. The resulting solid was dissolved in 5 mL of water, loaded onto a DEAE-Sephadex TM A-25 column (size: 70 cm³), and purified by ion-exchange chromatography (0-1.5 M TEAB buffer containing 10% CH₃CN, 15-270 minute linear gradient, flow rate 6 mL/min). Fractions with a retention time of 110 to 150 minutes were collected, and then the solvent was removed. 50 mg of a white solid was obtained (yield: 25%, over two steps). ¹H-NMR (600 MHz, D₂O) δ 7.99 (d, J = 3.0 Hz, 2H), 7.95 (d, J = 8.4 Hz, 1H), 6.06 (d, J = 7.2 Hz, 1H), 5.91-5.87 (m, 3H), 4.98-4.94 (m, 1H), 4.70-4.67 (m, 1H), 4.81-4.73 (m, 1H), 4.48-4.44 (m, 2H), 4.33-4.31 (m, 2H), 4.16-4.13 (m, 4H), 4.08 (td, J =11.4, 3.6 Hz, 1H), 3.87 (dd, J = 5.4, 3.0 Hz, 1H), 3.39 (s, 3H), 3.32 (s, 3H), 3.21 (q, J = 7.2 Hz, 2H), 3.07 (q, J = 7.2 Hz, 39H, TEA), 1.21-1.18 (t, J = 7.8Hz, 62H, TEA) ppm. ¹³C-NMR (150 MHz, D₂O) δ 165.9, 158.7, 153.8, 153.6, 151.7, 151.5, 151.4, 141.1, 138.1, 137.7, 116.7, 116.4, 102.6, 87.3, 86.3, 83.7, 83.6, 82.5, 81.5, 80.5, 73.2, 72.7, 72.1, 70.3, 65.2, 63.6, 58.9, 57.9, 57.6, 46.7, 42.3, 10.6, 8.3, 7.4 ppm. ³¹P-NMR (240 MHz, D₂O) δ 4.2, 0.0, -0.2 ppm. HRMS (ESI) Calcd. For C₃₁H₄₀N₁₂O₂₃P₃⁻ ([M-H]⁻) : 1041.1548. Obsd.1041.1543.

### Synthesis Scheme of Trinucleotide pCGG(X)

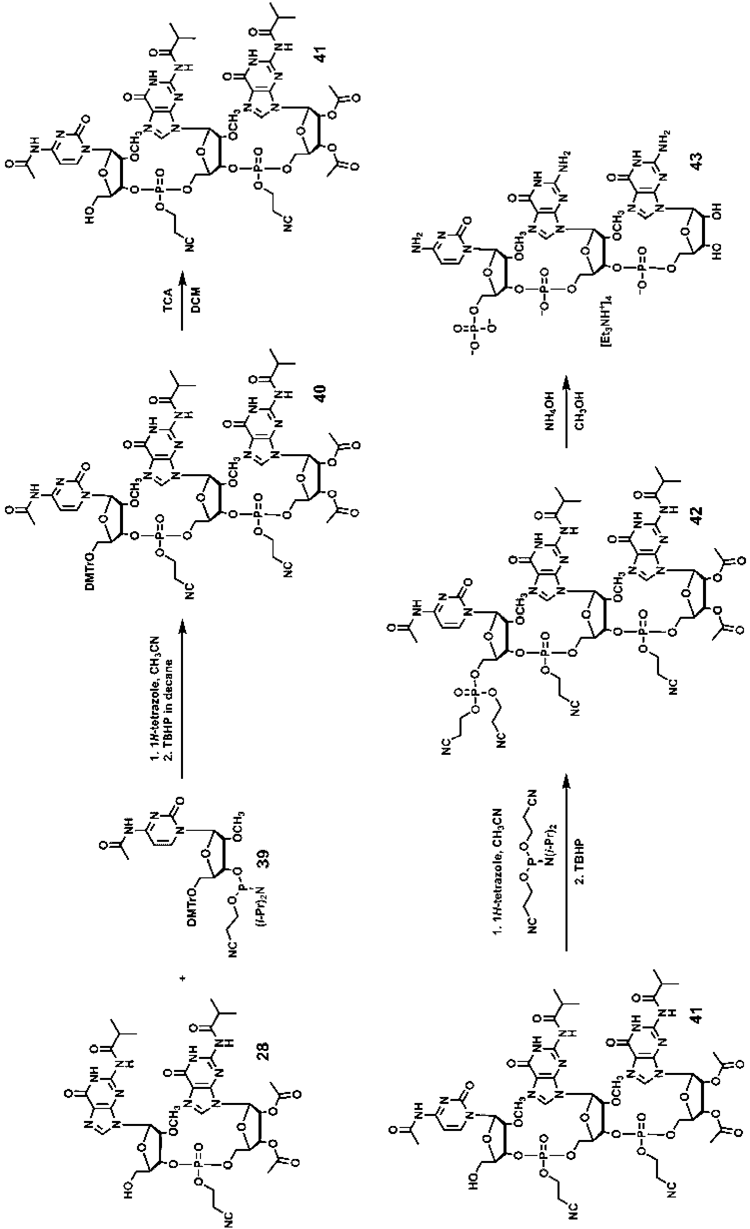

### Synthesis of Trinucleotide Form CGG (41)

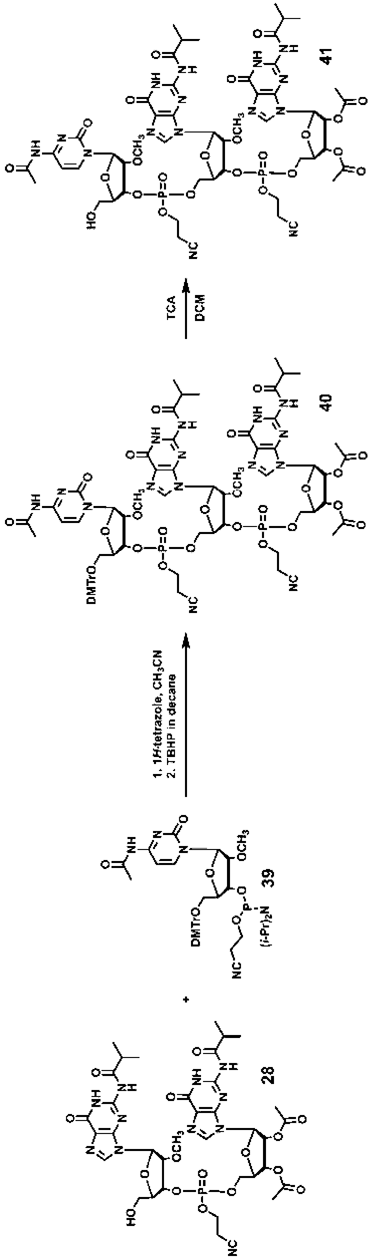

Compound 18-4 (95 mg, 0.1 mmol, 1 eq) was co-evaporated with toluene to remove water, and then 2'-OMe-CE-phosphoramidite (101 mg, 0.13 mmol, 1.3 eq) was added to the flask. 2 mL of acetonitrile was added to dissolve the compound, and then 126 mg of molecular sieve 3 Å was added. After stirring for 15 minutes, 1H-tetrazole (32 mg, 0.45 mmol, 4.5 eq) was added to the mixture. The mixture was thereafter stirred under an argon atmosphere for 3 hours, and a tert-butyl hydroperoxide solution (5-6 M in decane, 0.02 mL, 0.12 mmol, 1.3 eq) was added to oxidize the resulting compound. The mixture was thereafter stirred for an additional 45 minutes, and about 5 mL of dichloromethane was added to dilute the reaction mixture. After filtering MS 3 Å through filter paper, the mixture was washed with 5 mL of dichloromethane and then washed with 40 mL of water. The organic layer was collected, and trichloroacetic acid (220 mg, 1.3 mmol, 13 eq.) was added under an ice bath. The mixture was thereafter stirred for 30 minutes. After complete conversion, dichloroethane was added to the mixture, and the mixture was washed with NaHCO₃. The organic layer was dried (Na₂SO₄) and concentrated. Column chromatography was used for purification (silica gel, dichloromethane:methanol = 9:1, 1% TEA). 40 mg of a white solid was obtained (yield: 30%). ¹H-NMR (400 MHz, METHANOL-D4) δ 8.47-8.39 (m, 1H), 8.14-8.12 (m, 1H), 8.08-8.03 (m, 1H), 6.20-6.16 (m, 1H), 6.03-5.87 (m, 2H), 5.67-5.62 (m, 1H), 5.28-5.11 (m, 1H), 4.98 (dt, J = 17.5, 6.1 Hz, 1H), 4.75-4.65 (m, 1H), 4.56-4.41 (m, 5H), 4.38-4.23 (m, 6H), 4.16 (t, J = 4.9 Hz, 1H), 4.06-4.02 (m, 0H), 3.74-3.70 (m, 1H), 3.55 (d, J = 3.3 Hz, 1H), 3.50-3.47 (m, 1H), 3.41 (d, J = 3.8 Hz, 1H), 3.35 (q, J = 1.5 Hz, 2H), 3.29-3.28 (m, 2H), 2.95-2.85 (m, 4H), 2.80-2.60 (m, 2H), 2.13-2.10 (m, 3H), 2.05-2.01 (m, 3H), 1.21-1.15 (m, 12H) ppm. ¹³C-NMR (101 MHz, METHANOL-D4) δ 180.4, 180.3, 171.8, 171.7, 170.2, 170.2, 169.9, 169.9, 163.1, 163.0, 156.4, 156.2, 156.1, 156.0, 149.1, 149.0, 148.6, 148.5, 148.4, 145.0, 144.4, 139.6, 139.1, 138.8, 138.2, 121.2, 120.9, 120.6, 117.5, 117.3, 96.9, 96.5, 88.6, 88.5, 88.4, 86.7, 86.5, 86.3, 86.1, 85.5, 82.6, 82.1, 81.2, 81.1, 73.0, 72.9, 72.7, 72.6, 72.5, 70.6, 70.3, 67.5, 67.3, 63.5, 63.4, 63.4, 60.6, 59.4, 58.2, 58.1, 57.9, 57.5, 48.5, 48.3, 48.1, 48.0, 47.9, 47.7, 47.5, 47.3, 47.0, 35.7, 23.4, 23.3, 19.2, 19.2, 19.0, 19.0, 18.9, 18.8, 18.7, 18.4, 18.1, 18.0, 17.8 ppm. ³¹P-NMR (162 MHz, METHANOL-D4) δ -2.2, -2.3, -2.5 ppm. HRMS (ESI) Calcd. For C₅₁H₆₆N₁₅O₂₄P2⁺ ([M+H] ⁺) : 1334.3875. Obsd. 1334.3874.

### Synthesis of Trinucleotide Form pCGG (43)

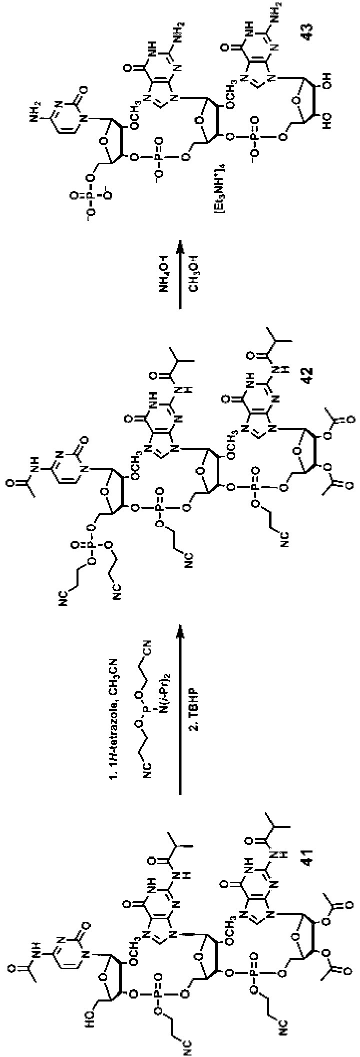

Compound 18-6-2 (110 mg, 0.13 mmol, 1 eq.) was co-evaporated once with toluene to remove water, and bis(2-cyanoethyl) diisopropylphosphoramidite (90 mg, 0.52 mmol, 4 eq.), 80 mg of molecular sieve 3 Å, and 5 mL of acetonitrile were added thereto. After stirring for 20 minutes under an argon atmosphere, 1H-tetrazole (45 mg, 0.64 mmol, 5 eq) was added. The mixture was stirred at ambient temperature for 2 hours and 15 minutes, and TBHP (5-6 M in decane, 0.12 mL, 0.26 mmol, 2 eq.) was added dropwise. The reaction mixture was diluted with dichloromethane after 2 hours and washed with water. The organic layer was dried (Na₂SO₄) and concentrated. The reaction crude was used as is for deprotection. The resulting solid was dissolved in 1.8 mL of methanol, and 1.7 mL of a NH₄OH solution (28%) was added thereto. After stirring for days, the reaction mixture was transferred to 55°C to achieve complete conversion of the starting compound. The solution was thereafter removed by rotary evaporation. The resulting solid was dissolved in 5 mL of water, loaded onto a DEAE-Sephadex TM A-25 column (size: 70 cm³), and purified by ion-exchange chromatography (0-1.5 M TEAB buffer containing 10% CH₃CN, 15-270 minute linear gradient, flow rate 6 mL/min). Fractions with a retention time of 110 to 150 minutes were collected, and then the solvent was removed. 35 mg of a white solid was obtained (yield: 25%, over two steps). ¹H-NMR (400 MHz, D₂O) δ 7.90 (d, J = 7.6 Hz, 1H), 7.85 (s, 1H), 7.79 (s, 1H), 5.90 (d, J = 8.0 Hz, 1H), 5.85 (d, J = 4.8 Hz, 1H), 5.74 (d, J = 6.4 Hz, 2H), 4.82-4.77 (m, 1H), 4.58-4.49 (m, 2H), 4.34-4.32 (m, 1H), 4.29 (s, 1H), 4.18-4.16 (m, 2H), 4.00-4.05 (m, 3H), 3.92-3.91 (m, 2H), 3.82-3.73 (m, 2H), 3.25 (d, J = 6.8 Hz, 6H) ppm. ¹³C-NMR (100 MHz, D₂O) δ 166.0, 160.1, 159.6, 157.3, 154.9, 154.2, 151.5, 151.5, 141.4, 137.6, 137.5, 137.1, 116.6, 116.5, 96.7, 87.3, 87.0, 86.1, 83.5, 83.4, 82.8, 81.8, 80.5, 73.3, 72.6, 72.2, 70.2, 65.0, 62.8, 59.0, 57.9, 57.5, 46.6, 42.2, 25.6, 23.3, 18.2, 14.8, 10.5, 8.7, 8.2, 7.5 ppm. ³¹P-NMR (160 MHz, D₂O) δ 4.1, -0.2, -0.3 ppm. HRMS (ESI) Calcd. For C₃₁H₄₁N₁₃O₂₂P₃⁻ ([M-H] ⁻): 1040.1707. Obsd.1040.1710.

### Solid-Phase Synthesis of Trinucleotides Using Automated Nucleic Acid Synthesizer

### Synthesis Scheme

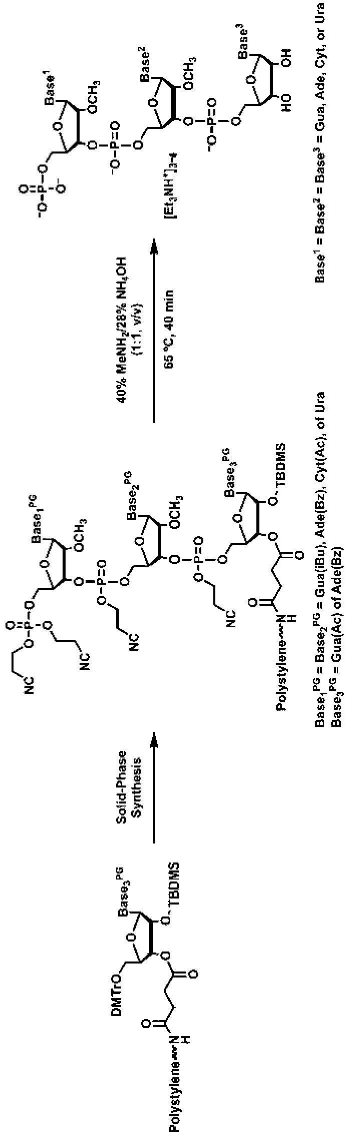

### Reaction Conditions and Experimental Procedures

Synthesis of dinucleotides and trinucleotides was carried out with the Primer Support 5G riboG 300 (Cytiva, 15.0 µmol-scale synthesis), using a DNA/RNA synthesizer NR-2A_7MX (Nihon Techno Service Co., Ltd., Ushiku City, Ibaraki Prefecture). In the coupling step,
100 mM 2'-O-methyl-adenosine (n-benzoyl)-CE-phosphoramidite (ChemGenes) in CH₃CN, 100 mM 2'-O-methyl-adenosine (n-benzoyl)-CE-phosphoramidite (synthesized according to literature) in CH₃CN, 100 mM 2'-O-methyl-guanosine (n-i-Bu)-CE-phosphoramidite (ChemGenes) in CH₃CN, 50-70 mM 5'-DMTr-2'-TOM-riboadenosine (n-acetyl) OP (ChemGenes) in CH₃CN, 150 mM bis(2-cyanoethyl)-N,N-diisopropylphosphoramidite in CH₃CN, and 0.3 M 5-(benzylthio)-1H-tetrazole in CH₃CN (activator) were circulated through the column. A solution of 184 mM trichloracetic acid/dichloromethane was used as a detritylation reagent, 0.05 M I₂ in pyridine/H₂O (9:1, v/v) was used for oxidation, 10% Ac₂O in THF/pyridine (8:1, v/v) was used as CapA, and 10% 1-methylimidazole in THF was used as CapB. Synthesis was carried out by applying standard RNA synthesis protocols on a 10 µmol scale. After the final cycle of synthesis, the solid support was treated with a 1:1 mixture of 28% ammonium hydroxide/40% methylamine (1.00 mL/15.0 µmol of the solid support) at 65°C for 1 to 2 hours to cleave the nucleotide from the solid support and remove the protecting group. The resulting nucleotide was concentrated to dryness and re-dissolved in DMSO (1.00 mL/60.0 µmol). TEA-3HF (1.00 mL/60.0 µmol) was added to this solution, and the mixture was incubated at 65°C for 3 to 5 hours to remove the 2'-O-silyl protecting group. The reaction mixture was neutralized by adding 250 mM Na₂CO₃ aq. (10.0 mL/60.0 µmol). The crude was purified by ion-exchange chromatography on DEAE-Sephadex (diameter = 4.5 cm, h = 8.4 cm, 140 cm³, 12.0 mL/min) with a linear 0-1.2 M gradient (270 min) of TEAB buffer (pH of 7.9) containing 0-8% CH₃CN. Fractions containing the product were collected and concentrated to give a di/trinucleotide. The yield was calculated using the absorbance of the product at 260 nm measured on the NanoDrop. In the calculation, extinction coefficient (ε₂₆₀) = 25,000 M⁻¹-cm⁻¹ was used for a dinucleotide, and 35,100 M⁻¹-cm⁻¹ was used for a trinucleotide.

pUUG (triethylammonium salt, ε₂₆₀ = 29,700 M⁻¹•cm⁻¹, 37.7% yield) (44): ¹H NMR (600 MHz, D₂O) δ 7.92 - 7.83 (m, 2H), 7.70 (dd, J= 8.2, 3.2 Hz, 1H), 5.84 - 5.73 (m, 3H), 5.70 (td, J = 6.9, 3.1 Hz, 2H), 4.62 (dt, J = 5.5, 2.7 Hz, 2H), 4.56 (dq, J = 5.5, 2.8 Hz, 1H), 4.34 - 4.29 (m, 1H), 4.26 (p, J = 2.8 Hz, 1H), 4.20 - 4.14 (m, 2H), 4.01 (dq, J = 11.4, 4.5 Hz, 3H), 3.98 - 3.94 (m, 1H), 3.92 (dq, J = 7.7, 4.6 Hz, 3H), 3.89 - 3.85 (m, 1H), 3.32 (dd, J = 6.2, 3.3 Hz, 6H), 3.14 - 2.78 (m, 28H), 1.10 (td, J = 7.1, 3.2 Hz, 36H) ppm. ¹³C NMR (151 MHz, D₂O) δ 181.28, 165.96, 165.60, 158.69, 153.80, 151.66, 151.47, 151.30, 141.57, 140.76, 137.73, 116.43, 102.61, 102.55, 87.43, 86.66, 86.35, 83.74, 83.69, 83.07, 81.97, 81.48, 81.38, 73.40, 72.29, 71.77, 70.45, 65.31, 64.27, 63.39, 58.94, 57.93, 57.73, 55.08, 50.02, 48.63, 46.64, 42.70, 42.23, 39.88, 39.23, 34.92, 34.50, 28.78, 25.62, 23.29, 21.87, 19.20, 19.01, 18.27, 14.84, 13.42, 12.76, 12.48, 12.37, 11.88, 10.75, 10.73, 10.58, 10.54, 10.22, 8.72, 8.25, 7.48 ppm. 31P NMR (243 MHz, D₂O) δ 1.90 (1P), -0.46(1P), -0.67 (1P) ppm. HR-ESI-MS calcd. for C₃₀H₃₉N₉O₂₄P₃, 1002.13263 [M - H]⁻; found 1002.13203.

pUCG (triethylammonium salt, ε₂₆₀ = 27,800 M⁻¹•cm⁻¹, 20.7% yield) (45): ¹H NMR (600 MHz, D₂O) δ 8.04 - 7.96 (m, 3H), 6.04 (d, J = 7.7 Hz, 1H), 5.96 (d, J = 2.5 Hz, 1H), 5.90 (d, J = 3.4 Hz, 1H), 5.87 - 5.83 (m, 2H), 4.73 (t, J = 5.6 Hz, 1H), 4.70 - 4.63 (m, 2H), 4.44 (dd, J = 5.2, 3.4 Hz, 1H), 4.39 (dq, J = 4.8, 2.3 Hz, 1H), 4.33 (ddt, J= 8.5, 5.9, 2.8 Hz, 2H), 4.25 - 4.10 (m, 7H), 4.02 (dd, J = 4.9, 2.5 Hz, 1H), 3.53 (dd, J = 6.6, 4.4 Hz, 6H), 3.18 (q, J = 7.3 Hz, 13H), 1.34 - 1.23 (m, 27H) ppm. ¹³C NMR (151 MHz, D₂O) δ 165.93, 163.21, 158.73, 153.75, 151.68, 151.22, 141.26, 140.86, 137.92, 116.40, 102.12, 95.72, 87.63, 86.89, 83.77, 83.71, 82.12, 81.97, 81.26, 80.93, 73.37, 71.02, 70.65, 70.33, 64.97, 63.14, 62.97, 58.62, 57.89, 57.63, 46.70, 8.27 ppm. ³¹P NMR (243 MHz, D₂O) δ 0.49 (1P), -0.45 (1P), -0.85 (1P) ppm. HR-ESI-MS calcd. for C₃₀H₄₀N₁₀O₂₃P₃, 1001.14861 [M - H]⁻; found 1001.14034.

pUAG (triethylammonium salt, ε₂₆₀ = 34,200 M⁻¹•cm⁻¹, 28.0% yield) (46): ¹H NMR (600 MHz, D₂O) δ 8.28 (s, 1H), 8.04 (s, 1H), 7.82 (s, 1H), 7.73 (d, J = 8.2 Hz, 1H), 7.24 (s, 4H), 5.98 (d, J = 4.8 Hz, 1H), 5.73 - 5.66 (m, 3H), 4.82 (dt, J = 8.0, 4.8 Hz, 1H), 4.61 - 4.55 (m, 2H), 4.36 - 4.31 (m, 3H), 4.19 (ddp, J = 6.9, 4.8, 2.3 Hz, 2H), 4.07 (q, J = 4.3 Hz, 2H), 3.98 (q, J = 2.9 Hz, 2H), 3.91 (dd, J = 4.4, 2.4 Hz, 2H), 3.86 (t, J = 5.1 Hz, 1H), 3.38 (s, 3H), 3.23 (s, 3H), 3.04 (q, J = 7.3 Hz, 18H), 1.12 (t, J = 7.4 Hz, 27H) ppm. ¹³C NMR (151 MHz, D₂O) δ 165.86, 158.44, 154.45, 153.64, 151.58, 151.42, 151.25, 148.56, 141.11, 139.61, 137.59, 128.47, 118.50, 116.20, 102.46, 87.54, 86.31, 85.62, 83.49, 82.49, 81.80, 81.40, 73.31, 72.30, 72.03, 70.17, 65.12, 64.62, 63.66, 58.60, 58.03, 57.58, 46.67, 8.24 ppm. ³¹P NMR (243 MHz, D₂O) δ 0.62 (s, 1P), -0.30 (s, 1P), -0.63 (s, 1P) ppm. ESI-TOF-MS calcd. for C₃₁H₃₉N₁₂O₂₂P₃, 512.0763 [M - 2H]²⁻; found 512.0774.

pCUG (triethylammonium salt, ε₂₆₀ = 26,300 M⁻¹•cm⁻¹, 32.4% yield) (47): ¹H NMR (600 MHz, D₂O) δ 8.06 - 8.00 (m, 2H), 7.86 (d, J = 8.2 Hz, 1H), 5.99 (d, J = 7.6 Hz, 1H), 5.94 (d, J = 2.9 Hz, 1H), 5.91 (d, J = 2.5 Hz, 1H), 5.85 (d, J = 6.1 Hz, 1H), 5.73 (d, J = 8.1 Hz, 1H), 4.72 (t, J = 5.6 Hz, 1H), 4.67 - 4.56 (m, 2H), 4.45 (dd, J = 5.3, 3.3 Hz, 1H), 4.38 - 4.30 (m, 3H), 4.24 (dt, J= 12.2, 3.3 Hz, 1H), 4.18 - 4.06 (m, 6H), 3.97 (dd, J = 4.9, 2.9 Hz, 1H), 3.58 (s, 3H), 3.50 (s, 3H), 3.17 (q, J = 7.4 Hz, 18H), 1.25 (t, J = 7.4 Hz, 27H) ppm. ¹³C NMR (151 MHz, D₂O) δ 165.90, 165.34, 158.87, 156.85, 153.88, 151.75, 150.90, 141.18, 139.80, 137.73, 116.44, 102.23, 96.01, 87.55, 87.37, 86.97, 83.87, 83.81, 81.76-81.03, 73.56, 70.80, 70.48, 65.09, 63.19, 62.65, 58.95, 57.85, 57.60, 46.69, 8.27 ppm. ³¹P NMR (243 MHz, D₂O) δ 1.12 (1P), -0.52 (1P), -0.92 (1P) ppm. HR-ESI-MS calcd. for C₃₀H₄₀N₁₀O₂₃P₃, 1001.14861 [M - H]-; found 1001.14034.

pCCG (triethylammonium salt, ε₂₆₀ = 24,800 M⁻¹•cm⁻¹, 37.7% yield) (48): ¹H NMR (600 MHz, D₂O) δ 7.90 (d, J = 7.6 Hz, 1H), 7.81 - 7.73 (m, 2H), 5.83 (d, J = 7.6 Hz, 1H), 5.81 - 5.75 (m, 2H), 5.70 (dd, J= 9.9, 3.5 Hz, 2H), 4.45 (qd, J= 11.5, 6.8 Hz, 3H), 4.27 (t, J= 4.5 Hz, 1H), 4.21 - 4.13 (m, 4H), 4.10 - 4.01 (m, 3H), 3.99 - 3.93 (m, 3H), 3.81 (d, J = 5.9 Hz, 1H), 3.49 - 3.42 (m, 6H), 3.20 - 2.84 (m, 24H), 1.10 (td, J = 7.3, 3.8 Hz, 36H) ppm. ¹³C NMR (151 MHz, D₂O) δ 181.18, 165.59, 165.11, 158.89, 156.42, 155.99, 153.78, 151.54, 141.07, 139.28, 137.28, 116.27, 96.04, 95.72, 87.72, 87.67, 83.35, 81.96, 81.57, 81.20, 80.25, 73.72, 70.21, 70.04, 64.62, 62.58, 62.20, 58.90, 57.68, 57.52, 48.64, 46.65, 42.71, 42.24, 39.88, 34.93, 34.51, 28.78, 25.62, 24.81, 23.22, 21.87, 19.21, 19.01, 18.26, 14.84, 13.42, 12.76, 12.37, 11.88, 10.73, 10.58, 10.22, 8.71, 8.26, 7.44 ppm. 31P NMR (243 MHz, D20) δ 1.08 (1P), -0.55 (1P), -1.05 (1P) ppm. HR-ESI-MS calcd. for C₃₀H₄₁N₁₁O₂₂P₃, 1000.16460 [M - H]⁻; found 1000.11641 pCAG (triethylammonium salt, ε₂₆₀ = 30,600 M⁻¹•cm⁻¹, 33.7% yield) (49): ¹H NMR (600 MHz, D₂O) δ 8.32 (s, 1H), 8.04 (d, J = 1.2 Hz, 1H), 7.87 (d, J = 7.6 Hz, 2H), 6.12 (d, J = 3.7 Hz, 1H), 5.89 (d, J = 7.6 Hz, 1H), 5.82 (d, J = 3.1 Hz, 1H), 5.76 (d, J = 5.4 Hz, 1H), 4.87 (dt, J = 7.9, 5.2 Hz, 1H), 4.67 (t, J = 5.4 Hz, 1H), 4.60 (q, J = 7.6 Hz, 1H), 4.42 (dt, J = 6.0, 3.7 Hz, 2H), 4.30 (dt, J = 5.9, 3.4 Hz, 3H), 4.21 - 4.10 (m, 5H), 4.09 - 4.02 (m, 2H), 3.53 (d, J= 0.8 Hz, 3H), 3.45 (s, 3H), 3.31 (q, J= 7.2 Hz, 2.5H), 3.15 (qd, J = 7.3, 0.8 Hz, 21.5H), 1.23 (td, J= 7.3, 0.9 Hz, 36H) ppm. ¹³C NMR (151 MHz, D₂O) δ 165.36, 158.50, 156.41, 155.14, 153.60, 152.67, 151.33, 148.30, 140.90, 138.60, 137.62, 118.45, 116.29, 95.96, 87.81, 87.40, 85.69, 83.50, 83.44, 82.15, 81.62, 73.29, 71.78, 71.22, 70.06, 64.87, 63.94, 63.02, 58.89, 58.00, 57.58, 46.68, 8.26 ppm. ³¹P NMR (243 MHz, D₂O) δ 0.94 (1P), -0.44 (1P), -0.71 (1P) ppm. HR-ESI-MS calcd. for C₃₁H₄₁N₁₃O₂₁P₃, 1024.16583 [M - H]⁻; found 1024.17943.

pAUG (triethylammonium salt, ε₂₆₀ = 34,100 M⁻¹•cm⁻¹, 35.0% yield) (50): ¹H NMR (600 MHz, D₂O) δ 8.48 (s, 1H), 8.15 (s, 1H), 7.98 (s, 1H), 7.74 (d, J= 8.2 Hz, 1H), 7.37 (s, 3H), 6.15 (d, J= 4.9 Hz, 1H), 5.82 (dd, J = 9.9, 4.9 Hz, 2H), 5.66 (d, J = 8.2 Hz, 1H), 4.92 - 4.87 (m, 1H), 4.72 - 4.63 (m, 2H), 4.54 (dq, J = 5.2, 2.6 Hz, 1H), 4.49 (t, J = 4.8 Hz, 1H), 4.44 (dd, J = 5.2, 3.6 Hz, 1H), 4.35 (dq, J = 5.4, 2.6 Hz, 1H), 4.33 - 4.29 (m, 1H), 4.22 (ddd, J = 11.6, 4.6, 2.5 Hz, 1H), 4.17 - 4.09 (m, 5H), 4.02 (t, J = 4.5 Hz, 1H), 3.52 (s, 3H), 3.48 (s, 3H), 3.17 (q, J = 7.3 Hz, 17H), 1.33 - 1.23 (m, 27H) ppm. ¹³C NMR (151 MHz, D₂O) δ 165.42, 158.56, 154.03, 153.68, 151.49, 151.01, 150.79, 148.50, 140.27, 140.19, 137.66, 128.49, 118.51, 116.20, 102.16, 87.40, 86.91, 85.61, 83.66, 83.08, 82.15, 81.64, 73.52, 72.35, 71.39, 70.41, 65.18, 63.85, 58.58, 58.03, 57.93, 46.69, 8.26 ppm. ³¹P NMR (243 MHz, D₂O) δ 0.71 (1P), -0.42 (1P), -0.64 (1P) ppm. HR-ESI-MS calcd. for C₃₁H₄₀N₁₂O₂₂P₃, 1025.15985 [M - H]⁻; found 1025.15478.

pACG (triethylammonium salt, ε₂₆₀ = 31,600 M⁻¹•cm⁻¹, 29.7% yield) (51): ¹H NMR (400 MHz, D₂O) δ 8.40 (s, 1H), 8.03 (s, 1H), 7.86 (s, 1H), 7.65 (d, J = 7.6 Hz, 1H), 7.34 (s, 0.5H), 6.03 (s, 1H), 5.75 (d, J = 5.3 Hz, 1H), 5.68 - 5.58 (m, 2H), 4.70 (d, J= 7.6 Hz, 1H), 4.58 - 4.43 (m, 5H), 4.39 - 4.26 (m, 5H), 4.22 (d, J = 2.7 Hz, 0.5H), 4.17 - 4.06 (m, 5.5H), 3.93 (d, J = 5.3 Hz, 1H), 3.64 (s, 3H), 3.56 (s, 3H), 3.49 (q, J = 7.3 Hz, 3H), 3.15 (q, J= 7.3 Hz, 15H), 1.31 - 1.20 (m, 27H) ppm. ¹³C NMR (101 MHz, D₂O) δ 162.82, 158.27, 154.47, 153.50, 153.10, 151.20, 147.69, 139.60, 137.09, 128.42, 118.57, 115.98, 94.89, 87.67, 86.17, 83.18, 81.72, 81.50, 80.40, 73.57, 70.83, 69.79, 64.51, 62.67, 58.57, 57.79, 57.72, 46.63, 42.25, 24.69, 10.55, 8.23, 7.18 ppm. ³¹P NMR (162 MHz, D₂O) δ 0.59 (1P), -0.53 (1P), -1.00 (1P) ppm. HR-ESI-MS calcd. for C₃₁H₄₁N₁₃O₂₁P₃, 1024.17583 [M - H]⁻; found 1024.17943.

pAAG (triethylammonium salt, ε₂₆₀ = 37,000 M⁻¹•cm⁻¹, 28.3% yield) (52): ¹H NMR (600 MHz, D₂O) δ 8.52 (s, 1H), 8.31 (s, 1H), 8.10 (s, 1H), 7.93 (s, 1H), 7.89 (s, 1H), 6.09 (d, J = 5.0 Hz, 1H, H1'), 5.97 (d, J = 5.8 Hz, 1H, H1'), 5.81 (d, J = 5.9 Hz, 1H, H1'), 4.91 (ddd, J = 10.1, 7.6, 5.1 Hz, 2H), 4.60 (t, J = 5.6 Hz, 1H), 4.53 - 4.49 (m, 2H), 4.45 - 4.40 (m, 3H), 4.29 (dt, J= 5.5, 2.7 Hz, 1H), 4.21 (h, J= 3.4 Hz, 2H), 4.17 - 4.10 (m, 2H), 3.94 (q, J = 3.2 Hz, 2H), 3.45 (d, J= 4.8 Hz, 6H, 2'-OCH₃), 2.99 (q, J = 7.3 Hz, 46H, triethylammonium), 1.16 (t, J = 7.4 Hz, 70H, triethylammonium) ppm. ¹³C NMR (151 MHz, D₂O) δ 165.69, 159.41, 155.19, 152.77, 152.41, 151.48, 148.70, 148.39, 140.07, 139.17, 135.56, 118.43, 118.40, 117.31, 86.56, 85.42, 84.80, 83.90, 83.30, 82.76, 82.05, 81.89, 73.69, 73.21, 72.44, 70.23, 65.11, 64.88, 63.33, 59.00, 58.17, 57.65, 46.37, 8.56 ppm. ³¹P NMR (243 MHz, D₂O) δ 4.31, -0.37, -0.48 ppm. ESI-TOF-MS calcd. for C₃₂H₄₀N₁₅O₂₀P₃, 523.5899 [M - 2H] ²⁻; found 523.5926.

pGUG (triethylammonium salt, ε₂₆₀ = 31,300 M⁻¹•cm⁻¹, 33.1% yield) (53): ¹H NMR (400 MHz, D₂O) δ 8.09 (s, 1H), 7.99 (s, 1H), 7.76 (d, J = 8.2 Hz, 1H), 5.90 (d, J = 4.8 Hz, 1H), 5.87 (d, J = 4.0 Hz, 1H), 5.82 (d, J = 5.8 Hz, 1H), 5.65 (d, J = 8.1 Hz, 1H), 4.87 - 4.83 (m, 1H), 4.72 (t, J = 5.6 Hz, 1H), 4.66 (dt, J = 8.1, 5.0 Hz, 1H), 4.47 - 4.42 (m, 3H), 4.33 - 4.28 (m, 2H), 4.17 - 4.06 (m, 6H), 4.02 (t, J = 4.5 Hz, 1H), 3.47 (d, J = 8.5 Hz, 6H), 3.15 (q, J = 7.3 Hz, 18H), 1.23 (t, J = 7.3 Hz, 27H) ppm. ¹³C NMR (101 MHz, D₂O) δ 165.35, 158.48, 158.40, 153.75, 153.68, 151.49, 151.29, 151.08, 140.29, 137.67, 136.92, 128.42, 116.09, 115.74, 102.07, 87.51, 86.82, 85.42, 83.73, 83.64, 82.87, 81.61, 73.41, 72.18, 71.42, 70.39, 65.15, 63.87, 58.49, 57.93, 57.79, 46.63, 8.22 ppm. ³¹P NMR (161 MHz, D₂O) δ 0.68 (1P), -0.44 (1P), -0.65 (1P) ppm. HR-ESI-MS calcd. for C₃₁H₄₀N₁₂O₂₃P₃, 1041.15476 [M - H]⁻; found 1041.14643.

pGCG (triethylammonium salt, ε₂₆₀ = 28,000 M⁻¹•cm⁻¹, 32.7% yield) (54) ¹H NMR (400 MHz, D₂O) δ 8.02 (s, 1H), 7.78 (s, 1H), 7.70 (d, J = 7.6 Hz, 1H), 5.80 (s, 1H), 5.74 (d, J = 4.2 Hz, 1H), 5.71 - 5.60 (m, 1H), 5.50 (d, J = 7.8 Hz, 1H), 4.62 (s, 1H), 4.48 (d, J= 4.4 Hz, 1.5H), 4.44 - 4.31 (m, 6H), 4.26 (dt, J = 5.4, 2.5 Hz, 1.5H), 4.17 - 4.05 (m, 5H), 3.69 (s, 3H), 3.61 (s, 3H), 3.47 (q, J = 7.3 Hz, 2H), 3.15 (q, J = 7.4 Hz, 14H), 3.07 - 2.98 (m, 2H), 1.29 - 1.20 (m, 27H) ppm. ¹³C NMR (100 MHz, D₂O) δ 158.83, 157.90, 153.33, 153.16, 150.79, 150.53, 139.08, 136.38, 128.43, 116.07, 115.78, 94.61, 88.17, 87.66, 86.45, 82.45, 81.41, 80.80, 79.77, 73.85, 69.97, 69.43, 69.00, 63.93, 62.22, 58.60, 57.46, 57.41, 46.62, 42.24, 10.55, 8.22, 7.21 ppm. ³¹P NMR (162 MHz, D₂O) δ 0.62 (1P), -0.51 (1P), -1.03 (1P) ppm. HR-ESI-MS calcd. for C₃₁H₄₁N₁₃O₂₂P₃, 1040.17074 [M - H]⁻; found 1040.16350.

pGAG (triethylammonium salt, ε₂₆₀ = 34,800 M⁻¹•cm⁻¹, 37.7% yield) (55): ¹H NMR (600 MHz, D₂O, 0.2:1 rotamer mixture) δ 9.31 (s, 0.2H), 8.42 (d, J = 4.1 Hz, 0.4H), 8.29 (s, 1H), 8.09 (s, 1H), 7.99 (s, 1H), 7.90 (s, 1H), 7.78 (s, 0.2H), 7.32 (s, 0.2H), 6.35 (s, 0.2H), 6.06 (d, J = 4.7 Hz, 1H), 5.88 (d, J = 1.7 Hz, 0.2H), 5.76 (dd, J = 11.7, 5.1 Hz, 2H), 5.31 (d, J = 5.5 Hz, 0.2H), 5.08 (d, J = 8.4 Hz, 0.2H), 4.90 (dq, J = 9.6, 5.0 Hz, 1.5H), 4.87 - 4.83 (m, 1.5H), 4.76 (d, J = 1.8 Hz, 0.5H), 4.73 (d, J = 4.6 Hz, 0.2H), 4.68 (t, J = 5.3 Hz, 1H), 4.58 (dd, J = 8.1, 3.4 Hz, 0.2H), 4.54 - 4.51 (m, 0.2H), 4.44 (t, J = 4.6 Hz, 2.5H), 4.39 (q, J = 4.6 Hz, 2H), 4.35 - 4.28 (m, 2.5H), 4.24 (d, J = 9.3 Hz, 0.4H), 4.18 (q, J = 4.4 Hz, 2H), 4.14 (t, J = 3.5 Hz, 2H), 4.11 - 4.05 (m, 2H), 3.99 - 3.93 (m, 0.2H), 3.90 (s, 1H), 3.84 - 3.76 (m, 0.4H), 3.49 (s, 3H), 3.45 - 3.41 (m, 3H), 3.40 (d, J = 2.3 Hz, 3H), 3.32 (s, 0.2H), 3.14 (q, J = 7.4 Hz, 20H), 3.03 (dtd, J = 10.3, 7.3, 2.5 Hz, 2H), 2.93 (d, J = 3.2 Hz, 1H), 1.28 - 1.19 (m, 37H) ppm. ¹³C NMR (151 MHz, D₂O, 0.2:1 rotamer mixture) δ 158.36, 158.28, 157.45, 157.09, 154.64, 153.58, 153.40, 152.60, 152.09, 151.57, 151.30, 151.14, 150.56, 148.27, 147.23, 139.13, 137.50, 136.97, 128.41, 118.33, 117.99, 116.15, 115.90, 114.60, 112.75, 87.61, 85.61, 85.33, 83.49, 83.43, 82.63, 82.41, 82.07, 81.39, 73.41, 73.28, 72.25, 70.14, 65.02, 64.46, 63.94, 58.67, 58.06, 57.63, 57.51, 46.67, 8.25 ppm. ³¹P NMR (243 MHz, D₂O, 0.2:1 rotamer mixture) δ 0.82 (1P), 0.66 (0.2P), 0.27 (0.2P), -0.33 (1P), -0.60 (1P), -2.26 (0.2P) ppm. HR-ESI-MS calcd. for C₃₂H₄₁N₁₅O₂₁P₃, 1064.18198 [M - H]⁻; found 1064.17879.

pGGG (triethylammonium salt, ε₂₆₀ = 31,700 M⁻¹•cm⁻¹, 40.0% yield) (56): ¹H NMR (400 MHz, D₂O) δ 7.97 (s, 1H), 7.86 (d, J = 10.8 Hz, 2H), 5.82 - 5.69 (m, 3H), 4.87 (t, J = 4.2 Hz, 1H), 4.73 (d, J = 5.4 Hz, 1H), 4.52 (t, J = 5.3 Hz, 1H), 4.46 (t, J = 4.7 Hz, 1H), 4.34 (s, 1H), 4.31 - 4.24 (m, 3H), 4.15 (d, J = 4.9 Hz, 2H), 4.06 (s, 1.5H), 3.95 (s, 1.5H), 3.85 - 3.63 (m, 2H), 3.50 (q, J = 7.2 Hz, 2H), 3.35 (d, J = 7.9 Hz, 6H), 3.14 (q, J = 7.3 Hz, 14H), 3.02 (q, J = 7.2 Hz, 2H), 1.22 (t, J = 7.3 Hz, 27H) ppm. ¹³C NMR (101 MHz, D₂O) δ 158.36, 158.24, 158.08, 153.59, 153.44, 151.38, 151.28, 151.09, 137.57, 136.70, 128.38, 116.15, 115.40, 87.65, 86.01, 85.59, 83.57, 83.48, 82.61, 81.52, 80.47, 73.26, 72.57, 72.12, 70.25, 65.17, 64.80, 63.75, 58.52, 57.82, 57.60, 46.62, 42.24, 8.22 ppm. ³¹P NMR (162 MHz, D₂O) δ 0.59 (1P), -0.28 (1P), -0.52 (1P) ppm. HR-ESI-MS calcd. for C₃₂H₄₁N₁₅O₂₂P₃, 1080.17689 [M - H]⁻; found 1080.17185.

pUUA (triethylammonium salt, ε₂₆₀ = 34,300 M⁻¹•cm⁻¹, 33.8% yield) (57): ¹H NMR (400 MHz, D₂O) δ 8.49 (s, 1H), 8.22 (s, 1H), 7.95 (d, J = 8.2 Hz, 1H), 7.84 (d, J = 8.2 Hz, 1H), 7.39 (s, 2H), 6.09 (d, J = 5.4 Hz, 1H), 5.92 (dd, J = 6.3, 4.5 Hz, 2H), 5.83 (dd, J = 8.1, 3.8 Hz, 2H), 4.77 - 4.68 (m, 3H), 4.51 (t, J = 4.5 Hz, 1H), 4.41 - 4.33 (m, 3H), 4.20 - 4.09 (m, 7H), 4.02 (t, J = 4.9 Hz, 1H), 3.50 (s, 3H), 3.39 (s, 3H), 3.18 (q, J = 7.3 Hz, 18H), 1.26 (t, J = 7.3 Hz, 27H) ppm. ¹³C NMR (101 MHz, D₂O) δ 165.85, 165.59, 154.80, 151.84, 151.32, 151.26, 148.96, 141.21, 140.72, 139.98, 128.50, 118.65, 102.55, 102.28, 87.31, 86.66, 86.62, 83.80, 83.70, 82.47, 81.95, 81.55-81.44, 74.26, 71.82-71.68, 70.39, 65.06, 64.21, 63.47, 58.62, 57.91, 57.81, 46.69, 8.27 ppm. ³¹P NMR (162 MHz, D₂O) δ 0.67 (1P), -0.47 (1P), -0.79 (1P) ppm. HR-ESI-MS calcd. for C₃₀H₃₉N₉O₂₃P₃, 986.13771 [M - H]⁻; found 986.13124.

pCUA (triethylammonium salt, ε₂₆₀ = 30,900 M⁻¹•cm⁻¹, 30.0% yield) (58): ¹H NMR (600 MHz, D₂O) δ 8.51 (s, 1H), 8.22 (s, 1H), 8.03 (d, J = 7.7 Hz, 1H), 7.83 (d, J = 8.1 Hz, 1H), 7.39 (s, 2H), 6.09 (d, J = 5.5 Hz, 1H), 5.98 - 5.91 (m, 2H), 5.87 (d, J = 2.5 Hz, 1H), 5.75 (d, J = 8.1 Hz, 1H), 4.73 (dd, J = 6.8, 3.8 Hz, 2H), 4.66 (dt, J = 8.0, 5.4 Hz, 1H), 4.50 (dd, J = 5.1, 3.8 Hz, 1H), 4.36 (ddt, J = 8.3, 5.6, 2.7 Hz, 3H), 4.27 (ddd, J = 12.0, 4.8, 2.3 Hz, 1H), 4.19 - 4.06 (m, 6H), 3.97 (t, J = 4.5 Hz, 1H), 3.57 (s, 3H), 3.51 (q, J = 7.3 Hz, 2H), 3.37 (s, 3H), 3.18 (q, J = 7.3 Hz, 16H), 1.26 (t, J = 7.4 Hz, 27H) ppm. ¹³C NMR (151 MHz, D₂O) δ 165.43, 163.84, 154.75, 154.32, 151.78, 151.06, 148.96, 141.86, 140.20, 139.91, 128.52, 118.67, 102.37, 95.44, 87.70, 87.26, 86.65, 83.92, 83.86, 81.71, 74.36, 71.34, 70.64, 70.48, 65.01, 63.67, 62.68, 58.63, 57.86, 57.78, 46.70, 8.27 ppm. ³¹P NMR (243 MHz, D₂O) δ 0.75 (1P), -0.46 (1P), -1.03 (1P) ppm. HR-ESI-MS calcd. for C₃₀H₄₀N₁₀O₂₂P₃, 985.15370 [M - H]⁻; found 985.14609.

pAUA (triethylammonium salt, ε₂₆₀ = 38,700 M⁻¹•cm⁻¹, 27.8% yield) (59): ¹H NMR (400 MHz, D₂O) δ 8.38 (s, 1H), 8.28 (s, 1H), 8.02 (s, 1H), 7.98 (s, 1H), 7.69 (d, J = 8.2 Hz, 1H), 7.31 (s, 1H), 6.04 (d, J = 4.4 Hz, 1H), 5.99 (d, J = 5.4 Hz, 1H), 5.79 (d, J = 4.4 Hz, 1H), 5.63 (d, J = 8.2 Hz, 1H), 4.95 (dt, J = 8.7, 4.7 Hz, 1H), 4.64 (t, J = 5.3 Hz, 1H), 4.57 (dd, J = 8.0, 5.2 Hz, 1H), 4.53 - 4.41 (m, 3H), 4.35 - 4.28 (m, 2H), 4.25 - 4.04 (m, 6H), 3.88 (t, J = 4.7 Hz, 1H), 3.52 (s, 3H), 3.43 (q, J = 7.2 Hz, 2H), 3.25 (s, 3H), 3.12 (q, J = 7.3 Hz, 16H), 1.20 (t, J = 7.4 Hz, 27H) ppm. ¹³C NMR (101 MHz, D₂O) δ 165.43, 154.63, 154.38, 151.78, 151.58, 151.00, 148.49, 148.16, 140.26, 139.73, 139.41, 128.38, 118.31, 102.18, 87.22, 86.59, 85.81, 83.68, 83.59, 81.97, 81.52, 74.19, 71.95, 71.44, 70.34, 64.91, 63.88, 63.45, 58.56, 57.99, 57.79, 46.58, 8.18 ppm. ³¹P NMR (162 MHz, D₂O) δ 0.89 (1P), -0.52 (1P), -0.97 (1P) ppm. HR-ESI-MS calcd. for C₃₁H₄₀N₁₂O₂₁P₃, 1009.16493 [M - H]⁻; found 1009.15687.

pGUA (triethylammonium salt, ε₂₆₀ = 35,900 M⁻¹•cm⁻¹, 33.8% yield) (60): ¹H NMR (600 MHz, D₂O) δ 8.39 (s, 1H), 8.16 (s, 1H), 8.09 (s, 1H), 7.81 (d, J = 8.2 Hz, 1H), 7.38 (s, 1H), 6.08 (d, J = 5.3 Hz, 1H), 5.90 (dd, J = 13.3, 4.8 Hz, 2H), 5.75 (d, J = 8.2 Hz, 1H), 4.97 (dt, J = 8.2, 4.5 Hz, 1H), 4.74 (t, J = 5.3 Hz, 1H), 4.69 (dt, J = 7.8, 5.1 Hz, 1H), 4.56 - 4.48 (m, 3H), 4.38 (dp, J = 6.6, 2.8 Hz, 2H), 4.26 - 4.10 (m, 7H), 4.00 (t, J= 4.8 Hz, 1H), 3.54 (s, 3H), 3.37 (s, 3H), 3.19 (q, J = 7.4 Hz, 18H), 1.27 (t, J = 7.3 Hz, 27H). ¹³C NMR (151 MHz, D₂O) δ 165.54, 158.45, 154.82, 153.63, 151.94, 151.24, 148.79, 140.59, 139.76, 137.07, 128.50, 118.58, 115.96, 102.37, 87.46, 86.74, 85.61, 83.74, 82.97, 82.05, 81.67, 74.29, 72.31, 71.73, 70.40, 65.07, 64.13, 63.90, 58.68, 57.99, 57.94, 46.73, 8.30 ppm. ³¹P NMR (243 MHz, D₂O) δ 0.90 (1P), -0.41 (1P), -0.74 (1P) ppm. HR-ESI-MS calcd. for C₃₁H₄₀N₁₂O₂₂P₃, 1025.15985 [M - H]⁻; found 1025.15478.

pUUAG (triethylammonium salt, ε₂₆₀ = 43,900 M⁻¹•cm⁻¹, % yield) (61): ¹H NMR (600 MHz, D₂O) δ 8.27 (s, 1H), 8.04 (s, 1H), 7.83 (d, J = 8.2 Hz, 2H), 7.68 (d, J = 8.2 Hz, 1H), 7.24 (s, 4H), 5.97 (d, J = 4.9 Hz, 1H), 5.81 (d, J = 4.7 Hz, 1H), 5.74 (d, J = 5.0 Hz, 1H), 5.73 (s, 1H), 5.71 (s, 1H), 5.69 (d, J = 5.7 Hz, 1H), 4.85 - 4.79 (m, 1H), 4.61 (t, J= 5.4 Hz, 1H), 4.59 - 4.54 (m, 1H), 4.37 - 4.32 (m, 3H), 4.27 (dq, J = 4.9, 2.5 Hz, 1H), 4.20 - 4.17 (m, 2H), 4.08 - 3.92 (m, 10H), 3.84 (t, J= 5.1 Hz, 1H), 3.37 (s, 3H), 3.35 (s, 3H), 3.24 (s, 3H), 3.03 (t, J = 7.4 Hz, 24H), 1.12 (t, J = 7.4 Hz, 36H) ppm. ¹³C NMR (151 MHz, D₂O) δ 165.93, 165.60, 158.49, 154.50, 153.68, 151.56-151.21, 148.64, 141.27, 140.84, 139.78, 137.62, 128.47, 118.62, 116.24, 102.60, 102.41, 87.52, 86.55, 86.47, 85.57, 83.60, 83.54, 82.62, 82.20, 81.75, 81.44, 81.32, 73.32, 72.44, 72.09, 71.85, 70.21, 65.10, 64.81, 64.51, 63.57, 58.61, 58.02, 57.80, 57.70, 46.68, 8.26 ppm. ³¹P NMR (243 MHz, D₂O) δ 0.62 (s, 1P), -0.30 (s, 1P), -0.64 (s, 1P), -0.70 (s, 1P) ppm. ESI-TOF-MS calcd. for C₄₁H₅₂N₁₄O₃₀P₄, 672.0968 [M - 2H] ²⁻; found 672.0945.

### Synthesis of Tetranucleotide PureCap Analogs

### Synthesis Scheme

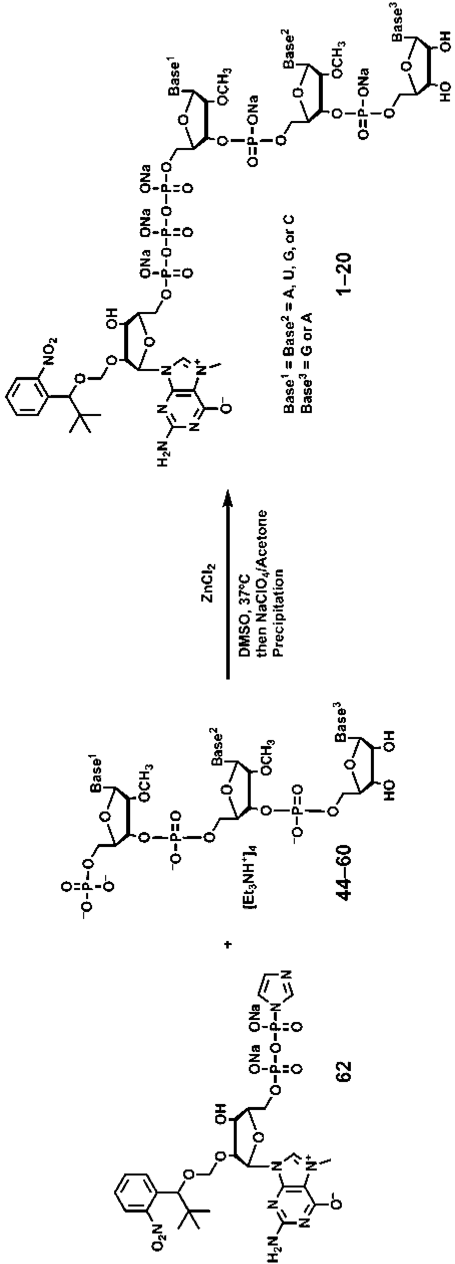

### Experimental Protocol

To a DMSO (802 µL) solution of the trinucleotide UUG triethylammonium salt (56.5 mg, 40.1 µmol), N⁷-methylguanosine diphosphate imidazolide disodium salt (92.7 mg, 120 µmol) was added, followed by zinc chloride (109 mg, 802 µmol). After incubation at 37°C for 5 days, the reaction mixture was quenched by adding 500 mM EDTA-NaOH aq. (pH of 8.0, 1.04 mmol, 2.0 mL), and diluted with water (40 mL). The crude product was purified by reversed-phase HPLC: Shimadzu preparative, column YMC-Actus Triart C8 (Preparative, 250 × 20.0 mm I.D.), solvent A: 50 mM TEAA buffer (pH of 6.0) containing 0.5% CH₃CN, solvent B: CH₃CN, linear gradient 5-80% B (25 min), flow rate: 10 ml/min, detection: 254 nm). Fractions containing the desired product were collected, concentrated, and lyophilized to give a tetranucleotide PureCap analog, m7GpppUUG, as a triethylammonium salt. The product was re-dissolved in methanol (2.0 mL), and 190 mM NaClO₄ in acetone (12 mL) was added. The resulting suspension was centrifuged (4,000 rpm, 10 min). The supernatant was discarded, and the precipitate was suspended in acetone. Suspending and centrifuging was carried out three more times. The precipitate was dried under reduced pressure to give the desired PureCap analog, m7GpppUUG (17.7 mg, 9.96 µmol, yield 24.8%) as a sodium salt. The yield was calculated using the absorbance of the product at 260 nm measured on the NanoDrop.

Nb-7mGpppUUG (sodium salt, ε₂₆₀ = 44,800 M⁻¹•cm⁻¹, 24.8% yield) (1): ¹H NMR (600 MHz, D₂O) δ 7.98 (s, 1H), 7.86 (dd, J = 13.3, 8.2 Hz, 1H), 7.80 (d, J = 8.2 Hz, 1H), 7.68 (d, J = 8.2 Hz, 0.5H), 7.59 (q, J = 8.0 Hz, 1H), 7.55 - 7.49 (m, 1.5H), 7.42 (t, J= 7.7 Hz, 0.5H), 7.31 (dt, J = 8.5, 4.7 Hz, 0.5H), 6.00 (d, J= 5.1 Hz, 0.5H), 5.92 (t, J = 7.7 Hz, 1H), 5.88 (dd, J = 4.8, 2.3 Hz, 1H), 5.85 - 5.81 (m, 3H), 5.04 (s, 0.5H), 4.99 (d, J = 7.6 Hz, 0.5H), 4.93 (s, 1H), 4.86 (d, J = 7.4 Hz, 0.5H), 4.75 (d, J = 5.4 Hz, 2.5H), 4.70 (s, 1H), 4.61 (q, J = 6.4 Hz, 1H), 4.46 (td, J = 13.4, 8.0 Hz, 2H), 4.38 (s, 1H), 4.32 - 4.23 (m, 5H), 4.19 - 4.12 (m, 4H), 4.10 (s, 1.5H), 4.04 (d, J = 6.7 Hz, 6H), 3.46 - 3.41 (m, 6H), 0.66 - 0.55 (m, 9H) ppm. ¹³C NMR (151 MHz, D₂O) δ 165.90, 165.67, 158.70, 157.99, 153.76, 151.65, 151.34, 149.75, 149.70, 149.23, 148.51, 141.21, 140.76, 137.66, 133.74, 132.70, 132.63, 132.21, 130.03, 129.97, 128.81, 128.06, 123.80, 123.41, 116.44, 108.32, 108.05, 102.92, 102.65, 95.10, 93.66, 87.57, 87.48, 87.40, 86.57, 86.01, 85.56, 85.11, 83.65, 83.59, 82.86, 82.00, 81.83, 81.51, 81.27, 80.61, 79.41, 78.92, 73.41, 72.46, 71.93, 70.46, 68.85, 68.52, 65.33, 65.02, 64.86, 64.63, 64.36, 57.91, 57.78, 36.44, 36.05, 35.71, 30.29, 24.81, 24.74, 23.31 ppm. ³¹P NMR (243 MHz, D₂O) δ -0.34 (1P), -0.56 (1P), -10.84 (t, J = 16.4 Hz, 2P), -21.98 (q, J = 16.4 Hz, 1P) ppm. HR-ESI-MS calcd. for C53H69N15037P5, 1662.26724 [M - H]-; found 1662.17281.

Nb-7mGpppUCG (sodium salt, ε₂₆₀ = 42,900 M⁻¹•cm⁻¹, 26.4% yield) (2): ¹H NMR (600 MHz, D₂O) δ 7.97 (s, 1H), 7.95 - 7.85 (m, 2H), 7.74 (d, J= 8.3 Hz, 0.5H), 7.64 (dd, J = 10.8, 7.9 Hz, 1H), 7.59 - 7.54 (m, 1.5H), 7.46 (t, J = 7.7 Hz, 0.5H), 7.37 (d, J = 5.8 Hz, 0.5H), 6.03 (d, J = 4.7 Hz, 0.5H), 6.00 - 5.94 (m, 2H), 5.92 (dd, J = 8.8, 4.4 Hz, 1H), 5.85 (dd, J = 13.6, 5.7 Hz, 1.5H), 5.79 (d, J = 4.7 Hz, 1H), 5.06 (s, 0.5H), 5.01 (d, J = 8.0 Hz, 0.5H), 4.97 (s, 0.5H), 4.94 (d, J = 7.1 Hz, 0.5H), 4.89 (d, J = 8.2 Hz, 1H), 4.86 (d, J = 6.0 Hz, 0.5H), 4.76 (s, 0.5H), 4.75 - 4.72 (m, 1H), 4.67 (q, J = 5.7 Hz, 2H), 4.61 (d, J= 3.6 Hz, 0.5H), 4.53 (t, J = 5.4 Hz, 0.5H), 4.46 (s, 1.5H), 4.39 (s, 1H), 4.35 - 4.29 (m, 4H), 4.26 (s, 0.5H), 4.21 - 4.06 (m, 10H), 4.03 (s, 1H), 3.54 - 3.52 (m, 2.5H), 3.47 - 3.45 (m, 2.5H), 3.34 (d, J = 1.2 Hz, 1H), 0.71 - 0.62 (m, 9H) ppm. ¹³C NMR (151 MHz, D₂O) δ 165.79, 165.48, 158.79, 156.68, 153.70, 151.64, 151.22, 149.82, 149.67, 149.24, 148.65, 141.12, 140.19, 137.94, 133.76, 132.70, 132.35, 130.17, 130.02, 128.86, 128.20, 123.88, 123.38, 116.51, 108.04, 107.72, 102.68, 96.22, 95.02, 87.91, 87.69, 87.48, 86.39, 83.68, 82.56, 82.00, 81.75, 81.24, 80.60, 79.14, 78.79, 73.37, 71.80, 71.13, 70.37, 68.72, 68.39, 65.01, 64.47, 63.53, 57.92, 57.66, 48.93, 36.47, 36.18, 35.76, 30.29, 24.87, 24.78 ppm. ³¹P NMR (243 MHz, D₂O) δ -0.52 (2s, 2P), -10.86 (d, J = 16.4 Hz, 2P), -22.07 (d, J = 16.4 Hz, 1P) ppm. ESI-TOF-MS calcd. for C₅₃H₆₉N₁₆O₃₆P₅, 830.1380 [M - 2H] ²⁻; found 830.1369.

Nb-7mGpppUAG (sodium salt, ε₂₆₀ = 49,300 M⁻¹•cm⁻¹, 26.4% yield) (3): ¹H NMR (600 MHz, D₂O) δ 8.34 (d, J = 2.7 Hz, 1H), 8.05 (s, 1H), 7.91 (d, J = 4.2 Hz, 1H), 7.83 - 7.71 (m, 1H), 7.62 (d, J = 8.0 Hz, 0.5H), 7.59 - 7.45 (m, 2.5H), 7.38 (t, J = 7.7 Hz, 0.5H), 7.27 (dt, J = 8.3, 4.3 Hz, 0.5H), 6.11 (t, J = 4.0 Hz, 1H), 5.98 (d, J= 5.6 Hz, 0.5H), 5.90 (dd, J = 13.5, 8.2 Hz, 1H), 5.82 - 5.76 (m, 1.5H), 5.72 - 5.64 (m, 1H), 5.04 (s, 0.5H), 5.00 - 4.91 (m, 2.5H), 4.89 (s, 0.5H), 4.69 - 4.63 (m, 2H), 4.57 (t, J = 5.5 Hz, 0.5H), 4.48 - 4.41 (m, 4H), 4.35 - 4.32 (m, 3H), 4.26 - 4.10 (m, 10H), 4.06 (s, 1.5H), 4.01 (dt, J = 10.3, 5.1 Hz, 1H), 3.52 (s, 3H), 3.40 (d, J = 4.6 Hz, 3H), 3.36 (s, 0.5H), 0.62 (s, 4.5H), 0.54 (s, 4.5H) ppm. ¹³C NMR (151 MHz, D₂O) δ 166.05, 161.52, 158.66, 155.40, 153.79, 152.79, 151.33, 149.86, 149.63, 149.30, 148.58, 148.27, 141.00, 139.15, 137.31, 133.83, 132.76, 132.50, 132.02, 129.92, 128.73, 127.84, 123.68, 123.48, 118.58, 116.32, 108.54, 102.71, 95.41, 93.78, 87.61, 87.05, 86.29, 85.47, 83.26, 82.36, 82.01, 81.30, 80.78, 80.02, 79.28, 73.57, 72.31, 70.05, 69.20, 68.87, 64.97, 64.55, 58.04, 57.63, 36.46, 35.98, 35.65, 30.33, 24.70, 24.65, 23.36 ppm. ³¹P NMR (243 MHz, D₂O) δ - 0.41 (2s, 2P), -10.72 (d, J = 19.7 Hz, 2P), -21.85 (d, J = 16.4 Hz, 1P) ppm. ESI-TOF-MS calcd. for C₅₄H₆₉N₁₈O₃₅P₅, 842.14359 [M - 2H] ²⁻; found 842.15626.

Nb-7mGpppUGG (triethylammonium salt, ε₂₆₀ = 48,920 M⁻¹•cm⁻¹, 21.0% yield) (4): ¹H-NMR (400 MHz, D₂O) δ 7.97 (s, 1H), 7.89-7.87 (m, 1H), 7.79-7.75 (m, 1H), 7.58-7.56 (m, 1H), 7.53-7.49 (m, 1H), 7.47-7.32 (m, 4H), 7.22-7.19 (m, 1H), 5.96-5.94 (m, 0.5H), 5.87-5.85 (m, 1.5H), 5.81-5.80 (m, 1H), 5.77-5.74 (m, 2H), 5.70-5.69 (m, 1H), 4.99-4.93 (m, 3H), 4.68-4.64 (m, 2H), 4.56-4.54 (m, 1H), 4.48-4.42 (m, 2H), 4.39-4.35 (m, 1H), 4.36-4.32 (m, 5H), 4.17-4.11 (m, 6H), 4.07-3.99 (m, 4H), 3.31-3.30 (m, 2H), 3.28-3.27 (m, 8H), 3.20-3.05 (m, 123H), 1.28-1.14 (m, 184H), 0.51 (d, J = 27.4 Hz, 9H) ppm. ¹³C-NMR (101 MHz, D₂O) δ 181.4, 165.9, 158.7, 153.9, 153.7, 151.7, 151.6, 151.3, 149.8, 149.6, 149.3, 148.2, 141.0, 137.5, 133.8, 132.7, 131.9, 129.9, 129.8, 128.7, 127.7, 123.6, 123.4, 116.6, 116.5, 116.3, 108.5, 102.8, 95.4, 87.2, 87.0, 86.8, 86.1, 85.9, 83.6, 83.6, 83.1, 82.7, 82.6, 81.8, 81.3, 80.6, 80.0, 79.1, 73.3, 72.9, 72.9, 72.3, 72.2, 70.4, 69.1, 68.8, 65.2, 64.9, 57.9, 57.5, 46.8, 46.6, 46.4, 36.4, 35.9, 35.9, 35.6, 24.6, 24.5, 23.3, 8.4, 8.2, 8.0 ppm. ³¹P-NMR (160 MHz, D₂O) δ -0.3, -0.6, -11.1, -11.2, -22.3, -22.4, -22.5 ppm. HRMS (ESI) Calcd. For C₅₄H₆₉N₁₈O₃₆P₅²⁻ ([M-2]²⁻): 850.1410. Obsd.850.1405.

Nb-7mGpppCUG (sodium salt, ε₂₆₀ = 41,400 M⁻¹•cm⁻¹, 26.0% yield) (5): ¹H NMR (600 MHz, D₂O) δ 7.99 (s, 1H), 7.80 (q, J = 9.1 Hz, 2H), 7.73 (d, J = 7.3 Hz, 0.5H), 7.63 (d, J = 8.2 Hz, 1H), 7.57 (d, J = 17.2 Hz, 1.5H), 7.45 (t, J = 7.6 Hz, 0.5H), 7.36 (d, J = 7.9 Hz, 0.5H), 6.04 - 5.95 (m, 1.5H), 5.91 - 5.80 (m, 2.5H), 5.74 - 5.65 (m, 2H), 5.09 (s, 0.5H), 5.05 - 4.99 (m, 1H), 4.92 (d, J = 8.8 Hz, 1H), 4.71 - 4.69 (m, 1H), 4.66 - 4.63 (m, 1H), 4.57 (s, 1H), 4.54 - 4.47 (m, 2H), 4.45 (s, 1H), 4.41 (s, 0.5H), 4.35 - 4.26 (m, 6H), 4.17 - 4.04 (m, 10H), 3.94 (s, 1H), 3.53 (d, J = 16.0 Hz, 2.5H), 3.47 (s, 2.5H), 3.34 (d, J= 2.3 Hz, 1H), 0.71 - 0.62 (m, 9H) ppm. ¹³C NMR (151 MHz, D₂O) δ 165.92, 165.33, 158.79, 156.70, 153.83, 151.67, 150.88, 149.76, 149.23, 148.73, 140.56, 139.83, 137.54, 133.70, 132.82, 132.73, 132.33, 130.01, 128.84, 128.22, 123.89, 123.46, 116.37, 107.98, 107.75, 102.26, 96.37, 95.08, 87.85, 87.34, 86.85, 83.66, 81.71, 81.49, 80.80, 79.45, 79.10, 73.61, 71.00, 70.46, 68.55, 68.14, 65.08, 64.24, 63.34, 57.82, 57.50, 48.93, 36.40, 36.15, 35.79, 30.30, 24.92, 24.84 ppm. ³¹P NMR (243 MHz, D₂O) δ -0.64 (2s, 2P), -10.44 - - 11.01 (m, 2P), -21.92 (d, J = 16.4 Hz, 1P) ppm. ESI-TOF-MS calcd. for C₅₃H₆₉N₁₆O₃₆P₅, 830.1380 [M - 2H] ²⁻; found 830.1369.

Nb-7mGpppCCG (sodium salt, ε₂₆₀ = 39,900 M⁻¹•cm⁻¹, 14.4% yield) (6): ¹H NMR (600 MHz, D₂O) δ7.79 - 7.58 (m, 3.5H), 7.57 - 7.41 (m, 2.5H), 7.37 - 7.20 (m, 1H), 5.88 - 5.61 (m, 5H), 5.40 (s, 0.5H), 5.02 - 4.89 (m, 1.5H), 4.81 (s, 1H), 4.46 (s, 2.5H), 4.37 - 4.26 (m, 3H), 4.18 (s, 7.5H), 4.09 - 3.93 (m, 8H), 3.90 - 3.80 (m, 3H), 3.44 (t, J = 11.0 Hz, 6H), 0.65 - 0.49 (m, 9H) ppm. ¹³C NMR (151 MHz, D₂O) δ 165.79, 165.47, 158.84, 156.46, 153.66, 151.48, 149.72, 149.24, 140.36, 139.06, 133.77, 132.71, 132.26, 130.04, 128.81, 128.13, 123.87, 123.51, 116.37, 115.36, 107.96, 95.96, 95.18, 87.61, 81.96, 81.46, 80.98, 80.19, 79.60, 79.20, 73.56, 70.11, 64.58, 63.50, 62.54, 57.69, 57.42, 36.42, 36.11, 35.78, 30.29, 24.88, 24.80, 23.31 ppm. ³¹P NMR (243 MHz, D₂O) δ-0.41 (1P), -0.93 (1P), -10.71 (2P), -21.78 (1P) ppm. HR-ESI-MS calcd. for C53H71N17035P5, 1660.29920 [M - H]-; found 1660.29965.

Nb-7mGpppCAG (sodium salt, ε₂₆₀ = 45,700 M⁻¹•cm⁻¹, 16.6% yield) (7): ¹H NMR (600 MHz, D₂O) δ 8.36 (s, 1H), 8.08 (s, 1H), 7.90 (s, 2H), 7.63 (d, J= 6.5 Hz, 0.5H), 7.57 (d, J = 8.0 Hz, 1H), 7.49 (s, 1.5H), 7.37 (s, 0.5H), 7.31 (s, 0.5H), 6.12 - 6.05 (m, 2H), 6.01 (s, 0.5H), 5.83 (d, J= 4.3 Hz, 0.5H), 5.79 - 5.75 (m, 1H), 5.72 (s, 1H), 5.03 - 4.96 (m, 1H), 4.91 (s, 1H), 4.85 (d, J = 7.6 Hz, 1H), 4.71 - 4.68 (m, 2H), 4.57 (s, 1H), 4.47 - 4.42 (m, 3H), 4.36 - 4.28 (m, 6H), 4.20 - 4.04 (m, 11H), 3.48 (d, J= 2.6 Hz, 2.5H), 3.45 - 3.43 (m, 2.5H), 3.34 (d, J = 2.9 Hz, 1H), 0.61 - 0.54 (m, 9H) ppm. ¹³C NMR (151 MHz, D₂O) δ 163.29, 158.41, 155.35, 154.61, 153.64, 150.47, 149.74, 149.11, 148.78, 148.27, 141.81, 139.70, 132.61, 130.06, 128.22, 123.81, 123.37, 118.47, 116.13, 107.78, 107.53, 96.21, 87.92, 87.69, 86.94, 85.88, 83.42, 82.23, 81.53, 80.42, 79.01, 73.37, 72.17, 70.17, 68.10, 66.07, 65.01, 64.27, 57.98, 57.63, 48.92, 36.30, 36.14, 35.71, 30.28, 24.87, 24.79 ppm. ³¹P NMR (243 MHz, D₂O) δ -0.48 (2s, 2P), -10.80 (2P), -21.97 (1P) ppm. ESI-TOF-MS calcd. for C₅₄H₇₀N₁₉O₃₄P₅, 841.6516 [M - 2H] ²⁻; found 841.6548.

Nb-7mGpppCGG (triethylammonium salt, ε₂₆₀ = 46,330 M⁻¹•cm⁻¹, 21.0% yield) (8): ¹H-NMR (400 MHz, D₂O) δ 9.90-9.89 (0H), 7.80-7.62 (m, 4H), 7.39-7.09 (m, 5H), 5.85-5.80 (m, 2H), 5.64-5.60 (m, 3H), 5.48 (s, 1H), 4.80 (s, 2H), 4.51-3.94 (m, 12H), 3.88 (d, J = 10.4 Hz, 3H), 3.28-3.15 (m, 6H), 0.32 (d, J = 12.7 Hz, 9H) ppm. ¹³C-NMR (100 MHz, D₂O) δ 180.6, 164.9, 158.3, 155.5, 155.4, 154.7, 153.6, 153.4, 151.3, 151.2, 149.6, 149.3, 149.0, 148.8, 137.3, 132.6, 132.1, 129.9, 129.7, 128.7, 123.7, 123.3, 116.1, 116.1, 107.5, 107.3, 96.4, 87.8, 87.5, 86.3, 81.5, 81.3, 80.7, 73.3, 72.1, 70.1, 68.3, 64.8, 64.4, 63.8, 57.8, 57.4, 46.6, 36.1, 35.7, 24.9, 24.8, 22.8, 22.6, 10.5, 8.2, 8.0, 7.3 ppm. ³¹P-NMR (160 MHz, D₂O) δ -0.4, -0.6, -10.9, -11.1, -22.5 ppm. HRMS (ESI) Calcd. For C₅₄H₇₂N₁₉O₃₅P₅1701.3126. Obsd. 1701.2997.

Nb-7mGpppAUG (sodium salt, ε₂₆₀ = 49,200 M⁻¹•cm⁻¹, 19.6% yield) (9): ¹H NMR (600 MHz, D₂O) δ 9.09 (s, 0.25H), 8.89 (s, 0.25H), 8.50 - 8.39 (m, 1H), 8.11 (d, J = 12.9 Hz, 1H), 7.98 (s, 1H), 7.74 (d, J = 8.2 Hz, 1H), 7.66 (d, J= 7.8 Hz, 0.5H), 7.58 (t, J = 6.7 Hz, 1H), 7.49 (d, J = 10.4 Hz, 1.5H), 7.40 (t, J = 7.2 Hz, 0.5H), 7.31 (t, J = 7.5 Hz, 0.5H), 6.05 - 5.96 (m, 1H), 5.82 (dt, J= 5.0, 2.5 Hz, 2.5H), 5.70 (d, J= 8.3 Hz, 1H), 5.64 (dd, J = 5.1, 2.3 Hz, 0.5H), 5.00 (d, J= 2.3 Hz, 0.5H), 4.95 - 4.89 (m, 2H), 4.84 (d, J = 1.9 Hz, 1H), 4.73 - 4.68 (m, 3H), 4.53 (s, 1.5H), 4.46 (t, J = 4.6 Hz, 2.5H), 4.36 (h, J = 5.0 Hz, 1H), 4.33 - 4.29 (m, 3H), 4.25 (s, 2H), 4.18 - 4.09 (m, 6H), 4.06 (d, J = 2.3 Hz, 1H), 4.01 (t, J= 4.3 Hz, 2.5H), 3.45 (dd, J = 6.2, 2.4 Hz, 5H), 3.34 (d, J = 2.5 Hz, 1H), 0.63 - 0.55 (m, 9H) ppm. ¹³C NMR (151 MHz, D₂O) δ 165.47, 158.54, 155.38, 155.15, 154.94, 154.34, 153.65, 152.38, 151.14, 149.70, 149.29, 148.84, 148.61, 140.40, 139.62, 133.68, 132.65, 132.30, 129.90, 128.78, 128.12, 123.81, 123.34, 107.23, 102.50, 94.71, 93.48, 87.59, 87.35, 86.48, 85.39, 84.51, 83.52, 81.84, 81.55, 80.46, 79.34, 79.01, 73.50, 72.74, 71.75, 70.46, 68.53, 68.18, 65.32, 64.76, 64.29, 57.91, 48.92, 36.35, 36.14, 35.70, 30.28, 24.82, 24.74ppm. ³¹P NMR (243 MHz, D₂O) δ -0.38 (2P), -10.83 (2P), -22.05 (1P) ppm. ESI-TOF-MS calcd. for C₅₄H₆₉N₁₈O₃₅P₅, 842.1436 [M - 2H] ²⁻; found 842.1431.

Nb-7mGpppACG (sodium salt, ε₂₆₀ = 46,700 M⁻¹•cm⁻¹, 34.3% yield) (10): ¹H NMR (400 MHz, D₂O) δ 9.20 - 8.88 (m, 0.5H), 8.40 (s, 1H), 8.04 (d, J= 7.0 Hz, 1H), 7.90 (d, J = 6.7 Hz, 1H), 7.71 (d, J = 8.4 Hz, 1H), 7.62 - 7.18 (m, 4.5H), 5.99 (s, 1H), 5.89 - 5.64 (m, 4H), 4.89 (s, 3H), 4.61 - 4.48 (m, 4H), 4.44 - 4.10 (m, 15H), 4.07 - 3.95 (m, 4H), 3.60 - 3.46 (m, 6H), 0.58 - 0.38 (m, 9H) ppm. ¹³C NMR (101 MHz, D₂O) δ 164.40, 158.48, 155.23, 154.95, 154.50, 153.58, 152.22, 151.24, 149.64, 149.27, 148.86, 148.67, 148.24, 139.76, 139.28, 137.10, 133.51, 132.56, 132.22, 129.93, 128.71, 128.10, 123.74, 123.33, 118.34, 116.19, 107.48, 107.21, 95.60, 94.49, 87.65, 85.15, 83.16, 82.52, 81.77, 81.23, 80.80, 80.19, 79.34, 78.98, 73.78, 71.90, 70.68, 69.91, 68.40, 68.07, 64.62, 63.34, 57.79, 36.20, 35.61, 30.26, 24.82, 24.73 ppm. ³¹P NMR (162 MHz, D₂O) δ -0.53 (2P), -10.78 (2P), -21.90 (1P) ppm. ESI-TOF-MS calcd. for C₅₄H₇₁N₁₉O₃₄P₅, 1684.31044 [M - H]⁻; found 1684.31722.

Nb-7mGpppAAG (sodium salt, ε₂₆₀ = 52,100 M⁻¹•cm⁻¹, 37.4% yield) (11): ¹H NMR (600 MHz, D₂O) δ 9.01 - 8.67 (2s, 1H), 8.26 (d, J = 7.6 Hz, 1H), 8.10 (d, J = 11.0 Hz, 1H), 7.98 (d, J = 7.2 Hz, 1H), 7.83 - 7.68 (m, 2H), 7.40 - 6.92 (m, 4H), 5.89 - 5.75 (m, 2H), 5.65 - 5.53 (m, 1.5H), 5.38 (s, 0.5H), 4.77 (s, 3H), 4.64 - 4.57 (m, 1.5H), 4.55 - 4.39 (m, 2H), 4.39 - 3.95 (m, 16.5H), 3.91 - 3.80 (m, 3H), 3.41 - 3.26 (m, 5.5H), 3.20 (s, 0.5H), 0.35 - 0.12 (2s, 9H) ppm. ¹³C NMR (151 MHz, D₂O) δ 158.11, 155.27, 155.07, 154.25, 154.13, 153.90, 153.50, 151.46, 151.06, 149.52, 149.08, 148.81, 148.68, 148.19, 148.00, 139.48, 139.11, 136.92, 133.32, 132.49, 132.13, 129.85, 129.61, 128.58, 128.06, 123.62, 123.20, 119.15, 118.27, 117.98, 115.95, 107.46, 107.18, 93.37, 87.66, 87.45, 85.55, 84.91, 83.16, 82.77, 82.22, 81.81, 80.88, 80.03, 79.37, 78.91, 73.73, 72.38, 72.15, 70.07, 67.92, 64.97, 64.54, 58.06, 58.03, 57.75, 48.91, 36.13, 36.10, 36.03, 35.57, 24.75 ppm. ³¹P NMR (243 MHz, D₂O) δ -0.38 (2s, 2P), -10.76 (d, J = 19.7 Hz, 2P), -21.82 (t, J = 19.7 Hz, 1P) ppm. ESI-TOF-MS calcd. for C₅₅H₇₀N₂₁O₃₃P₅, 853.65720 [M - 2H]²⁻; found 853.66766.

Nb-7mGpppAGG (sodium salt, ε₂₆₀ = 50,200 M⁻¹•cm⁻¹, 36.0% yield) (12): ¹H NMR (600 MHz, D₂O) δ 8.42-8.34 (m, 1H), 8.06 (d, J = 12.4 Hz, 1.5H), 7.97 (s, 1H), 7.84 (s, 1H), 7.67 (s, 0.5H), 7.57 (d, J= 7.6 Hz, 1H), 7.51-7.36 (m, 2H), 7.29 (s, 1H), 6.88 (s, 1H), 5.94 (dd, J = 11.6, 5.1 Hz, 2H), 5.88-5.80 (m, 1.5H), 5.73 (s, 1H), 5.68 (d, J= 4.3 Hz, 0.5H), 4.89 (s, 4H), 4.58 (s, 2.5H), 4.52 (s, 0.5H), 4.46 (s, 3H), 4.38 (s, 3H), 4.34-4.24 (m, 8H), 4.21-4.08 (m, 11H), 4.05 (s, 1.5H), 4.00 (s, 1.5H), 3.83-3.47 (m, 6H), 0.64-0.46 (m, 9H) ppm. ¹³C NMR (151 MHz, D₂O) δ 159.70-158.2 (m), 153.71-150.8 (m), 149.70-148.00 (m), 132.07, 129.36, 128.17, 123.14, 117.26, 114.91, 107.66, 87.66, 84.99, 82.62, 80.56, 79.37, 75.68, 72.44, 68.13, 65.01, 61.77, 58.27, 57.91, 56.82, 48.94, 46.69, 36.13, 35.36, 30.30, 28.25, 24.76, 20.57, 16.84, 13.30, 8.32 ppm. ³¹P NMR (243 MHz, D₂O) δ -0.26 (2P), -10.84 (2P), -22.09 (1P) ppm. HR-ESI-MS calcd. for C₅₅H₇₀N₂₁O₃₄P₅, 861.6546 [M - 2H]²⁻; found 861.6557

Nb-7mGpppGUG (sodium salt, ε₂₆₀ = 46,400 M⁻¹•cm⁻¹, 31.5% yield) (13): ¹H NMR (400 MHz, D₂O) δ 8.15 - 7.93 (m, 2H), 7.72 (s, 1H), 7.63 - 7.18 (m, 4H), 5.90 - 5.59 (m, 5H), 4.97 (d, J = 14.5 Hz, 3H), 4.73-4.63 (m, 3H), 4.58 - 3.80 (m, 20H), 3.53 - 3.30 (m, 6H), 0.72 - 0.31 (m, 9H) ppm. ¹³C NMR (101 MHz, D₂O) δ 165.39, 158.51, 156.35, 153.86, 153.65, 151.53, 151.16, 149.56, 149.33, 148.86, 140.34, 137.35, 136.81, 133.45, 132.56, 132.05, 129.86, 128.72, 128.09, 123.75, 123.37, 116.20, 115.72, 107.76, 107.44, 102.64, 94.32, 93.54, 87.64, 87.20, 86.18, 84.43, 83.54, 82.13, 81.64, 80.34, 79.50, 78.96, 73.37, 72.77, 72.00, 70.44, 68.12, 65.35, 64.38, 57.78, 36.18, 35.67, 30.26, 30.22, 24.76 ppm. ³¹P NMR (162 MHz, D₂O) δ -0.27 (2P), -10.82 (2P), -22.06 (1P) ppm. ESI-TOF-MS calcd. for C₅₄H₇₀N₁₈O₃₆P₅, 1701.28937 [M - H]⁻; found 1701.28013.

Nb-7mGpppGCG (sodium salt, ε₂₆₀ = 43,100 M⁻¹•cm⁻¹, 11.6% yield) (14): ¹H NMR (400 MHz, D₂O) δ 9.21 - 8.73 (m, 1H), 7.89 (s, 2H), 7.72 - 7.22 (m, 5H), 5.80 (s, 2H), 5.57 (s, 1.5H), 5.43 (s, 1.5H), 5.02 (d, J = 16.2 Hz, 3H), 4.68 (d, J = 14.2 Hz, 3H), 4.61 - 3.90 (m, 20H), 3.61 (t, J= 13.8 Hz, 6H), 0.59 (q, J = 12.7 Hz, 9H) ppm. ¹³C NMR (151 MHz, D₂O) δ 164.95, 158.94, 158.48, 155.47, 155.22, 154.66, 154.54, 154.13, 153.51, 150.97, 149.55, 149.35, 148.83, 148.52, 139.21, 136.70, 133.92, 132.75, 132.26, 129.96, 128.78, 128.08, 123.84, 123.43, 116.45, 115.80, 107.62, 107.34, 95.34, 93.60, 88.29, 87.59, 85.68, 81.59, 80.68, 73.97, 70.40, 69.38, 68.82, 68.34, 65.11, 64.15, 57.53, 36.41, 36.19, 35.77, 30.28, 25.20, 24.80, 24.76, 24.72 ppm. ³¹P NMR (162 MHz, D₂O) δ - 0.58 (2P), -10.85 (2P), -22.17 (1P) ppm. ESI-TOF-MS calcd. for C₅₄H₇₁N₁₆O₃₅P₅, 1700.40535 [M - H]⁻; found 1700.36111.

Nb-7mGpppGAG (sodium salt, ε₂₆₀ = 49,900 M⁻¹•cm⁻¹, 32.3% yield) (15): ¹H NMR (400 MHz, D₂O) δ 9.08 - 8.68 (m, 0.5H), 8.18 (d, J = 8.0 Hz, 1H), 7.98 - 7.73 (m, 3H), 7.42 - 6.99 (m, 4H), 5.86 (d, J = 6.4 Hz, 1H), 5.68 - 5.38 (m, 3.5H), 4.86 (s, 2H), 4.75 (d, J = 8.1 Hz, 3.5H), 4.56 (s, 2H), 4.42 - 4.24 (m, 4H), 4.23 - 3.97 (m, 10H), 3.93 - 3.77 (m, 4.5H), 3.45 - 3.16 (m, 6H), 0.49 - 0.10 (m, 9H) ppm. ¹³C NMR (101 MHz, D₂O) δ 158.33, 155.12, 154.17, 153.71, 153.51, 151.46, 151.22, 148.79, 139.09, 137.08, 133.22, 129.62, 118.27, 116.04, 115.55, 107.43, 107.12, 87.99, 87.34, 85.60, 83.36, 82.84, 80.93, 73.37, 70.08, 67.82, 65.03, 58.05, 57.91, 36.14, 36.01, 35.64, 30.26, 24.81, 24.72 ppm. ³¹P NMR (159 MHz, D₂O) δ -0.28 (2P), -10.82 (2P), -22.11 (1P) ppm. ESI-TOF-MS calcd. for C₅₅H₇₁N₂₁O₃₄P₅, 1724.31659 [M - H]⁻; found 1724.31732.

Nb-7mGpppGGG (sodium salt, ε₂₆₀ = 46,800 M⁻¹•cm⁻¹, 28.8% yield) (16): ¹H NMR (600 MHz, D₂O) δ 8.20 (s, 0.5H), 7.91 (s, 3H), 7.26 (s, 4.5H), 6.12 (s, 2H), 5.96 (s, 0.5H), 5.77 (s, 1.5H), 5.12 (d, J = 1.9 Hz, 1.5H), 4.85 (s, 1.5H), 4.67 - 3.65 (m, 29H), 0.15 (s, 9H) ppm. ¹³C NMR (151 MHz, D₂O) δ 160.45, 159.60, 155.75-147.96, 136.37-127.56, 123.60, 116.54-113.91, 107.66, 94.16, 88.36-85.23, 83.43, 81.62-78.68, 75.72, 71.70, 69.97, 69.59, 68.54-67.97, 67.30, 65.70, 64.85-63.23, 61.99-61.52, 60.01, 57.92-56.88, 36.05, 35.44, 30.35, 24.78 ppm. ³¹P NMR (243 MHz, D₂O) δ 0.91 - -2.52 (2P), -10.37 (2P), -21.96 (1P) ppm. ESI-TOF-MS calcd. for C₅₅H₇₁N₂₁O₃₅P₅, 1740.31150 [M - H]⁻; found 1740.31166.

Nb-7mGpppUUA (sodium salt, ε₂₆₀ = 49,400 M⁻¹•cm⁻¹, 7.39% yield) (17): ¹H NMR (600 MHz, D₂O) δ 8.44 (d, J = 4.7 Hz, 1H), 8.19 (d, J = 6.7 Hz, 1H), 7.90 - 7.84 (m, 1H), 7.80 (d, J = 8.0 Hz, 1H), 7.69 (t, J = 6.8 Hz, 0.5H), 7.62 (t, J = 6.9 Hz, 0.5H), 7.57 (d, J = 7.8 Hz, 1H), 7.53 (d, J = 5.4 Hz, 1H), 7.44 (d, J = 6.2 Hz, 0.5H), 7.31 (d, J = 6.5 Hz, 0.5H), 6.08 (t, J = 6.0 Hz, 1H), 6.00 (d, J = 5.9 Hz, 0.5H), 5.91 (dd, J = 16.9, 5.7 Hz, 2H), 5.82 (q, J = 7.1 Hz, 2.5H), 5.08 (d, J = 6.9 Hz, 1H), 5.01 (q, J = 7.1 Hz, 2H), 4.94 (d, J = 6.3 Hz, 1H), 4.63 (d, J = 17.8 Hz, 2H), 4.53 - 4.50 (m, 1H), 4.46 (s, 1H), 4.42 (s, 1H), 4.37 (s, 1H), 4.31 (s, 4H), 4.23 (s, 0.5H), 4.18 (s, 4H), 4.11 (d, J = 6.6 Hz, 1H), 4.06 (d, J = 6.5 Hz, 6.5H), 3.45 - 3.41 (m, 6H), 0.67 (d, J= 6.6 Hz, 5.5H), 0.60 (d, J = 6.5 Hz, 3.5H) ppm. ¹³C NMR (151 MHz, D₂O) δ 165.72, 155.57, 152.91, 151.39, 149.00, 148.23, 141.19, 140.82, 139.68, 133.90, 132.59, 132.12, 129.98, 127.84, 123.45, 108.43, 102.86, 102.65, 95.42, 87.34, 86.52, 86.00, 83.59, 82.28, 81.52, 81.26, 79.13, 74.27, 72.51, 72.01, 70.33, 69.16, 65.10, 64.32, 57.91, 57.73, 36.53, 35.96, 30.28, 24.66 ppm. ³¹P NMR (243 MHz, D₂O) δ -0.53 (2s, 2P), -10.68 - -11.20 (m, 2P), -22.14 (1P) ppm. ESI-TOF-MS calcd. for C₅₃H₆₉N₁₅O₃₆P₅, 1646.2732 [M - H]⁻; found 1646.44951.

Nb-7mGpppCUA (sodium salt, ε₂₆₀ = 46,000 M⁻¹•cm⁻¹, 38.9% yield) (18): ¹H NMR (600 MHz, D₂O) δ 8.40 (d, J = 11.7 Hz, 1H), 8.10 (d, J = 14.2 Hz, 1H), 7.77 (s, 2.5H), 7.66 - 7.50 (m, 2.5H), 7.46 - 7.30 (m, 1H), 6.07 - 5.94 (m, 2.5H), 5.91 - 5.82 (m, 1.5H), 5.68 (s, 1H), 5.58 (s, 1H), 5.13 - 5.02 (m, 2H), 4.96 (s, 1H), 4.88 (s, 2H), 4.67 - 4.57 (m, 2H), 4.55 - 4.44 (m, 3H), 4.39 - 4.25 (m, 6H), 4.21 - 4.02 (m, 9H), 3.94 (s, 1H), 3.57 - 3.29 (m, 6H), 0.75 - 0.53 (m, 9H) ppm. ¹³C NMR (151 MHz, D₂O) δ 165.79, 165.31, 156.31, 156.01, 155.85, 155.32, 152.60, 150.72, 149.65, 149.44, 149.18, 148.76, 139.72, 139.37, 136.28, 133.49, 132.99, 132.73, 132.34, 130.14, 129.94, 128.77, 128.39, 123.94, 123.53, 118.46, 107.49, 102.12, 96.30, 95.03, 88.40, 87.31, 86.73, 83.47, 81.79, 81.41, 81.00, 79.53, 74.62, 70.99, 70.09, 67.90, 67.40, 64.82, 63.76, 63.21, 57.81, 57.28, 36.19, 35.85, 30.26, 24.99 ppm. ³¹P NMR (243 MHz, D₂O) δ -0.65 (2s, 2P), -10.73 (2P), -21.94 (1P) ppm. ESI-TOF-MS calcd. for C₅₃H₇₀N₁₆O₃₅P₅, 1645.28831 [M - H]⁻; found 1645.28716.

Nb-7mGpppAUA (sodium salt, ε₂₆ₒ = 53,800 M⁻¹•cm⁻¹, 32.4% yield) (19) : ¹H NMR (600 MHz, D₂O) δ 8.55 - 8.30 (m, 2H), 8.21 - 8.02 (m, 2H), 7.76 (dd, J= 8.1, 3.4 Hz, 1H), 7.64 - 7.22 (m, 4H), 6.08 - 5.97 (m, 2H), 5.89 - 5.81 (m, 1.5H), 5.76 - 5.62 (m, 1.5H), 4.98 - 4.88 (m, 3H), 4.84 (s, 1H), 4.72 - 4.69 (m, 1H), 4.66 - 4.62 (m, 1.5H), 4.55 (d, J= 4.8 Hz, 1.5H), 4.48 (q, J = 7.4 Hz, 1.5H), 4.43 (d, J = 5.7 Hz, 1H), 4.38 - 4.32 (m, 4.5H), 4.25 (s, 2H), 4.22 - 4.17 (m, 2H), 4.17 - 4.10 (m, 3H), 4.06 - 3.97 (m, 4H), 3.43 (d, J= 4.8 Hz, 5.7H), 3.34 (d, J = 2.2 Hz, 0.3H), 0.58 - 0.46 (m, 9H) ppm. ¹³C NMR (151 MHz, D₂O) δ 165.48, 155.36, 155.16, 154.41, 154.30, 154.14, 151.19, 149.70, 149.23, 148.84, 148.63, 140.58, 140.00, 136.29, 133.54, 132.60, 132.27, 130.00, 129.82, 128.75, 128.16, 123.76, 123.35, 118.53, 118.13, 107.61, 107.31, 102.54, 94.51, 93.49, 87.70, 87.55, 86.54, 84.85, 84.53, 83.57, 82.26, 82.14, 82.00, 81.61, 81.36, 80.26, 79.47, 79.03, 74.54, 72.90, 71.91, 70.23, 68.44, 68.13, 65.31, 65.05, 64.67, 64.33, 58.01, 57.94, 36.25, 36.13, 35.67, 30.32, 24.86, 24.78 ppm. ³¹P NMR (243 MHz, D₂O) δ - 0.41 (2P), -10.71 (2P), -21.75 (1P) ppm. ESI-TOF-MS calcd. for C₅₄H₇₀N₁₈O₃₄P₅, 1669.29954 [M - H]⁻; found 1669.29949.

Nb-7mGpppGUA (sodium salt, ε₂₆ₒ = 51,000 M⁻¹•cm⁻¹, 24.3% yield) (20) : ¹H NMR (600 MHz, D₂O) δ 8.24 (s, 1H), 8.00 - 7.96 (m, 1H), 7.96 - 7.88 (m, 1H), 7.63 (d, J = 8.2 Hz, 1H), 7.49 (d, J= 8.2 Hz, 0.5H), 7.42 (t, J = 9.8 Hz, 1H), 7.36 - 7.31 (m, 1.5H), 7.25 (d, J = 7.1 Hz, 0.5H), 7.16 (t, J = 7.8 Hz, 0.5H), 5.97 - 5.88 (m, 1H), 5.75 - 5.69 (m, 1.5H), 5.66 - 5.60 (m, 2H), 5.56 - 5.53 (m, 0.5H), 4.90 (s, 0.5H), 4.82 (t, J = 9.7 Hz, 2H), 4.76 (s, 1H), 4.60 - 4.56 (m, 2.5H), 4.42 (s, 0.5H), 4.40 - 4.37 (m, 2H), 4.33 (s, 1H), 4.29 (s, 0.5H), 4.25 - 4.22 (m, 1.5H), 4.18 (s, 1H), 4.13 - 4.02 (m, 7.5H), 3.94 - 3.86 (m, 6H), 3.34 - 3.29 (m, 6H), 0.47 - 0.38 (m, 9H) ppm. ¹³C NMR (151 MHz, D₂O) δ 165.53, 158.49, 157.94, 155.43, 153.70, 152.75, 151.63, 151.26, 149.66, 149.40, 148.88, 148.76, 148.47, 140.58, 139.63, 136.95, 133.68, 132.69, 132.19, 129.93, 128.74, 127.97, 123.75, 123.43, 118.66, 115.83, 107.73, 102.58, 94.74, 93.49, 87.60, 87.38, 87.23, 86.31, 84.50, 83.54, 81.96, 81.59, 80.47, 79.76, 79.20, 74.30, 72.96, 71.98, 70.29, 68.67, 68.30, 65.50, 65.13, 64.90, 64.28, 57.89, 36.35, 36.04, 35.65, 30.29, 24.75, 24.71 ppm. ³¹P NMR (243 MHz, D₂O) δ -0.39 (2s, 2P), -10.73 (d, J = 19.7 Hz, 2P), -22.04 (1P) ppm. ESI-TOF-MS calcd. for C₅₄H₇₀N₁₈O₃₅P₅, 1685.29445 [M - H]⁻; found 1685.29855.

### Synthesis of Pentanucleotide PureCap Analog

### Synthesis Scheme

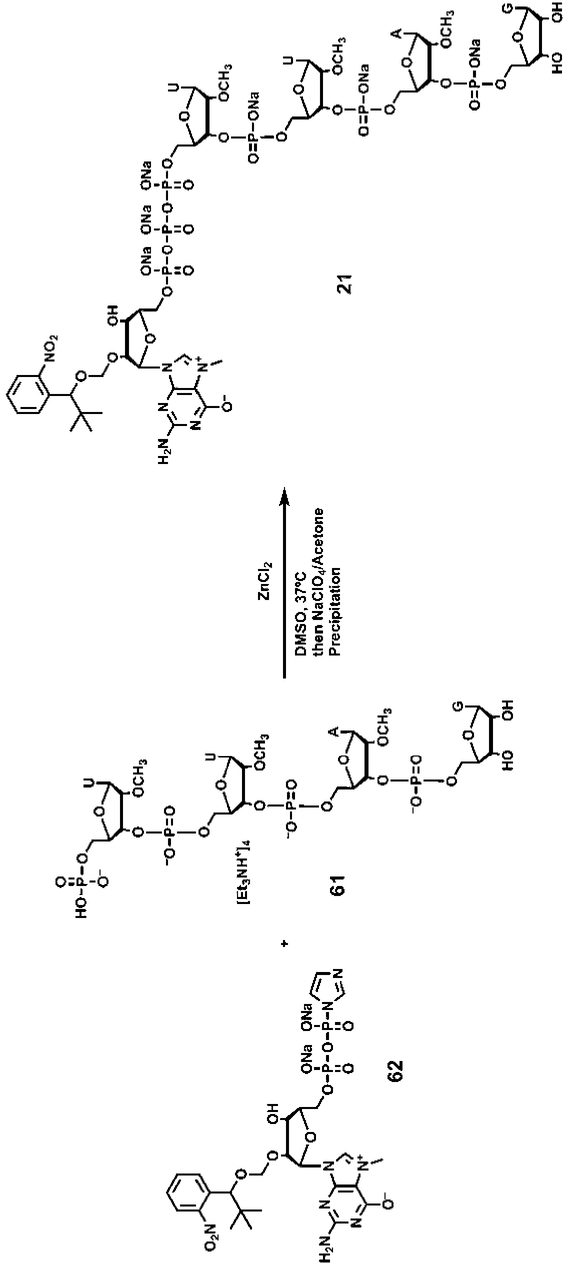

Nb-7mGpppUUAG (sodium salt, ε₂₆ₒ = 59,000 M⁻¹•cm⁻¹, 29.2% yield) (21) : ¹H NMR (600 MHz, D₂O) δ 8.36 (s, 1H), 8.12 (d, J = 3.1 Hz, 1H), 7.95 (s, 1H), 7.88 (dd, J = 16.5, 8.1 Hz, 1H), 7.80 (d, J = 8.2 Hz, 1H), 7.65 - 7.47 (m, 3H), 7.39 (t, J = 7.2 Hz, 0.5H), 7.29 (dt, J = 8.7, 4.1 Hz, 0.5H), 6.10 (d, J = 4.9 Hz, 1H), 6.02 (d, J = 5.4 Hz, 0.5H), 5.95 (t, J = 8.8 Hz, 1H), 5.89 - 5.81 (m, 4.5H), 5.05 (s, 0.5H), 4.98 (q, J= 7.5 Hz, 2H), 4.90 (s, 0.5H), 4.84 (d, J= 7.1 Hz, 1.5H), 4.71 (q, J = 6.8 Hz, 2.5H), 4.65 (t, J = 4.3 Hz, 0.5H), 4.60 (t, J = 5.3 Hz, 0l5H) , 4.47 (tt, J = 14.9, 4.1 Hz, 5H), 4.33 (d, J = 15.4 Hz, 4.5H), 4.22 (d, J = 14.3 Hz, 5H), 4.14 (d, J = 13.3 Hz, 3H), 4.08 (d, J = 8.5 Hz, 4H), 3.99 (s, 1H), 3.51 (s, 3H), 3.44 (d, J= 4.2 Hz, 3H), 3.40 (s, 3H), 3.36 (s, 0.5H), 0.63 (s, 5H), 0.55 (s, 4H) ppm.¹³C NMR (151 MHz, D₂O) δ 166.08, 165.81, 161.46, 158.69, 155.43, 153.82, 152.90, 151.39, 149.88, 149.68, 149.34, 148.71, 148.32, 141.17, 140.87, 139.31, 137.40, 133.80, 132.73, 132.50, 132.03, 129.91, 128.75, 127.88, 123.69, 123.48, 118.67, 116.33, 108.60, 102.87, 102.69, 95.35, 93.75, 87.55, 87.12, 86.94, 86.48, 86.16, 85.47, 83.37, 82.78, 82.52, 82.16, 81.88, 81.37, 80.72, 79.90, 79.16, 73.59, 72.43, 72.26, 70.13, 69.15, 68.83, 65.04, 64.81, 64.66, 58.08, 57.78, 57.73, 36.46, 35.97, 35.65, 30.33, 24.73, 24.67, 23.37 ppm. ³¹P NMR (241 MHz, D₂O) δ -0.22 (s, 1P), -0.52 (d, J = 13.2 Hz, 2P), -10.75 (d, J = 19.7 Hz, 2P), -21.84 (q, J = 16.4 Hz, 1P) ppm. ESI-TOF-MS calcd. for C₆₄H₈₁N₂₀O₄₃P₆, 667.77362 [M - 3H]³-; found 667.78314

### Test Example 5-2. Synthesis of mRNA by co-transcription reactions using TetraPureCap analogs

Linear RNA was synthesized by transcription using double-stranded DNA as a template and using T7 RNA polymerase. The template double-stranded DNA was obtained by amplifying pNL-TK1.1 (Promega) by PCR reaction using KOD-Plus-Neo (TOYOBO) (1.0 ng/µL DNA vector, 1 × KOD buffer, 0.2 mM dNTPs, 0.3 µM primers, 1.5 mM MgSO₄, 0.02 units/µL polymerase). The primers used are as follows. Fw 1; CCCGGATCCTAATACGACTCACTATAGGCGCATATTAAGGTGACGCGT (SEQ ID NO: 24) Fw 2; CCCGGATCCTAATACGACTCACTATAAGGCGCATATTAAGGTGACGCGT (SEQ ID NO: 25) Fw 3; CCCGGATCCTAATACGACTCACTATATGGCGCATATTAAGGTGACGCGT (SEQ ID NO: 26) Fw 4; CCCGGATCCTAATACGACTCACTATACGGCGCATATTAAGGTGACGCGT (SEQ ID NO: 27) Fw 5; CCCGGATCCTAATACGACTCACTATAGGGCGCATATTAAGGTGACGCGT (SEQ ID NO: 28) Fw 6; CCCGGATCCTAATACGACTCACTATATCGGCGCATATTAAGGTGACGCGT (SEQ ID NO: 29) Fw 7; CCCGGATCCTAATACGACTCACTATATAGGCGCATATTAAGGTGACGCGT (SEQ ID NO: 30) Fw 8; CCCGGATCCTAATACGACTCACTATATTGGCGCATATTAAGGTGACGCGT (SEQ ID NO: 31) Fw 9; CCCGGATCCTAATACGACTCACTATACCGGCGCATATTAAGGTGACGCGT (SEQ ID NO: 32) Fw 10; CCCGGATCCTAATACGACTCACTATACTGGCGCATATTAAGGTGACGCGT (SEQ ID NO: 33) Fw 11; CCCGGATCCTAATACGACTCACTATACAGGCGCATATTAAGGTGACGCGT (SEQ ID NO: 34) Fw 12; CCCGGATCCTAATACGACTCACTATAGTGGCGCATATTAAGGTGACGCGT (SEQ ID NO: 35) Fw 13; CCCGGATCCTAATACGACTCACTATAGCGGCGCATATTAAGGTGACGCGT (SEQ ID NO: 36) Fw 14; CCCGGATCCTAATACGACTCACTATAGAGGCGCATATTAAGGTGACGCGT (SEQ ID NO: 37) Fw 15; CCCGGATCCTAATACGACTCACTATAATGGCGCATATTAAGGTGACGCGT (SEQ ID NO: 38) Fw 16; CCCGGATCCTAATACGACTCACTATAACGGCGCATATTAAGGTGACGCGT (SEQ ID NO: 39) Fw 17; CCCGGATCCTAATACGACTCACTATAAAGGCGCATATTAAGGTGACGCGT (SEQ ID NO: 40) Fw 18; CCCGGATCCTAATACGACTCACTATTAGGCGCATATTAAGGTGACGCGT (SEQ ID NO: 41) Fw 19; CCCGGATCCTAATACGACTCACTATTTAGGCGCATATTAAGGTGACGCGT (SEQ ID NO: 42) Fw 20; CCCGGATCCTAATACGACTCACTATCTAGGCGCATATTAAGGTGACGCGT (SEQ ID NO: 43) Fw 21; CCCGGATCCTAATACGACTCACTATGTAGGCGCATATTAAGGTGACGCGT (SEQ ID NO: 44) Rev; TTTTTTTTTTTTTTTTTTTTTTTTTTTTTTCTAGAATTACGCCAGAATGCG (SEQ ID NO: 45)

A transcription reaction mixture (template DNA 10 ng/µL, 1 × T7 RNA Polymerase buffer (Takara) (40 mM Tris-HCl (pH of 8.0), 8 mM MgCl₂, 2 mM spermidine), 2.5 U/µL T7 RNA polymerase (Takara), 5 mM DTT, 2 mM ATP, 2 mM UTP, 2 mM CTP, 2 mM GTP, 0.5-2 mM TetraPureCap analog) was prepared and incubated at 37°C for 1 hour. Recombinant DNase (Takara) was added to the reaction mixture at 0.1 U/µL, and incubated at 37°C for 30 minutes to degrade the template DNA.

After the reaction, 7.5 M LiCl was added in an equal amount to the IVT reaction mixture, and the mixture was allowed to stand at -30°C for 30 minutes and centrifuged at 15000 rpm for 30 minutes to obtain a precipitate. The precipitate was purified by reversed-phase HPLC. The purification conditions were as follows: column, YMC-TriartBioC4 (250 mm × 4.6 mm I.D.); solvent A, 100 mM triethylammonium acetate (pH of 7.0), 5% acetonitrile; solvent B, 100 mM triethylammonium acetate (pH of 7.0), 50% acetonitrile; Linear gradient 10-30% solvent B (0-20 min); flow rate: 1 mL/min; detection wavelength, 260 nm; column temperature, 50°C.

### Deprotection by Light Irradiation

The RNA solution was added to a transparent 96-well multiwell plate and irradiated with 365-nm light at a light intensity of 4 mW/cm² for 10 minutes using a MAX-305 light source apparatus (Asahi Spectra). HPLC purification was carried out: column, YMC-TriartBioC4 (250 mm × 4.6 mm I.D.); solvent A, 100 mM triethylammonium acetate (pH of 7.0), 5% acetonitrile; solvent B, 100 mM triethylammonium acetate (pH of 7.0), 50% acetonitrile; linear gradient 10-30% solvent B (0-20 min); flow rate: 1 mL/min; detection wavelength, 260 nm; column temperature, 50°C.

### Evaluation of Translation Activity in Cells

HeLa cells (RIKEN Cell Bank) were cultured in Dulbecco's modified Eagle's medium (DMEM; WAKO) supplemented with 10% fetal bovine serum (FBS; Invitrogen) (37°C, 5% CO₂). On the day before transfection, the HeLa cells were seeded in a 96-well plate (1.0 × 10⁴ cells/well). The next day, the medium was removed and replaced with 100 µL/well Opti-MEM (registered trademark) (Thermo Fisher SCIENTIFIC). 0.15 µL Lipofectamine (registered trademark) MessengerMAX^{™} and 5 ng mRNA were diluted in 10 µL Opti-MEM (registered trademark) and transfected into the cells. After 2 hours, the medium was replaced with Dulbecco's modified Eagle's medium (DMEM; WAKO) supplemented with 10% fetal bovine serum (FBS; Invitrogen). After incubation at 37°C for 22 hours, 20 µL/well of 1× Cell Lysis Buffer (Promega) was added to lyse the cells. Luminescence was measured using Nano-Glo (registered trademark) luciferase assay (registered trademark) (Promega).

The results are shown in Figs. 5 to 7. It was found that the ratio of the amount of synthesized capped RNA to the total transcription amount (capping efficiency) was improved by inserting base(s) into the promoter so that the base sequence of the polynucleotide of the cap analog and the base sequence upstream from the transcription initiation site of the promoter are complementary to each other in a specific positional relationship. It was also found that high translation efficiency can be obtained depending on the sequence of the polynucleotide of the cap analog.

### Test Example 6. Translation reaction using chemically synthesized circular mRNA containing an m7G cap structure

For synthesis of the RNA oligonucleotides whose sequences are shown in Fig. 8 as starting materials for circular RNA, the RNA oligonucleotide sequences were synthesized using commercially available phosphoramidite compounds (manufactured by ChemGenes and Glen Research) in accordance with a conventional method using an automated nucleic acid synthesizer NTS T8-A20R8NC (manufactured by Nihon Techno Service Co., Ltd.). A branched structure was introduced using the reagent Asymmetric Doubler (Lev) Phosphoramidite (cat# 10-1981) manufactured by Glen Research. After completion of the synthesis, a 1:1 mixture of 40% aqueous methylamine solution and 28% concentrated aqueous ammonia was added to the CPG solid support, and the mixture was incubated at 65°C for 30 minutes to cleave the oligonucleotide from the support and deprotect the phosphate and base moieties. After evaporating to dryness under reduced pressure, the residue was dissolved by adding a 1 M TBAF/THF solution, and the solution was incubated overnight at room temperature to deprotect the TOM protecting group. The resulting product was desalted using a NAP-25 gel filtration column (Cytiva) and alcohol-precipitated to obtain an RNA oligonucleotide as a precipitate. The oligonucleotide was further purified by reversed-phase HPLC. The purity and molecular weight of the resulting desired product was confirmed using a Q-TOF LC/MS System (Agilent Technologies). The 5'-end phosphate group of the branched chain was reacted with m7GDP imidazolide (Im-m7GDP) as previously reported (ACS Chem Biol 2022, 17, 1308-1314) to introduce an m7G cap structure. The capping reaction mixture (20 µM phosphorylated RNA oligonucleotide, 10 mM Im-m7GDP, 10 mM 2-nitroimidazole, 10 mM calcium chloride, DMSO solution) was incubated at 55°C for 3 hours, then RNA was recovered by alcohol precipitation, and the desired RNA was separated and purified by reversed-phase HPLC. The 5' end of the oligonucleotide backbone was enzymatically phosphorylated using T4 polynucleotide kinase (Takara Bio Inc) (8 µM oligo, 1 mM ATP, 50 mM Tris-HCl (pH of 8.0), 10 mM MgCl₂, 5 mM DTT, 0.2 U/µL T4 PNK; incubated at 37°C for 1 hour). Two fragments of the RNA oligonucleotides thus prepared were reacted with T4 RNA ligase 2 and ligated in the presence of template DNA oligonucleotides to prepare a circular RNA. As the template DNA oligonucleotides, sequences each having a sequence complementary to the terminal portion of the RNA strand to be ligated were used. The two types of RNA oligonucleotides used to construct 127-nt circRNA were as follows: 5' d(CACCATGGTGGCTCTGGACTAGGAG (SEQ ID NO: 52)) 3' and 5' d(TTTTTTTTTTTTTTTTTATCACAGTTTTTC (SEQ ID NO: 53)) 3. The two types of RNA oligonucleotides used to construct 167-nt circRNA were as follows: 5' d(CACCGAGGCTCCAGCTTATCACAGTTTTTC (SEQ ID NO: 54)) 3' and 5' d(CACCATGGTGGCTCTGGACTAGGAG (SEQ ID NO: 55)) 3'. The composition of the ligase reaction mixture was as follows: 1 µM RNA oligonucleotides (two types), 3 µM DNA oligonucleotides (two types), 10% PEG8000, 50 mM Tris-HCl (pH of 7.5), 2 mM MgCl₂, 1 mM DTT, 400 µM ATP, 0.1 µg/µL T4 RNA ligase 2. The reaction mixture was incubated at 37°C for 2 hours and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The progress of the reaction was confirmed by denaturing acrylamide gel electrophoresis (PAGE), and the desired RNA ligation product was isolated and purified by preparative denaturing polyacrylamide gel electrophoresis (Sci. Rep., 5, 16435, 2015). An experiment evaluating the expression level of the HiBiT peptide was carried out using a human-derived cultured cell strain HeLa (Riken Cell Bank, RCB0007). HeLa cells were cultured in DMEM (Wako Pure Chemical Industries, Ltd.) medium containing 10% FBS (Thermo Fisher) at a CO₂ concentration of 5% and 37°C. The HeLa cells were seeded in a 96-well multiwell plate at 1 × 10E4 cells/well on the day before transfection. For each well, 0.2 or 2 pmol of the circular RNA was mixed with 0.3 µL of Lipofectamine MessengerMAX (Thermo Fisher) and incubated for 5 minutes, and then the mixture was added to the wells containing the HeLa cells in which the growth medium had been replaced with Opti-MEM^{™} I Reduced Serum Medium (Thermo Fisher) to transfect the cells with the RNA. After 3 hours, an equal amount (100 µL) of the growth medium was added, and the cells were further cultured for 3 hours. Then, the expression level of the HiBiT peptide in the cell lysate was evaluated using the Nano Glo HiBiT Lytic Detection System (Promega). The TriStar5 multimode plate reader (Berthold) was used for measuring the amount of chemiluminescence.

The results are shown in Figs. 8 and 9. It was found that the peptide is produced highly efficiently from the chemically synthesized circular mRNAs containing an m7G cap structure.

### Test Example 7. In vivo persistence of chemically synthesized circular mRNA containing an m7G cap structure

### Experimental Section: Evaluation of in vivo expression of HiBiT peptide

Lipid nanoparticles encapsulating mRNA encoding HiBiT (HiBiT-mRNA, SEQ ID NO: 59) were administered to mice by intravenous tail vein administration, and the expression level of the HiBiT peptide in the liver was measured. First, the lipid nanoparticles encapsulating HiBiT-mRNA were administered into the tail vein of C57BL/6N mice (5 weeks old, female) at an mRNA dosage of 10 µg per mouse, and the livers were collected 6, 24, 30, or 48 hours after administration. The livers were cut into small pieces with dissecting scissors and then homogenized by bead beating in Lysis Buffer (100 mM Tris-HCl, 1 mM EDTA, 0.1% TritonX-100, pH of 7.8). The resulting homogenates were centrifuged (15,000 rpm, 4°C, 10 minutes), and the supernatants were collected. After mixing 30 µL of the supernatants and 60 µL of HiBiT substrate solution (Nano Glo HiBiT Lytic Detection System, Promega, N3040), the luminescence value (RLU) was measured with a luminometer. The supernatants were diluted 50-fold, the total protein amount (mg protein) was measured by BCA protein assay, and the luminescence value was corrected using the protein amount (RLU/mg protein).

The results are shown in Fig. 10. It was found that the chemically synthesized circular mRNA containing an m7G cap structure has higher in vivo persistence than the linear mRNA.

### Test Example 8. Evaluation of translation activity of chemically synthesized circular mRNA containing an m7G cap structure

The base sequences of the circular RNAs used are as follows.

In the sequences,
m7G represents N7-methylguanosine (cap structure linked via a 5'-5' triphosphate structure (-ppp-));
mG represents 2'-O-methylguanosine;
Ψ represents 1-methylpseudouridine; and
X represents a branched structure derived from Asymmetric Doubler (Lev) Phosphoramidite (Glen Research).
Cap-binding circular mRNA (Cap-circ) (200-nt)

5'-GGCGCAUAUUAAGGUGACGCGUGUGGCCUCGAACACCGAGGGUA-X-

AAGCCACCAUGGΨGAGCGGCΨGGCGGCΨGΨΨCAAGAAGAΨΨAGCGGGAGCΨCCGGΨAGΨAΨAAΨC

AACΨΨΨGAAAAACΨGΨGAΨAAGCΨGGAGCCΨCGGΨGGCCAΨGCΨΨCΨΨGCCCCΨΨGGGCCΨAAAA

AAAAAAAAAAAAAAAAAAAAAAAAAAA-3'

The 5' and 3' ends are linked to form a circular structure.

The circular mRNA contains a sub-strand (5'm7G-ppp-mGmGAGCG-) branched from X (SEQ ID NO: 69).
HRV-B3-IRES-containing circ mRNA (HRV-B3 circ/U) (816-nt)

5'GGUUAAAACAGCGGAUGGGUACCCCACCAUCCGACCCACUGGGUGUAGUACUCUGGUACUUCG
UACCUUUGUACGCCUGUUCUUCCCAUUGUACCCUUCCUGAACUUCCAACCCAAGUAACGUUAGAA
GCUCAACAUUUAGUACAACAGGAAGCACCACAUCCAGUGGUGUUUAGUACAAGCACUUCUGUUUC
CCCGGAGCGAGGUAUAGGCUGUACCCACUGCCAAAAACCUUUAACCGUUAUCCGCCAACCAACUA
CGUAAAAGCUAGUAGUAUUAUGUUUUUAACUAGGCGUUCGAUCAGGUGGAUUUCCCCUCCACUAG
UUUGGUCGAUGAGGCUAGGAAUUCCCCACGGGUGACCGUGUCCUAGCCUGCGUGGCGGCCAACCC
AGCCCACUCACUAUUUGUUUUCGCGCCCAGUUGCAAAAAGUGUCGGGGCUGGGACGCCUUUUUAU
AGACAUGGUGUGAAGACUCGCAUGUGCUUGGUUGUGAUUCCUCCGGCCCCUGAAUGCGGCUAACC
UUAACCCUGGAGCCUUGUGUCACAAACCAGUGAUGAUAAGGUCGUAAUGAGCAAUUCCGGGACGG
GACCGACUACUUUGGGUGUCCGUGUUUCUUAUUUUUCUUAUUAUUGUCUUAUGGUCACAGCAUAU
AUAUAACAUAUACUGUGAUCAUGGUGAGCGGCUGGCGGCUGUUCAAGAAGAUUAGCGGGAGCUCC
GGUAGUAUAAUCAACUUUGAAAAACUGUGAUAAGCUGGAGCCUCGGUGGCCAUGCUUCUUGCCCC
UUGGGCCUAAAAAAAAAAAAAAAAAAAAAAAAAAAAAA 3'

The 5' and 3' ends are linked to form a circular structure (SEQ ID NO: 70).
HRV-B3-IRES-containing circ mRNA (HRV-B3 circ/m1Ψ) (816-nt)

5'GGΨΨAAAACAGCGGAΨGGGΨACCCCACCAΨCCGACCCACΨGGGΨGΨAGΨACΨCΨGGΨACΨΨCG
ΨACCΨΨΨGΨACGCCΨGΨΨCΨΨCCCAΨΨGΨACCCΨΨCCΨGAACΨΨCCAACCCAAGΨAACGΨΨAGAA
GCΨCAACAΨΨΨAGΨACAACAGGAAGCACCACAΨCCAGΨGGΨGΨΨΨAGΨACAAGCACΨΨCΨGΨΨΨC
CCCGGAGCGAGGΨAΨAGGCΨGΨACCCACΨGCCAAAAACCΨΨΨAACCGΨΨAΨCCGCCAACCAACΨA
CGΨAAAAGCΨAGΨAGΨAΨΨAΨGΨΨΨΨΨAACΨAGGCGΨΨCGAΨCAGGΨGGAΨΨCCCCΨCCACΨAG
ΨΨΨGGΨCGAΨGAGGCΨAGGAAΨΨCCCCACGGGΨGACCGΨGΨCCΨAGCCΨGCGΨGGCGGCCAACCC
AGCCCACΨCACΨAΨΨΨGΨΨΨΨCGCGCCCAGΨΨGCAAAAAGΨGΨCGGGGCΨGGGACGCCΨΨΨΨAΨ
AGACAΨGGΨGΨGAAGACΨCGCAΨGΨGCΨΨGGΨΨGΨGAΨΨCCΨCCGGCCCCΨGAAΨGCGGCΨAACC
ΨΨAACCCΨGGAGCCΨΨGΨGΨCACAAACCAGΨGAΨGAΨAAGGΨCGΨAAΨGAGCAAΨΨCCGGGACGG
GACCGACΨACΨΨΨGGGΨGΨCCGΨGΨΨΨCΨΨAΨΨΨΨΨCΨΨAΨΨAΨΨGΨCΨΨAΨGGΨCACAGCAΨAΨ
AΨAΨAACAΨAΨACΨGΨGAΨCAΨGGΨGAGCGGCΨGGCGGCΨGΨΨCAAGAAGAΨΨAGCGGAGCΨCC
GGΨAGΨAΨAAΨCAACΨΨΨGAAAAACΨGΨGAΨAAGCΨGGAGCCΨCGGΨGGCCAΨGCΨΨCΨΨGCCCC
ΨΨGGGCCΨAAAAAAAAAAAAAAAAAAAAAAAAAAAAAA 3'

The 5' and 3' ends are linked to form a circular structure (SEQ ID NO: 71).

Each circular RNA was prepared as follows. First, 5'-end phosphorylated linear RNA as a pre-circularization starting material was prepared by a transcription reaction of the following composition: 5 ng/µL dsDNA (PCR product containing T7 promoter), 2 mM ATP, 2 mM UTP or 1-methylpseudouridine-5'-triphosphate, 2 mM CTP, 2 mM GTP, 10 mM GMP, 40 mM Tris-HCl (pH of 8.0), 8 mM MgCl₂, 2 mM spermidine, 5 mM DTT, 0.002 U/µL pyrophosphatase, 4.7 ng/µL T7 RNA polymerase. The reaction mixture was incubated at 37°C for 2 hours, DNase I (Takara Bio Inc.) was then added to a final concentration of 0.1 U/µL, and the mixture was incubated at 37°C for 15 minutes. The reaction mixture was extracted with a mixture of equal amounts of TE-saturated phenol and chloroform and deproteinated, and then the transcribed RNA was recovered by alcohol precipitation. The resulting transcribed RNA as a crude product was purified by reversed-phase HPLC as previously reported (Nat. Commun., 14, 2657, 2023). The resulting 5'-end phosphorylated transcribed RNA was ligated using T4 RNA ligase 2 to obtain circular RNA. The composition of the ligase reaction mixture was as follows: 0.5 µM 5'-phosphorylated RNA, 1 µM template oligo DNA, 10% PEG8000, 50 mM Tris-HCl (pH of 7.5), 2 mM magnesium chloride, 1 mM DTT, 400 µM ATP, 0.1 µg/µL T4 RNA ligase 2. The reaction mixture was incubated at 37°C for 1 hour and then extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis.

An experiment evaluating the expression level of the HiBiT peptide was carried out using a human-derived cultured cell strain HeLa (Riken Cell Bank, RCB0007). HeLa cells were cultured in DMEM (Wako Pure Chemical Industries, Ltd.) containing 10% FBS (Thermo Fisher) at a CO₂ concentration of 5% and 37°C. The HeLa cells were seeded in a 96-well multiwell plate at 1 × 10E4 cells/well on the day before transfection. mRNA (20 ng/well) was mixed with 0.3 µL/well of Lipofectamine MessengerMAX (Thermo Fisher) and incubated for 10 minutes, and then the mixture was added to the wells containing the HeLa cells in which the growth medium had been replaced with Opti-MEM (trademark) I Reduced Serum Medium (Thermo Fisher) to transfect the cells with the RNA. After 3 hours, the supernatant was replaced with the growth medium, and, after culturing for a total of 5, 10, 20, 30, or 45 hours, HiBiT peptide expression levels in the cells were evaluated using the Nano-Glo (registered trademark) HiBiT Lytic Detection System (Promega) and the TriStar5 multimode plate reader (Berthold).

The results are shown in Fig. 11. It was found that the chemically synthesized circular mRNA containing an m7G cap structure (branched cap-introduced circular mRNA), which does not contain an IRES (about 600-nt), can be minimized in size and exhibits high translation activity.

### Test Example 9. Phenomenon of enhanced translation of circular mRNA by hybridization with endogenous mRNA (ACTB)

A conceptual diagram of this experiment is shown in Fig. 12(A).

The nucleobase sequences used in this experiment are as follows.
ACTB siRNA top strand:
   5' _UACCUGUACACUGACUUGAGAUU_3' (SEQ ID NO: 60).
ACTB siRNA bottom strand:
   5' _UCUCAAGUCAGUGUACAGGUAUU_ 3' (SEQ ID NO: 61).
NoAntisense Nluc circRNA (682-nt):

5'_GGUCCCAGGUCCACUUCAAGUAAACCCCUACCAACUGGUCGGGGUUUGAAAC<>GCGUGUGG
CCUCGAACACCGAGCGACCCUGCAGCGACCCGCUUAAAAGCUUGGCAAUCCGGUACUGUUGGUAA
AGCCACCAUGGUCUUCACACUCGAAGAUUUCGUUGGGGACUGGCGACAGACAGCCGGCUACAACC
UGGACCAAGUCCUUGAACAGGGAGGUGUGUCCAGUUUGUUUCAGAAUCUCGGGGUGUCCGUAACU
CCGAUCCAAAGGAUUGUCCUGAGCGGUGAAAAUGGGCUGAAGAUCGACAUCCAUGUCAUCAUCCC
GUAUGAAGGUCUGAGCGGCGACCAAAUGGGCCAGAUCGAAAAAAUUUUUAAGGUGGUGUACCCUG
UGGAUGAUCAUCACUUUAAGGUGAUCCUGCACUAUGGCACACUGGUAAUCGACGGGGUUACGCCG
AACAUGAUCGACUAUUUCGGACGGCCGUAUGAAGGCAUCGCCGUGUUCGACGGCAAAAAGAUCAC
UGUAACAGGGACCCUGUGGAACGGCAACAAAAUUAUCGACGAGCGCCUGAUCAACCCCGACGGCU
CCCUGCUGUUCCGAGUAACCAUCAACGGAGUGACCGGCUGGCGGCUGUGCGAACGCAUUCUGGCG
UAAUUCUAAAAAAAAAAAAAAAAAAAAAAAAAAAAAA_3'

The 5' and 3' ends are linked. A sequence in which a complementary sequence to ACTB mRNA has been introduced to the portion indicated by "<>" is the ACTB antisense Nluc circRNA (SEQ ID NO: 62) shown below.
ACTB antisense Nluc circRNA (705-nt):

5'_GGUCCCAGGUCCACUUCAAGUAAACCCCUACCAACUGGUCGGGGUUUGAAACGGCAAAGGCG
AGGCTCTGTGCTCGCGUGUGGCCUCGAACACCGAGCGACCCUGCAGCGACCCGCUUAAAAGCUUG
GCAAUCCGGUACUGUUGGUAAAGCCACCAUGGUCUUCACACUCGAAGAUUUCGUUGGGGACUGGC
GACAGACAGCCGGCUACAACCUGGACCAAGUCCUUGAACAGGGAGGUGUGUCCAGUUUGUUUCAG
AAUCUCGGGGUGUCCGUAACUCCGAUCCAAAGGAUUGUCCUGAGCGGUGAAAAUGGGCUGAAGAU
CGACAUCCAUGUCAUCAUCCCGUAUGAAGGUCUGAGCGGCGACCAAAUGGGCCAGAUCGAAAAAA
UUUUUAAGGUGGUGUACCCUGUGGAUGAUCAUCACUUUAAGGUGAUCCUGCACUAUGGCACACUG
GUAAUCGACGGGGUUACGCCGAACAUGAUCGACUAUUUCGGACGGCCGUAUGAAGGCAUCGCCGU
GUUCGACGGCAAAAAGAUCACUGUAACAGGGACCCUGUGGAACGGCAACAAAAUUAUCGACGAGC
GCCUGAUCAACCCCGACGGCUCCCUGCUGUUCCGAGUAACCAUCAACGGAGUGACCGGCUGGCGG
CUGUGCGAACGCAUUCUGGCGUAAUUCUAAAAAAAAAAAAAAAAAAAAAAAAAAAAAAA_3'

The 5' and 3' ends are linked. The underlined portion indicates the complementary sequence to ACTB mRNA. All the underlined nucleotides are native for ACTBnative, 2'Ome-modified for ACTB_2'OMe, and LNA-modified for ACTB_LNA. (SEQ ID NO: 63).
NoAntisense splint (24-nt):
   5'_dGAGGCCACACGCGTTTCAAACCCC _3' (SEQ ID NO: 64).
ACTB antisense splint 1 (24-nt):
   5'_dCTCGCCTTTGCCGTTTCAAACCCC_3' (SEQ ID NO: 65).
ACTB antisense splint 2 (24-nt):
   5' dGAGGCCACACGCGAGCACAGAGCC 3' (SEQ ID NO: 66).
circRNA backbone (682-nt): 
5'_phos-
GCGUGUGGCCUCGAACACCGAGCGACCCUGCAGCGACCCGCUUAAAAGCUUGGCAAUCCGGUACU
GUUGGUAAAGCCACCAUGGUCUUCACACUCGAAGAUUUCGUUGGGGACUGGCGACAGACAGCCGG
CUACAACCUGGACCAAGUCCUUGAACAGGGAGGUGUGUCCAGUUUGUUUCAGAAUCUCGGGGUGU
CCGUAACUCCGAUCCAAAGGAUUGUCCUGAGCGGUGAAAAUGGGCUGAAGAUCGACAUCCAUGUC
AUCAUCCCGUAUGAAGGUCUGAGCGGCGACCAAAUGGGCCAGAUCGAAAAAAUUUUUAAGGUGGU
GUACCCUGUGGAUGAUCAUCACUUUAAGGUGAUCCUGCACUAUGGCACACUGGUAAUCGACGGGG
UUACGCCGAACAUGAUCGACUAUUUCGGACGGCCGUAUGAAGGCAUCGCCGUGUUCGACGGCAAA
AAGAUCACUGUAACAGGGACCCUGUGGAACGGCAACAAAAUUAUCGACGAGCGCCUGAUCAACCC
 
CGACGGCUCCCUGCUGUUCCGAGUAACCAUCAACGGAGUGACCGGCUGGCGGCUGUGCGAACGCA

UUCUGGCGUAAUUCUAAAAAAAAAAAAAAAAAAAAAAAAAAAAAAGGUCCCAGGUCCACUUCAAG

UAAACCCCUACCAACUGGUCGGGGUUUGAAAC_3' (SEQ ID NO: 67).
ACTB antisense insert (47-nt):
   5' _phos-GGGGTTTGAAACGGCAAAGGCGAGGCTCTGTGCTCGCGTGTGGCCTC_3'

The underlined portion indicates the complementary sequence to ACTB mRNA. Three types in which all the underlined nucleotides are native, 2'OMe-modified, or LNA-modified were used. (SEQ ID NO: 68).

The circRNA backbone was synthesized by transcription using DNA encoding the corresponding sequence as a template and T7 RNA polymerase. The composition of the transcription reaction was as follows: 10 ng/µL dsDNA (PCR product containing T7 promoter), 2 mM ATP, 2 mM UTP, 2 mM CTP, 2 mM GTP, 10 mM GMP, 40 mM Tris-HCl (pH of 8.0), 8 mM MgCl₂, 2 mM spermidine, 5 mM DTT, 0.002 U/µL pyrophosphatase (New England Biolabs), 2 U/µL T7 RNA polymerase (Takara Bio Inc). The reaction mixture was incubated at 37°C for 2 hours. After the transcription reaction, DNase I (Takara Bio Inc) was added to a final concentration of 0.1 U/µL, and the mixture was incubated at 37°C for 15 minutes. An aqueous 7.5 M lithium chloride solution was added to the reaction mixture to give a final concentration of 2.5 M, and the mixture was cooled at -30°C for 30 minutes and then centrifuged (20,000Xg, 20 min) to recover the transcribed RNA as a precipitate. The resulting transcribed RNA as a crude product was purified by reversed-phase HPLC as previously reported (Nat. Commun., 14, 2657, 2023). For NoAntisense Nluc circRNA, 8 µM of NoAntisense splint was mixed with 2 µM of the 5'-phosphorylated RNA prepared by the above-described transcription reaction. For ACTB antisense Nluc circRNA, 2 µM of the 5'-phosphorylated RNA, 8 µM of ACTB antisense splint 1, 8 µM of ACTB antisense splint 2, and 4 µM of ACTB antisense insert were mixed. Each of these mixtures was treated at 37°C for 1 hour in presence of 10% PEG8000, 50 mM Tris-HCl (pH of 7.5), 2 mM MgCl₂, 1 mM DTT, 400 µM ATP, and 0.1 µg/µL T4 RNA ligase 2 so as to be ligated. Thereafter, the reaction mixture was extracted with a mixture of equal amounts of TE-saturated phenol and chloroform and deproteinated, an aqueous sodium acetate solution (pH of 5.2, final concentration: 0.3 M) and 2-propanol were added, and the mixture was cooled at -30°C and then centrifuged to recover RNA as a precipitate. The desired circularized RNA was isolated and purified by preparative denaturing polyacrylamide gel electrophoresis (Sci. Rep., 5, 16435, 2015).

An experiment evaluating the expression level of the Nluc protein was carried out using a human-derived cultured cell strain HeLa (Riken Cell Bank, RCB0007). HeLa cells were cultured in DMEM (Wako Pure Chemical Industries, Ltd.) containing 10% FBS (Thermo Fisher) at a CO₂ concentration of 5% and 37°C. The HeLa cells were seeded in a 96-well multiwell plate at 1 × 10E4 cells/well on the day before transfection.

For knockdown of ACTB, 10 µM ACTB siRNA top strand, 10 µM ACTB siRNA bottom strand, 50 mM NaCl, and 40 mM Tris-HCl (pH of 8.0) were treated at 80°C for 5 minutes and slowly cooled to 15°C at -0.1°C/sec to prepare siRNA. The medium of the HeLa cells in the 96-well multiwell plate prepared on the previous day was replaced, then the siRNA was added at 1 pmol/well using Lipofectamine RNAiMAX (Thermo Fisher) as a lipofection reagent, and the cells were cultured at a CO₂ concentration of 5% and 37°C for 24 hours.

24 hours after the addition of siRNA, the medium was replaced again, then NoAntisense Nluc circRNA or each ACTB antisense Nluc circRNA was added at 25 ng/well using Lipofectamine MessengerMax (ThermoFisher) as a lipofection reagent, and the cells were cultured at a CO₂ concentration of 5% and 37°C for 8 hours. For both Lipefectamine RNAiMAX and Lipofectamine Messenger Max, the manufacturers' protocols were followed in the liposome formation.

8 hours after the addition of circRNA, each well was washed once with D-PBS (nacalai), and Nluc expression levels in the cells were evaluated using the Nano-Glo (registered trademark) Luciferase Assay System (Promega). The TriStar5 multimode plate reader (Berthold) was used for measuring the amount of chemiluminescence.

The results are shown in Fig. 12. The hybridization with endogenous mRNA (ACTB) enhanced the translation of circular mRNA.

## Claims

1. A method for producing or expressing a protein or a peptide, the method comprising:
carrying out a translation reaction in a reaction system comprising:
a 5'-capped polynucleotide containing an arbitrary base sequence A; and
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure.

2. The method according to claim 1, wherein the 5'-capped polynucleotide is a 5'-capped RNA.

3. The method according to claim 1, wherein the 5'-capped polynucleotide or the single-stranded RNA is a polynucleotide endogenous to a living organism or a cell.

4. The method according to claim 3, wherein the polynucleotide endogenous to a living organism or a cell is a lncRNA or an mRNA.

5. The method according to claim 1, wherein the translation efficiency enhancement structure is a structure for stabilizing the binding between the 5'-capped polynucleotide and the single-stranded RNA.

6. The method according to claim 1, wherein the method satisfies the requirement (b) that the 5'-capped polynucleotide and/or the single-stranded RNA has a nuclease-binding structure, and the reaction system contains the nuclease,
wherein the nuclease is RISC or Cas, and
the 5'-capped polynucleotide is a siRNA or a miRNA, or
the 5'-capped polynucleotide is a lncRNA or an mRNA, and the single-stranded RNA contains a tracrRNA sequence.

7. The method according to claim 1, wherein the method satisfies at least one requirement selected from the group consisting of:
(a) the single-stranded RNA is a circular RNA; and
(c) the 5'-capped polynucleotide and/or the single-stranded RNA has a structure allowing the 5'-capped polynucleotide and the single-stranded RNA to be covalently linked.

8. The method according to claim 7, wherein the method satisfies the requirement (a), and the circular RNA contains no stop codon, and/or
the method satisfies the requirement (c), and the structure is a photocrosslinkable structure.

9. A composition comprising:
a 5'-capped polynucleotide containing an arbitrary base sequence A; and/or
a single-stranded RNA containing a base sequence B capable of binding to the base sequence A via complementary base pairing, and a protein-coding or peptide-coding sequence,
wherein the 5'-capped polynucleotide and/or the single-stranded RNA has a translation efficiency enhancement structure.

10. The composition according to claim 9 for use in the method according to any one of claims 1 to 8.

11. A method for producing or expressing a protein or a peptide, the method comprising:
carrying out a translation reaction in a reaction system comprising:
a single-stranded circular RNA having a length of 500 or less bases, the single-stranded circular RNA comprising a 5'-capped polynucleotide linked thereto via a linker.

12. A composition comprising a single-stranded circular RNA having a length of 500 or less bases, the single-stranded circular RNA comprising a 5'-capped polynucleotide linked thereto via a linker.

13. The composition according to claim 12 for use in the method according to claim 11.

14. A method for producing a 5'-capped polynucleotide comprising:
carrying out a transcription reaction in a reaction system comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side) -Y1-Y2-Y3, wherein Y1 and Y2 each represent a base in the base-inserted mutant sequence, and Y3 represents a base at the transcription initiation site; and
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2.

15. The method according to claim 14, wherein an antisense strand of the phage promoter contains the base sequence Y:
(upstream side)-Y1-Y2-Y3, and wherein X1 is a base complementary to Y1, and X2 is a base complementary to Y2.

16. The method according to claim 14, wherein the 5'-capped polynucleotide is a 5'-capped RNA.

17. The method according to claim 14, wherein the phage promoter is a T7 promoter.

18. The method according to claim 14, wherein the base sequence X is (cap side)-CUG, CAG, GUG, or GAG, and Y3 is C.

19. A composition comprising:
a double-stranded polynucleotide comprising a phage promoter containing a base-inserted mutant sequence adjacent upstream of a transcription initiation site, and comprising an antisense strand containing a base sequence Y: (upstream side)-Y2-Y3 or (upstream side)-Y1-Y2-Y3, wherein Y1 and Y2 each represent a base in the base-inserted mutant sequence, and Y3 represents a base at the transcription initiation site; and/or
a 5'-cap analog comprising a polynucleotide containing a base sequence X: (cap side)-X2-X3 or (cap side)-X1-X2-X3,
wherein X3 is a base complementary to Y3, and
X1 is a base complementary to Y1, and/or X2 is a base complementary to Y2.

20. The composition according to claim 19 for use in the method according to any one of claims 14 to 18.

21. A T7 RNA polymerase comprising a D130W mutation.
